(19) Europäisches Patentamt
European Patent Office
Office européen des brevets

(11) EP 4 310 073 A1

(12) **EUROPEAN PATENT APPLICATION**
published in accordance with Art. 153(4) EPC

(43) Date of publication:
**24.01.2024 Bulletin 2024/04**

(21) Application number: **22770989.6**

(22) Date of filing: **16.02.2022**

(51) International Patent Classification (IPC):
***C07C 307/02*** *(2006.01)* ***C07C 311/53*** *(2006.01)*
***H01M 4/505*** *(2010.01)* ***H01M 4/525*** *(2010.01)*
***H01M 10/052*** *(2010.01)* ***H01M 10/0567*** *(2010.01)*
***H01M 10/058*** *(2010.01)*

(52) Cooperative Patent Classification (CPC):
**C07C 307/02; C07C 311/53; H01M 4/505; H01M 4/525; H01M 10/052; H01M 10/0567; H01M 10/058;** Y02E 60/10

(86) International application number:
**PCT/JP2022/006194**

(87) International publication number:
**WO 2022/196230 (22.09.2022 Gazette 2022/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.03.2021 JP 2021044154**
**03.09.2021 JP 2021143890**

(71) Applicant: **Mitsui Chemicals, Inc.**
**Tokyo 104-0028 (JP)**

(72) Inventors:
- **KUBO, Teruhiko**
**Sodegaura-shi, Chiba 299-0265 (JP)**
- **MIO, Shigeru**
**Sodegaura-shi, Chiba 299-0265 (JP)**
- **SHIMIZU, Yusuke**
**Sodegaura-shi, Chiba 299-0265 (JP)**
- **OJI, Yurika**
**Sodegaura-shi, Chiba 299-0265 (JP)**
- **SUGURO, Masahiro**
**Sodegaura-shi, Chiba 299-0265 (JP)**

(74) Representative: **J A Kemp LLP**
**80 Turnmill Street**
**London EC1M 5QU (GB)**

# (54) LITHIUM (N-CARBONYL)SULFONAMIDE COMPOUND, ADDITIVE FOR LITHIUM SECONDARY BATTERY, NON-AQUEOUS ELECTROLYTE FOR LITHIUM SECONDARY BATTERY, LITHIUM SECONDARY BATTERY PRECURSOR, LITHIUM SECONDARY BATTERY, AND METHOD FOR PRODUCING LITHIUM SECONDARY BATTERY

(57) Provided is a lithium (N-carbonyl)sulfonamide compound represented by the following Formula (I). In Formula (I), $R^1$ and $R^2$ each represent an alkyl group having from 1 to 10 carbon atoms, an alkenyl group having from 2 to 10 carbon atoms, an alkynyl group having from 2 to 10 carbon atoms, or an aryl group, and $L^1$ and $L^2$ each represent a single bond or -O-, with a proviso that a case in which $L^1$ and $L^2$ are both single bonds are excluded.

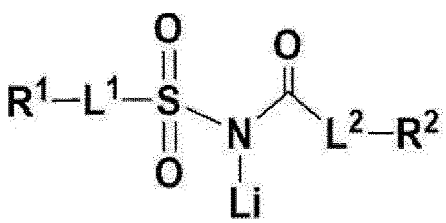

(I)

EP 4 310 073 A1

## Description

### Technical Field

The present disclosure relates to: a lithium (*N*-carbonyl)sulfonamide compound; an additive for a lithium secondary battery; a nonaqueous electrolyte solution for a lithium secondary battery; a lithium secondary battery precursor; a lithium secondary battery; and a method of producing a lithium secondary battery.

### Background Art

Lithium secondary batteries have been attracting attention as batteries having a high energy density.
Patent Document 1 discloses a compound used as a salt in an electrolyte composition. The compound concretely disclosed in Patent Document 1 is lithium trifluoromethylcarbonyl trifluoromethyl sulfonamide.
Patent Document 1: Japanese National-Phase Publication (JP-A) No. 2020-515558

## SUMMARY OF THE INVENTION

### Technical Problem

However, in a lithium secondary battery in which lithium trifluoromethylcarbonyl trifluoromethyl sulfonamide disclosed in Patent Document 1 is added to a nonaqueous solvent, charging or discharging in a high-temperature environment may cause an increase in the direct-current resistance as well as a decrease in the discharge capacity.
In view of the above-described circumstances, an object of the disclosure is to provide: a lithium (*N*-carbonyl)sulfonamide compound with which an increase in the direct-current resistance and a decrease in the discharge capacity of a lithium secondary battery can be inhibited even when the lithium secondary battery is stored in a high-temperature environment; an additive for a lithium secondary battery; a nonaqueous electrolyte solution for a lithium secondary battery; a lithium secondary battery precursor; a lithium secondary battery; and a method of producing a lithium secondary battery.

### Solution to Problem

Means for solving the above-described problem include the following embodiments.

<1> A lithium (*N*-carbonyl)sulfonamide compound represented by the following Formula (I):

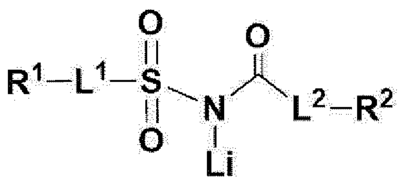

(I)

wherein, in Formula (I):

each of $R^1$ and $R^2$ represents an alkyl group having from 1 to 10 carbon atoms, wherein at least one hydrogen atom of the alkyl group is optionally substituted with a halogen atom, an alkenyl group having from 2 to 10 carbon atoms, wherein at least one hydrogen atom of the alkenyl group is optionally substituted with a halogen atom, an alkynyl group having from 2 to 10 carbon atoms, wherein at least one hydrogen atom of the alkynyl group is optionally substituted with a halogen atom, or an aryl group, wherein at least one hydrogen atom of the aryl group is optionally substituted with a halogen atom, an alkoxy group having from 1 to 6 carbon atoms or an alkyl group having from 1 to 6 carbon atoms, and
$L^1$ and $L^2$ each represent a single bond or -O-, and $L^1$ and $L^2$ are not both single bonds.

<2> An additive for a lithium secondary battery, containing a lithium (N-carbonyl)sulfonamide compound (I) represented by the following Formula (I):

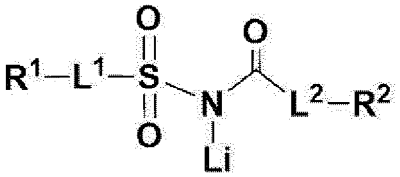

(I)

wherein, in Formula (I):

each of $R^1$ and $R^2$ represents an alkyl group having from 1 to 10 carbon atoms, wherein at least one hydrogen atom of the alkyl group is optionally substituted with a halogen atom, that is not a trifluoromethyl group, an alkenyl group having from 2 to 10 carbon atoms, wherein at least one hydrogen atom of the alkenyl group is optionally substituted with a halogen atom, an alkynyl group having from 2 to 10 carbon atoms, wherein at least one hydrogen atom of the alkynyl group is optionally substituted with a halogen atom, or an aryl group, wherein at least one hydrogen atom of the aryl group is optionally substituted with a halogen atom, an alkoxy group having from 1 to 6 carbon atoms, or an alkyl group having from 1 to 6 carbon atoms; and
$L^1$ and $L^2$ each represent a single bond or -O-.

<3> The additive for a lithium secondary battery according to <2>, wherein

each of $R^1$ and $R^2$ represents, in place of the alkyl group, the alkenyl group, the alkynyl group, or the aryl group, an alkyl group having from 1 to 10 carbon atoms, wherein at least one hydrogen atom of this alkyl group is optionally substituted with a halogen atom, the alkenyl group, the alkynyl group, the aryl group, an aralkyl group having from 7 to 16 carbon atoms, wherein at least one hydrogen atom of an aromatic ring in the aralkyl group is optionally substituted with a halogen atom, an alkoxy group having from 1 to 6 carbon atoms, or an alkyl group having from 1 to 6 carbon atoms, or $R^1$ and $R^2$ represent a halogen atoms, and
a case in which $R^1$ is a halogen atom and $L^1$ is -O-, a case in which $R^2$ is a halogen atom and $L^2$ is -O-, and a case in which $R^1$ and $R^2$ are both the alkyl groups or the aryl groups, and $L^1$ and $L^2$ are both single bonds, are excluded.

<4> A nonaqueous electrolyte solution for a lithium secondary battery, the solution containing a lithium (*N*-carbonyl)sulfonamide compound (I) represented by the following Formula (I):

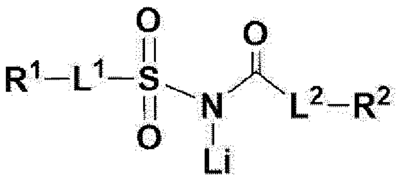

(I)

wherein, in Formula (I):

each of $R^1$ and $R^2$ represents an alkyl group having from 1 to 10 carbon atoms, wherein at least one hydrogen atom of the alkyl group is optionally substituted with a halogen atom, that is not a trifluoromethyl group, an alkenyl group having from 2 to 10 carbon atoms, wherein at least one hydrogen atom of the alkenyl group is optionally substituted with a halogen atom, an alkynyl group having from 2 to 10 carbon atoms, wherein at least one hydrogen atom of the alkynyl group is optionally substituted with a halogen atom, or an aryl group, wherein at least one hydrogen atom of the aryl group is optionally substituted with a halogen atom, an alkoxy group having from 1 to 6 carbon atoms, or an alkyl group having from 1 to 6 carbon atoms, and
$L^1$ and $L^2$ each represent a single bond or -O-.

<5> The nonaqueous electrolyte solution for a lithium secondary battery according to <4>, wherein:

$R^1$ and $R^2$ each represent, in place of the alkyl group, the alkenyl group, the alkynyl group, or the aryl group: an alkyl group having from 1 to 10 carbon atoms, wherein at least one hydrogen atom of this alkyl group is optionally substituted with a halogen atom, the alkenyl group, the alkynyl group, the aryl group, an aralkyl group having from 7 to 16 carbon atoms, wherein at least one hydrogen atom of an aromatic ring in the aralkyl group is optionally substituted with a halogen atom, an alkoxy group having from 1 to 6 carbon atoms, or an alkyl

group having from 1 to 6 carbon atoms, or $R^1$ and $R^2$ represent a halogen atoms, and
a case in which $R^1$ is a halogen atom and $L^1$ is -O-, a case in which $R^2$ is a halogen atom and $L^2$ is -O-, and a case in which $R^1$ and $R^2$ are both the alkyl groups or the aryl groups, and $L^1$ and $L^2$ are both single bonds, are excluded.

<6> The nonaqueous electrolyte solution for a lithium secondary battery according to <4> or <5>, further containing an electrolyte,
wherein the electrolyte is at least one selected from the group consisting of lithium hexafluorophosphate ($LiPF_6$), lithium tetrafluoroborate ($LiBF_4$), lithium hexafluoroarsenate ($LiAsF_6$), lithium hexafluorotantalate ($LiTaF_6$), lithium trifluoromethane sulfonate ($LiCF_3SO_3$), lithium bis(trifluoromethanesulfonyl)imide ($Li(CF_3SO_2)_2N$), and lithium bis(pentafluoroethanesulfonyl)imide ($Li(C_2F_5SO_2)_2N$).
<7> The nonaqueous electrolyte solution for a lithium secondary battery according to any one of <4> to <6>, wherein, in the lithium (N-carbonyl)sulfonamide compound (I):

$R^1$ represents the aryl group,
$L^1$ represents a single bond,
$R^2$ represents the alkyl group, the alkenyl group, the alkynyl group, the aryl group, or the aralkyl group, and
$L^2$ represents -O-.

<8> The nonaqueous electrolyte solution for a lithium secondary battery according to any one of <4> to <6>, wherein, in the lithium (*N*-carbonyl)sulfonamide compound (I):

$R^1$ represents the alkyl group,
$L^1$ represents a single bond,
$R^2$ represents the alkyl group, the alkenyl group, the alkynyl group, the aryl group, or the aralkyl group, and
$L^2$ represents -O-.

<9> The nonaqueous electrolyte solution for a lithium secondary battery according to any one of <4> to <6>, wherein, in the lithium (*N*-carbonyl)sulfonamide compound (I):

$R^1$ represents a fluorine atom,
$L^1$ represents a single bond,
$R^2$ represents the alkyl group, the alkenyl group, the alkynyl group, the aryl group, or the aralkyl group, and
$L^2$ represents -O-.

<10> The nonaqueous electrolyte solution for a lithium secondary battery according to any one of <4> to <9>, containing a compound (II) that is at least one selected from the group consisting of lithium monofluorophosphate and lithium difluorophosphate.
<11> The nonaqueous electrolyte solution for a lithium secondary battery according to any one of <4> to <10>, containing a compound (III) represented by the following Formula (III):

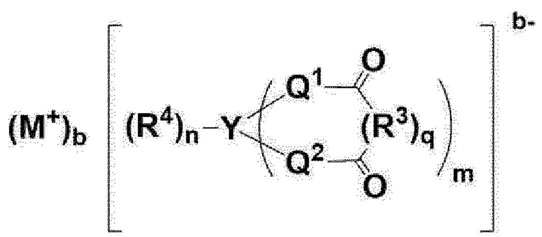

( III )

whrein, in Formula (III):

M represents an alkali metal,
Y represents a transition element, or an element of Group 13, 14, or 15 of the periodic table,
b represents an integer from 1 to 3,
m represents an integer from 1 to 4,
n represents an integer from 0 to 8,
q represents 0 or 1,

$R^3$ represents an alkylene group having from 1 to 10 carbon atoms, a halogenated alkylene group having from 1 to 10 carbon atoms, an arylene group having from 6 to 20 carbon atoms, or a halogenated arylene group having from 6 to 20 carbon atoms, the halogenated alkylene group, the arylene group and the halogenated arylene group each optionally containing a substituent or a heteroatom in a structure thereof and, when q is 1 and m is from 2 to 4, m instances of $R^3$ are optionally bound to each other,
$R^4$ represents a halogen atom, an alkyl group having from 1 to 10 carbon atoms, a halogenated alkyl group having from 1 to 10 carbon atoms, an aryl group having from 6 to 20 carbon atoms, or a halogenated aryl group having from 6 to 20 carbon atoms, the halogenated alkyl group, the aryl group and the halogenated aryl group each optionally containing a substituent or a heteroatom in a structure thereof and, when n is from 2 to 8, n instances of $R^4$ are optionally bound to each other to form a ring, and
each of $Q^1$ and $Q^2$ independently represents an oxygen atom or a carbon atom.

<12> The nonaqueous electrolyte solution for a lithium secondary battery according to any one of <4> to <11>, containing a compound (IV) represented by the following Formula (IV):

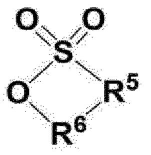 ( IV ) 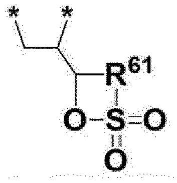 ( iv-1 ) 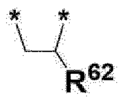 ( iv-2 )

wherein, in Formula (IV):

$R^5$ represents an oxygen atom, an alkylene group having from 1 to 6 carbon atoms, or an alkenylene group having from 2 to 6 carbon atoms,
$R^6$ represents an alkylene group having from 1 to 6 carbon atoms, an alkenylene group having from 2 to 6 carbon atoms, a group represented by the following Formula (iv-1), or a group represented by the following Formula (iv-2):

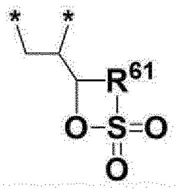 ( iv-1 ) 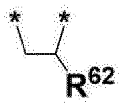 ( iv-2 )

wherein, in Formula (iv-1) and Formula (iv-2), * represents a bonding position,
in Formula (iv-1), $R^{61}$ represents an oxygen atom, an alkylene group having from 1 to 6 carbon atoms, an alkenylene group having from 2 to 6 carbon atoms, or an oxymethylene group, and
in Formula (iv-2), $R^{62}$ represents an alkyl group having from 1 to 6 carbon atoms, or an alkenyl group having from 2 to 6 carbon atoms.

<13> The nonaqueous electrolyte solution for a lithium secondary battery according to any one of <4> to <12>, wherein a content of the lithium (N-carbonyl)sulfonamide compound (I) is from 0.01% by mass to 5% by mass with respect to a total amount of the nonaqueous electrolyte solution for a lithium secondary battery.
<14> A lithium secondary battery precursor, including:

a casing; and
a positive electrode, a negative electrode, a separator, and an electrolyte solution, which are housed in the casing, wherein:

the positive electrode is configured to occlude and release lithium ions,
the negative electrode is configured to occlude and release lithium ions, and
the electrolyte solution is the nonaqueous electrolyte solution for a lithium secondary battery according to any one of <4> to <13>.

<15> The lithium secondary battery precursor according to <14>, wherein the positive electrode contains a lithium-containing composite oxide represented by the following Formula (C1) as a positive electrode active material:

$$LiNi_aCo_bMn_co_2 \qquad (C1)$$

wherein, in Formula (C1), each of a, b, and c independently represents a number larger than 0 but smaller than 1, and a sum of a, b, and c is from 0.99 to 1.00.

<16> A method of producing a lithium secondary battery, the method including:

a step of preparing the lithium secondary battery precursor according to <14> or <15>; and
a step of charging and discharging the lithium secondary battery precursor.

<17> A lithium secondary battery, obtained by charging and discharging the lithium secondary battery precursor according to <14> or <15>.

Advantageous Effects of Invention

According to the disclosure, the following are provided: a lithium (N-carbonyl)sulfonamide compound with which an increase in the direct-current resistance and a decrease in the discharge capacity of a lithium secondary battery can be inhibited even when the lithium secondary battery is stored in a high-temperature environment; an additive for a lithium secondary battery; a nonaqueous electrolyte solution for a lithium secondary battery; a lithium secondary battery precursor; a lithium secondary battery; and a method of producing a lithium secondary battery.

BRIEF DESCRIPTION OF DRAWINGS

FIG. 1 is a schematic cross-sectional view illustrating a laminate-type battery that is one example of the lithium secondary battery precursor of the disclosure.
FIG. 2 is a schematic cross-sectional view illustrating a coin-type battery that is another example of the lithium secondary battery precursor of the disclosure.

DESCRIPTION OF EMBODIMENTS

In the present specification, those numerical ranges that are expressed with "to" each denote a range that includes the numerical values stated before and after "to" as the lower limit value and the upper limit value, respectively.

In the present specification, when there are plural substances that correspond to a component of a composition, an indicated amount of the component in the composition means, unless otherwise specified, a total amount of the plural substances existing in the composition.

In the present specification, the term "step" encompasses not only a discrete step but also a step that cannot be clearly distinguished from other steps, as long as the intended purpose of the step is achieved.

Hereinafter, embodiments of the lithium (N-carbonyl)sulfonamide compound, the additive for a lithium secondary battery, the nonaqueous electrolyte solution for a lithium secondary battery, the lithium secondary battery precursor, the lithium secondary battery, and the method of producing a lithium secondary battery, which are according to the disclosure, are described referring to the drawings. In the drawings, the same or corresponding parts are denoted by the same reference symbol, and description thereof is not repeated.

[Lithium (*N*-carbonyl)sulfonamide Compound]

(Compound (A))

The lithium (*N*-carbonyl)sulfonamide compound of the disclosure will now be described.

The lithium (*N*-carbonyl)sulfonamide compound of the disclosure is a novel compound represented by the following Formula (I) (hereinafter, may be referred to as "compound (A)").

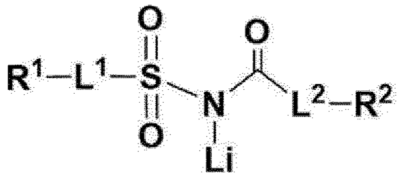

(I)

In Formula (I),

each of $R^1$ and $R^2$ represents an alkyl group having from 1 to 10 carbon atoms (at least one hydrogen atom of the alkyl group is optionally substituted with a halogen atom), an alkenyl group having from 2 to 10 carbon atoms (at least one hydrogen atom of the alkenyl group is optionally substituted with a halogen atom), an alkynyl group having from 2 to 10 carbon atoms (at least one hydrogen atom of the alkynyl group is optionally substituted with a halogen atom), or an aryl group (at least one hydrogen atom of the aryl group is optionally substituted with a halogen atom, an alkoxy group having from 1 to 6 carbon atoms, or an alkyl group having from 1 to 6 carbon atoms); and

$L^1$ and $L^2$ each represent a single bond or -O-, and $L^1$ and $L^2$ are not both single bonds.

The lithium (*N*-carbonyl)sulfonamide compound of the disclosure (i.e. compound (A)) is represented by the below-described Formula (I); therefore, when it is added to a nonaqueous electrolyte solution for a lithium secondary battery (hereinafter, may be referred to as "nonaqueous electrolyte solution"), an increase in the direct-current resistance and a decrease in the discharge capacity can be inhibited even if the lithium secondary battery is stored in a high-temperature environment.

The details of the lithium secondary battery will be described below referring to FIGs. 1 and 2.

The reason why the above-described effect is exerted is presumed to be as follows.

When a lithium secondary battery is produced using a nonaqueous electrolyte solution to which the lithium (*N*-carbonyl)sulfonamide compound of the disclosure (i.e. compound (A)) is added, it is believed that, in the production process (e.g., the below-described aging step), a reaction product is generated in the vicinity of the surface of a negative electrode of the lithium secondary battery, and a component that is a decomposition product of the reaction product is generated as well. This reaction product refers to a product generated by a reaction between the lithium (*N*-carbonyl)sulfonamide compound and a compound (e.g., LiF) generated from an electrolyte. Such a reaction product and the like adhere to the surface of the negative electrode to form a solid electrolyte interphase (SEI) film (hereinafter, referred to as "negative electrode SEI film"). It is believed that, in the production process, this component migrates to the vicinity of the surface of a positive electrode, adheres to the surface of the positive electrode, and forms another SEI film (hereinafter, referred to as "positive electrode SEI film"). By this, the stability of the lithium secondary battery in a high-temperature environment is improved. For example, elution of a metal element contained in a positive electrode active material is inhibited. It is believed that, as a result, an increase in the direct-current resistance of the lithium secondary battery is inhibited even when the lithium secondary battery is stored in a high-temperature environment.

The formation of the positive electrode SEI film is believed to progress even during the period of storing the lithium secondary battery. Therefore, it is believed that, when the lithium secondary battery is stored, the rate of increase in the direct-current resistance of the lithium secondary battery during the period of storing the lithium secondary battery is reduced.

Further, as described above, the lithium secondary battery in which the lithium (*N*-carbonyl)sulfonamide compound of the disclosure (i.e. compound (A)) is used has excellent stability even in a high-temperature environment. In other words, even when the lithium secondary battery is stored in a high-temperature environment, it is believed that a side reaction, which is not a natural battery reaction, is unlikely to proceed. The "battery reaction" refers to a reaction causing lithium ions to move in and out of the positive electrode and the negative electrode (intercalation). Examples of the side reaction include: a reductive decomposition reaction of the electrolyte solution by the negative electrode; an oxidative decomposition reaction of the electrolyte solution by the positive electrode; and elution of a metal element contained in the positive electrode active material. By this, the progress of a decomposition reaction of the nonaqueous electrolyte solution is inhibited. It is believed that, as a result, a decrease in the discharge capacity of the lithium secondary battery is made unlikely to occur even when the lithium secondary battery is stored in a high-temperature environment.

Because of the above-described reason, when the lithium (*N*-carbonyl)sulfonamide compound of the disclosure (i.e. compound (A)) is added to the nonaqueous electrolyte solution, an increase in the direct-current resistance and a decrease in the discharge capacity can be inhibited even if the lithium secondary battery is stored in a high-temperature environment.

Hereinafter, a negative electrode SEI film and a positive electrode SEI film are each simply referred to as "SEI film" when they are not distinguished from each other.

In Formula (I), the "alkyl group having from 1 to 10 carbon atoms" represented by each of $R^1$ and $R^2$ is a linear

or branched alkyl group having from 1 to 10 carbon atoms. Examples of the "alkyl group having from 1 to 10 carbon atoms" include a methyl group, an ethyl group, a propyl group, an isopropyl group, a butyl group, an isobutyl group, a *sec*-butyl group, a *tert*-butyl group, a pentyl group, a 2-methylbutyl group, a 1-methylpentyl group, a neopentyl group, a 1-ethylpropyl group, a hexyl group, a 3,3-dimethylbutyl group, a heptyl group, an octyl group, a nonyl group, and a decyl group. Thereamong, the "alkyl group having from 1 to 10 carbon atoms" is preferably an alkyl group having from 1 to 6 carbon atoms, more preferably an alkyl group having from 1 to 3 carbon atoms.

At least one hydrogen atom of the "alkyl group having from 1 to 10 carbon atoms" is optionally substituted with a halogen atom. The halogen atom is preferably a fluorine atom, a chlorine atom, a bromine atom, or an iodine atom, more preferably a fluorine atom, a chlorine atom, or a bromine atom, still more preferably a fluorine atom or a chlorine atom, particularly preferably a fluorine atom.

In the "alkyl group having from 1 to 10 carbon atoms", the number of hydrogen atoms substituted with the halogen atom is not particularly limited and is selected as appropriate in accordance with the number of carbon atoms of the alkyl group, and it is preferably from 1 to 7.

In Formula (I), the "alkenyl group having from 2 to 10 carbon atoms" represented by each of $R^1$ and $R^2$ is a linear or branched alkenyl group having from 2 to 10 carbon atoms. Examples of the "alkenyl group having from 2 to 10 carbon atoms" include a vinyl group, a 2-propenyl group, a 2-butenyl group, a 3-butenyl group, a 2-pentenyl group, a 3-pentenyl group, a 4-pentenyl group, and a 5-hexenyl group. Thereamong, the "alkenyl group having from 2 to 10 carbon atoms" is preferably an alkenyl group having from 2 to 6 carbon atoms, more preferably an alkenyl group having from 2 to 3 carbon atoms.

At least one hydrogen atom of the "alkenyl group having from 2 to 10 carbon atoms" is optionally substituted with a halogen atom. The halogen atom is preferably a fluorine atom, a chlorine atom, a bromine atom, or an iodine atom, more preferably a fluorine atom, a chlorine atom, or a bromine atom, still more preferably a fluorine atom or a chlorine atom, particularly preferably a fluorine atom.

In the "alkenyl group having from 2 to 10 carbon atoms", the number of hydrogen atoms substituted with the halogen atom is not particularly limited and is selected as appropriate in accordance with the number of carbon atoms of the alkenyl group, and it is preferably from 1 to 7.

In Formula (I), the "alkynyl group having from 2 to 10 carbon atoms" represented by each of $R^1$ and $R^2$ is a linear or branched alkynyl group having from 2 to 10 carbon atoms. Examples of the "alkynyl group having from 2 to 10 carbon atoms" include an ethinyl group, a propargyl group (a 2-propynyl group), a 2-butynyl group, a 3-butynyl group, a 2-pentynyl group, a 3-pentynyl group, a 4-pentynyl group, and a 5-hexynyl group. Thereamong, the "alkynyl group having from 2 to 10 carbon atoms" is preferably an alkynyl group having from 2 to 6 carbon atoms, more preferably an alkynyl group having from 2 to 3 carbon atoms.

At least one hydrogen atom of the "alkynyl group having from 2 to 10 carbon atoms" is optionally substituted with a halogen atom. The halogen atom is preferably a fluorine atom, a chlorine atom, a bromine atom, or an iodine atom, more preferably a fluorine atom, a chlorine atom, or a bromine atom, still more preferably a fluorine atom or a chlorine atom, particularly preferably a fluorine atom.

In the "alkynyl group having from 2 to 10 carbon atoms", the number of hydrogen atoms substituted with the halogen atom is not particularly limited and is selected as appropriate in accordance with the number of carbon atoms of the alkynyl group, and it is preferably from 1 to 7.

In Formula (I), at least one hydrogen atom of the "aryl group" represented by each of $R^1$ and $R^2$ is optionally substituted with a halogen atom, an alkoxy group having from 1 to 6 carbon atoms, or an alkyl group having from 1 to 6 carbon atoms.

The halogen atom is preferably a fluorine atom, a chlorine atom, a bromine atom, or an iodine atom, more preferably a fluorine atom, a chlorine atom, or a bromine atom, still more preferably a fluorine atom or a chlorine atom, particularly preferably a fluorine atom. In the "aryl group", the number of hydrogen atoms substituted with the halogen atom is not particularly limited, and it is preferably from 1 to 5.

In the alkoxy group having from 1 to 6 carbon atoms, its alkyl group may be in any of a linear form, a branched form, and a cyclic form. Examples of the alkoxy group having from 1 to 6 carbon atoms include a methoxy group, an ethoxy group, an *n*-propoxy group, an isopropoxy group, an *n*-butoxy group, a *t*-butoxy group, and a pentyloxy group. Thereamong, the alkoxy group having from 1 to 6 carbon atoms is preferably an alkoxy group having from 1 to 3 carbon atoms, more preferably a methoxy group or an ethoxy group. In the "aryl group", the number of hydrogen atoms substituted with the alkoxy group having from 1 to 6 carbon atoms is not particularly limited, and it is preferably from 1 to 3.

The alkyl group having from 1 to 6 carbon atoms may be in any of a linear form, a branched form, and a cyclic form. Examples of the alkyl group having from 1 to 6 carbon atoms include a methyl group, an ethyl group, an *n*-propyl group, an *i*-propyl group, an *n*-butyl group, an *i*-butyl group, a *t*-butyl group, an *n*-pentyl group, an *n*-hexyl group, and a cyclohexyl group. Thereamong, the alkyl group having from 1 to 6 carbon atoms is preferably an alkyl group having from 1 to 3 carbon atoms, more preferably a methyl group or an ethyl group. In the "aryl group", the number of hydrogen atoms substituted with the alkyl group having from 1 to 6 carbon atoms is not particularly limited, and it is preferably

from 1 to 3.

In Formula (I), $L^1$ and $L^2$ each represent a single bond or -O-. However, a case in which $L^1$ and $L^2$ are both single bonds are excluded. In other words, a case in which $L^1$ is a single bond and $L^2$ is a single bond are excluded. Particularly, $L^1$ preferably represents a single bond, and $L^2$ preferably represents -O-.

Specific examples of the lithium (*N*-carbonyl)sulfonamide compound (i.e. compound (A)) include Synthesis Compounds (I-1) to (I-41) (excluding Synthesis Compound (I-9)) that are synthesized in the below-described Examples.

(Compound (B))

The lithium (*N*-carbonyl)sulfonamide compound of the disclosure may be the above-described lithium (*N*-carbonyl)sulfonamide compound (i.e. compound (A)) in which each of $R^1$ and $R^2$ represents, in place of the above-described alkyl group, the above-described alkenyl group, the above-described alkynyl group, or the above-described aryl group:

the above-described alkyl group, the above-described alkenyl group, the above-described alkynyl group, the above-described aryl group, an aralkyl group having from 7 to 16 carbon atoms (at least one hydrogen atom of an aromatic ring in the aralkyl group is optionally substituted with a halogen atom, an alkoxy group having from 1 to 6 carbon atoms, or an alkyl group having from 1 to 6 carbon atoms), or $R^1$ and $R^2$ represent a halogen atoms, and instead of that a case in which $L^1$ and $L^2$ are each a single bond are excluded,

a case in which $R^1$ is a halogen atom and $L^1$ is -O-, a case in which $R^2$ is a halogen atom and $L^2$ is -O-, and a case in which $R^1$ and $R^2$ are both the above-described alkyl groups or the above-described aryl groups, and $L^1$ and $L^2$ are both single bonds, are excluded.

In other words, the lithium (*N*-carbonyl)sulfonamide compound of the disclosure may be a novel compound represented by the following Formula (I) (hereinafter, may be referred to as "compound (B)").

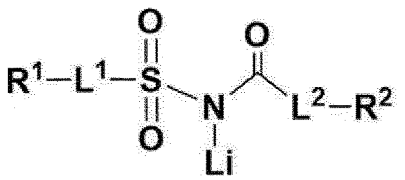 (I)

In Formula (I),

each of $R^1$ and $R^2$ represents an alkyl group having from 1 to 10 carbon atoms (at least one hydrogen atom of the alkyl group is optionally substituted with a halogen atom), an alkenyl group having from 2 to 10 carbon atoms (at least one hydrogen atom of the alkenyl group is optionally substituted with a halogen atom), an alkynyl group having from 2 to 10 carbon atoms (at least one hydrogen atom of the alkynyl group is optionally substituted with a halogen atom), an aryl group (at least one hydrogen atom of the aryl group is optionally substituted with a halogen atom, an alkoxy group having from 1 to 6 carbon atoms, or an alkyl group having from 1 to 6 carbon atoms), an aralkyl group having from 7 to 16 carbon atoms (at least one hydrogen atom of an aromatic ring in the aralkyl group is optionally substituted with a halogen atom, an alkoxy group having from 1 to 6 carbon atoms, or an alkyl group having from 1 to 6 carbon atoms), or $R^1$ and $R^2$ represent a halogen atoms, and

$L^1$ and $L^2$ each represent a single bond or -O-, with a proviso that a case in which $R^1$ is a halogen atom and $L^1$ is -O-, a case in which $R^2$ is a halogen atom and $L^2$ is -O-, and a case in which $R^1$ and $R^2$ are both the above-described alkyl groups or the above-described aryl groups, and $L^1$ and $L^2$ are both single bonds, are excluded.

When the lithium (*N*-carbonyl)sulfonamide compound of the disclosure (i.e. compound (B)) is added to the nonaqueous electrolyte solution, an increase in the direct-current resistance and a decrease in the discharge capacity can be inhibited even if the lithium secondary battery is stored in a high-temperature environment.

The reason why this effect is exerted is presumed to be the same as the reason why an increase in the direct-current resistance and a decrease in the discharge capacity can be inhibited when the compound (A) is added to the nonaqueous electrolyte solution, even if the lithium secondary battery is stored in a high-temperature environment.

Regarding the compound (B), examples of the "alkyl group having from 1 to 10 carbon atoms" represented by each of $R^1$ and $R^2$ in Formula (I) are the same as those exemplified above as the "alkyl group having from 1 to 10 carbon atoms" in the compound (A).

Regarding the compound (B), examples of the "alkenyl group having from 2 to 10 carbon atoms" represented

by each of $R^1$ and $R^2$ in Formula (I) are the same as those exemplified above as the "alkenyl group having from 2 to 10 carbon atoms" in the compound (A).

Regarding the compound (B), examples of the "alkynyl group having from 2 to 10 carbon atoms" represented by each of $R^1$ and $R^2$ in Formula (I) are the same as those exemplified above as the "alkynyl group having from 2 to 10 carbon atoms" in the compound (A).

Regarding the compound (B), examples of the "aryl group" represented by each of $R^1$ and $R^2$ in Formula (I) are the same as those exemplified above as the "aryl group" in the compound (A).

Regarding the compound (B), the "aralkyl group having from 7 to 16 carbon atoms" represented by each of $R^1$ and $R^2$ in Formula (I) is an aralkyl group that contains an aryl group and has from 7 to 16 carbon atoms. At least one hydrogen atom of an aromatic ring in the aralkyl group is optionally substituted with a halogen atom, an alkoxy group having from 1 to 6 carbon atoms, or an alkyl group having from 1 to 6 carbon atoms.

The halogen atom is preferably a fluorine atom, a chlorine atom, a bromine atom, or an iodine atom, more preferably a fluorine atom, a chlorine atom, or a bromine atom, still more preferably a fluorine atom or a chlorine atom, particularly preferably a fluorine atom. In the "aralkyl group", the number of hydrogen atoms substituted with the halogen atom is not particularly limited, and it is preferably from 1 to 5.

In the alkoxy group having from 1 to 6 carbon atoms, its alkyl group may be in any of a linear form, a branched form, and a cyclic form. Examples of the alkoxy group having from 1 to 6 carbon atoms include a methoxy group, an ethoxy group, an n-propoxy group, an isopropoxy group, an n-butoxy group, a t-butoxy group, and a pentyloxy group. Thereamong, the alkoxy group having from 1 to 6 carbon atoms is preferably an alkoxy group having from 1 to 3 carbon atoms, more preferably a methoxy group or an ethoxy group. In the "aralkyl group", the number of hydrogen atoms substituted with the alkoxy group having from 1 to 6 carbon atoms is not particularly limited, and it is preferably from 1 to 3.

The alkyl group having from 1 to 6 carbon atoms may be in any of a linear form, a branched form, and a cyclic form. Examples of the alkyl group having from 1 to 6 carbon atoms include a methyl group, an ethyl group, an *n*-propyl group, an *i*-propyl group, an *n*-butyl group, an *i*-butyl group, a *t*-butyl group, an *n*-pentyl group, an *n*-hexyl group, and a cyclohexyl group. Thereamong, the alkyl group having from 1 to 6 carbon atoms is preferably an alkyl group having from 1 to 3 carbon atoms, more preferably a methyl group or an ethyl group. In the "aralkyl group", the number of hydrogen atoms substituted with the alkyl group having from 1 to 6 carbon atoms is not particularly limited, and it is preferably from 1 to 3.

The aralkyl group having from 7 to 16 carbon atoms is preferably an aralkyl group formed of an aryl group substituted with an alkylene group having from 1 to 6 carbon atoms. The aryl group substituted with an alkylene group having from 1 to 6 carbon atoms is preferably an aryl group having from 6 to 10 carbon atoms.

Specific examples of the "aralkyl group having from 7 to 16 carbon atoms" include a benzyl group, a phenylethyl group, and a naphthylmethyl group.

Regarding the compound (B), the "halogen atom" represented by each of $R^1$ and $R^2$ in Formula (I) is preferably a fluorine atom, a chlorine atom, a bromine atom, or an iodine atom, more preferably a fluorine atom, a chlorine atom, or a bromine atom, still more preferably a fluorine atom or a chlorine atom, particularly preferably a fluorine atom.

In Formula (I), $L^1$ and $L^2$ each represent a single bond or -O-. However, a case in which $R^1$ is a halogen atom and $L^1$ is -O-, a case in which $R^2$ is a halogen atom and $L^2$ is -O-, and a case in which $R^1$ and $R^2$ are both the above-described alkyl groups or the above-described aryl groups, and $L^1$ and $L^2$ are both single bonds, are excluded.

Particularly, $L^1$ preferably represents a single bond, and $L^2$ preferably represents -O-.

Specific examples of the lithium (*N*-carbonyl)sulfonamide compound (i.e. compound (B)) include Synthesis Compounds (I-1) to (I-48) that are synthesized in the below-described Examples.

[Additive for Lithium Secondary Battery]

(Additive (A))

The additive for a lithium secondary battery according to the disclosure (hereinafter, may be simply referred to as "additive") will now be described.

The additive of the disclosure contains a lithium (*N*-carbonyl)sulfonamide compound (I) represented by the following Formula (I) (hereinafter, may be referred to as "compound (C)").

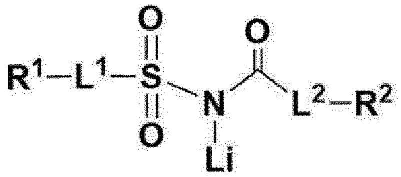

(I)

In Formula (I),

each of $R^1$ and $R^2$ represents an alkyl group having from 1 to 10 carbon atoms (at least one hydrogen atom of the alkyl group is optionally substituted with a halogen atom, with a proviso that a trifluoromethyl group is excluded), an alkenyl group having from 2 to 10 carbon atoms (at least one hydrogen atom of the alkenyl group is optionally substituted with a halogen atom), an alkynyl group having from 2 to 10 carbon atoms (at least one hydrogen atom of the alkynyl group is optionally substituted with a halogen atom), or an aryl group (at least one hydrogen atom of the aryl group is optionally substituted with a halogen atom, an alkoxy group having from 1 to 6 carbon atoms, or an alkyl group having from 1 to 6 carbon atoms); and
$L^1$ and $L^2$ each represent a single bond or -O-.

In the disclosure, the phrase "with a proviso that a trifluoromethyl group is excluded" regarding the alkyl group having from 1 to 10 carbon atoms indicates that a case in which $R^1$ is a trifluoromethyl group, $L^1$ is a single bond, $R^2$ is a trifluoromethyl group, and $L^2$ is a single bond is excluded from Formula (I).
Hereinafter, an additive containing the compound (C) may be referred to as "additive (A)".
The additive (A) of the disclosure contains a lithium (*N*-carbonyl)sulfonamide compound (I) (i.e. compound (C)); therefore, when it is added to the nonaqueous electrolyte solution, an increase in the direct-current resistance and a decrease in the discharge capacity can be inhibited even if the lithium secondary battery is stored in a high-temperature environment.
The reason why this effect is exerted is presumed to be the same as the reason why an increase in the direct-current resistance and a decrease in the discharge capacity can be inhibited when the lithium (*N*-carbonyl)sulfonamide compound (I) of the disclosure (i.e. compound (A)) is added to the nonaqueous electrolyte solution, even if the lithium secondary battery is stored in a high-temperature environment.
The additive (A) of the disclosure is suitable as an additive for a nonaqueous electrolyte solution of a lithium secondary battery.
Examples of $R^1$, $R^2$, $L^1$, and $L^2$ include the same ones as those exemplified above as $R^1$, $R^2$, $L^1$, and $L^2$ of the above-described lithium (*N*-carbonyl)sulfonamide compound (i.e. compound (A)), except that a trifluoromethyl group is excluded from $R^1$ and $R^2$, and that $L^1$ and $L^2$ may each be a single bond.

(Additive (B))

The additive of the disclosure may be the above-described additive for a lithium secondary battery (i.e. additive (A)) in which
$R^1$ and $R^2$ each represent, in place of the above-described alkyl group, the above-described alkenyl group, the above-described alkynyl group, or the above-described aryl group:

an alkyl group having from 1 to 10 carbon atoms (at least one hydrogen atom of the alkyl group is optionally substituted with a halogen atom), the above-described alkenyl group, the above-described alkynyl group, the above-described aryl group, an aralkyl group having from 7 to 16 carbon atoms (at least one hydrogen atom of an aromatic ring in the aralkyl group is optionally substituted with a halogen atom, an alkoxy group having from 1 to 6 carbon atoms, or an alkyl group having from 1 to 6 carbon atoms), or $R^1$ and $R^2$ represent a halogen atoms, and
a case in which $R^1$ is a halogen atom and $L^1$ is -O-, a case in which $R^2$ is a halogen atom and $L^2$ is -O-, and a case in which $R^1$ and $R^2$ are both the above-described alkyl groups or the above-described aryl groups, and $L^1$ and $L^2$ are both single bonds, are excluded.

In other words, the additive for a lithium secondary battery according to the disclosure may be an additive containing a lithium (*N*-carbonyl)sulfonamide compound (I) represented by the following Formula (I) (i.e. compound (B)) (this additive may be hereinafter referred to as "additive (B)").

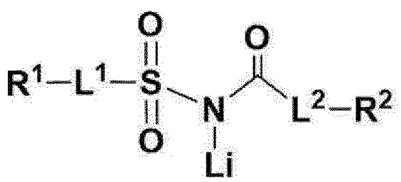

(I)

In Formula (I),

each of $R^1$ and $R^2$ represents an alkyl group having from 1 to 10 carbon atoms (at least one hydrogen atom of the alkyl group is optionally substituted with a halogen atom), an alkenyl group having from 2 to 10 carbon atoms (at least one hydrogen atom of the alkenyl group is optionally substituted with a halogen atom), an alkynyl group having from 2 to 10 carbon atoms (at least one hydrogen atom of the alkynyl group is optionally substituted with a halogen atom), an aryl group (at least one hydrogen atom of the aryl group is optionally substituted with a halogen atom, an alkoxy group having from 1 to 6 carbon atoms, or an alkyl group having from 1 to 6 carbon atoms), an aralkyl group having from 7 to 16 carbon atoms (at least one hydrogen atom of an aromatic ring in the aralkyl group is optionally substituted with a halogen atom, an alkoxy group having from 1 to 6 carbon atoms, or an alkyl group having from 1 to 6 carbon atoms), or $R^1$ and $R^2$ represent a halogen atoms, and

$L^1$ and $L^2$ each represent a single bond or -O-, with a proviso that a case in which $R^1$ is a halogen atom and $L^1$ is -O-, a case in which $R^2$ is a halogen atom and $L^2$ is -O-, and a case in which $R^1$ and $R^2$ are both the above-described alkyl groups or the above-described aryl groups, and $L^1$ and $L^2$ are both single bonds, are excluded.

The additive (B) of the disclosure contains a lithium (*N*-carbonyl)sulfonamide compound (I) (i.e. compound (B)); therefore, when it is added to the nonaqueous electrolyte solution, an increase in the direct-current resistance and a decrease in the discharge capacity can be inhibited even if the lithium secondary battery is stored in a high-temperature environment.

The reason why this effect is exerted is presumed to be the same as the reason why an increase in the direct-current resistance and a decrease in the discharge capacity can be inhibited when the additive (A) is added to the nonaqueous electrolyte solution, even if the lithium secondary battery is stored in a high-temperature environment.

The additive (B) of the disclosure is suitable as an additive for a nonaqueous electrolyte solution of a lithium secondary battery.

Examples of the lithium (*N*-carbonyl)sulfonamide compound (I) (i.e. compound (B)) contained in the additive (B) of the disclosure include the same ones as those exemplified above as the lithium (*N*-carbonyl)sulfonamide compound (I) (i.e. compound (B)) described above.

[Nonaqueous Electrolyte Solution for Lithium Secondary Battery]

(Nonaqueous Electrolyte Solution (A))

The nonaqueous electrolyte solution for a lithium secondary battery according to the disclosure will now be described.

The nonaqueous electrolyte solution of the disclosure is used as an electrolyte solution of a lithium secondary battery.

The nonaqueous electrolyte solution of the disclosure contains a lithium (*N*-carbonyl)sulfonamide compound (I) represented by the following Formula (I) (i.e. compound (C)).

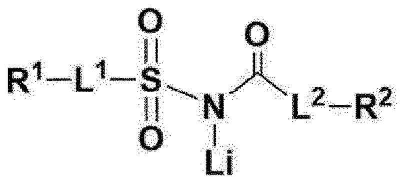

(I)

In Formula (I),

each of $R^1$ and $R^2$ represents an alkyl group having from 1 to 10 carbon atoms (at least one hydrogen atom of the alkyl group is optionally substituted with a halogen atom, with a proviso that a trifluoromethyl group is excluded), an alkenyl group having from 2 to 10 carbon atoms (at least one hydrogen atom of the alkenyl group is optionally substituted with a halogen atom), an alkynyl group having from 2 to 10 carbon atoms (at least one hydrogen atom of the alkynyl group is optionally substituted with a halogen atom), or an aryl group (at least one hydrogen atom of the aryl group is optionally substituted with a halogen atom, an alkoxy group having from 1 to 6 carbon atoms, or an alkyl group having from 1 to 6 carbon atoms); and

$L^1$ and $L^2$ each represent a single bond or -O-.

In the disclosure, the phrase "with a proviso that a trifluoromethyl group is excluded" indicates that a case in which $R^1$ is a trifluoromethyl group, $L^1$ is a single bond, $R^2$ is a trifluoromethyl group, and $L^2$ is a single bond is excluded from Formula (I).

Hereinafter, a nonaqueous electrolyte solution containing the compound (C) may be referred to as "nonaqueous electrolyte solution (A)".

The nonaqueous electrolyte solution (A) of the disclosure contains a lithium (*N*-carbonyl)sulfonamide compound (I) (i.e. compound (C)); therefore, it can inhibit an increase in the direct-current resistance and a decrease in the discharge capacity even if the lithium secondary battery is stored in a high-temperature environment.

The reason why this effect is exerted is presumed to be the same as the reason why an increase in the direct-current resistance and a decrease in the discharge capacity can be inhibited when the lithium (*N*-carbonyl)sulfonamide compound (I) of the disclosure (i.e. compound (A)) is added to the nonaqueous electrolyte solution, even if the lithium secondary battery is stored in a high-temperature environment.

Examples of the lithium (*N*-carbonyl)sulfonamide compound (I) (i.e. compound (C)) contained in the nonaqueous electrolyte solution (A) of the disclosure include the same ones as those exemplified above as the lithium (*N*-carbonyl)sulfonamide compound (I) (i.e. compound (C)) contained in the above-described additive (A) of the disclosure.

(Nonaqueous Electrolyte Solution (B))

The nonaqueous electrolyte solution of the disclosure may be the above-described nonaqueous electrolyte solution for a lithium secondary battery (i.e. nonaqueous electrolyte solution (A)) in which
each of $R^1$ and $R^2$ represents, in place of the above-described alkyl group, the above-described alkenyl group, the above-described alkynyl group, or the above-described aryl group:

an alkyl group having from 1 to 10 carbon atoms (at least one hydrogen atom of the alkyl group is optionally substituted with a halogen atom), the above-described alkenyl group, the above-described alkynyl group, the above-described aryl group, an aralkyl group having from 7 to 16 carbon atoms (at least one hydrogen atom of an aromatic ring in the aralkyl group is optionally substituted with a halogen atom, an alkoxy group having from 1 to 6 carbon atoms, or an alkyl group having from 1 to 6 carbon atoms), or $R^1$ and $R^2$ represent a halogen atoms, and
a case in which $R^1$ is a halogen atom and $L^1$ is -O-, a case in which $R^2$ is a halogen atom and $L^2$ is -O-, and a case in which $R^1$ and $R^2$ are both the above-described alkyl groups or the above-described aryl groups, and $L^1$ and $L^2$ are both single bonds, are excluded.

In other words, the nonaqueous electrolyte solution of the disclosure may be an additive containing a lithium (*N*-carbonyl)sulfonamide compound (I) represented by the following Formula (I) (i.e. compound (B)) (this additive may be hereinafter referred to as "nonaqueous electrolyte solution (B)").

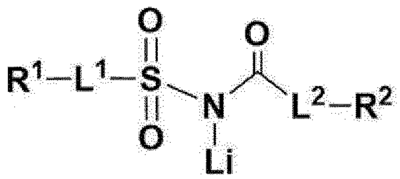

(I)

In Formula (I),

each of $R^1$ and $R^2$ represents an alkyl group having from 1 to 10 carbon atoms (at least one hydrogen atom of the alkyl group is optionally substituted with a halogen atom), an alkenyl group having from 2 to 10 carbon atoms (at least one hydrogen atom of the alkenyl group is optionally substituted with a halogen atom), an alkynyl group having from 2 to 10 carbon atoms (at least one hydrogen atom of the alkynyl group is optionally substituted with a halogen atom), an aryl group (at least one hydrogen atom of the aryl group is optionally substituted with a halogen atom, an alkoxy group having from 1 to 6 carbon atoms, or an alkyl group having from 1 to 6 carbon atoms), an aralkyl group having from 7 to 16 carbon atoms (at least one hydrogen atom of an aromatic ring in the aralkyl group is optionally substituted with a halogen atom, an alkoxy group having from 1 to 6 carbon atoms, or an alkyl group having from 1 to 6 carbon atoms), or $R^1$ and $R^2$ represent a halogen atoms, and
$L^1$ and $L^2$ each represent a single bond or -O-, with a proviso that a case in which $R^1$ is a halogen atom and $L^1$ is -O-, a case in which $R^2$ is a halogen atom and $L^2$ is -O-, and a case in which $R^1$ and $R^2$ are both the above-described alkyl groups or the above-described aryl groups, and $L^1$ and $L^2$ are both single bonds, are excluded.

The nonaqueous electrolyte solution (B) of the disclosure can inhibit an increase in the direct-current resistance and a decrease in the discharge capacity even if the lithium secondary battery is stored in a high-temperature environment.

The reason why this effect is exerted is presumed to be the same as the reason why an increase in the direct-current resistance and a decrease in the discharge capacity can be inhibited when the nonaqueous electrolyte solution (A) is added to the nonaqueous electrolyte solution, even if the lithium secondary battery is stored in a high-temperature environment.

Examples of the lithium (*N*-carbonyl)sulfonamide compound (I) (i.e. compound (B)) contained in the nonaqueous electrolyte solution (B) of the disclosure include the same ones as those exemplified above as the lithium (*N*-carbonyl)sulfonamide compound (I) (i.e. compound (B)) described above.

Hereinafter, the compound (C) or compound (D) is simply referred to as "lithium (*N*-carbonyl)sulfonamide compound (I)".

Hereinafter, the additive (A) or additive (B) is simply referred to as "additive".

Hereinafter, the nonaqueous electrolyte solution (A) or nonaqueous electrolyte solution (B) is simply referred to as "nonaqueous electrolyte solution".

(Aryl Group-Containing Compound)

The lithium (*N*-carbonyl)sulfonamide compound (I) is preferably an aryl group-containing compound.

When the nonaqueous electrolyte solution of the disclosure is an aryl group-containing compound, the nonaqueous electrolyte solution can further inhibit an increase in the direct-current resistance and a decrease in the discharge capacity even when the lithium secondary battery is stored in a high-temperature environment.

The aryl group-containing compound is represented by Formula (I) wherein

$R^1$ represents an aryl group (at least one hydrogen atom of the aryl group is optionally substituted with a halogen atom, an alkoxy group having from 1 to 6 carbon atoms, or an alkyl group having from 1 to 6 carbon atoms);
$L^1$ represents a single bond;
$R^2$ represents an alkyl group having from 1 to 10 carbon atoms (at least one hydrogen atom of the alkyl group is optionally substituted with a halogen atom), an alkenyl group having from 2 to 10 carbon atoms (at least one hydrogen atom of the alkenyl group is optionally substituted with a halogen atom), an alkynyl group having from 2 to 10 carbon atoms (at least one hydrogen atom of the alkynyl group is optionally substituted with a halogen atom), an aryl group (at least one hydrogen atom of the aryl group is optionally substituted with a halogen atom, an alkoxy group having from 1 to 6 carbon atoms, or an alkyl group having from 1 to 6 carbon atoms), or an aralkyl group having from 7 to 16 carbon atoms (at least one hydrogen atom of an aromatic ring in the aralkyl group is optionally substituted with a halogen atom, an alkoxy group having from 1 to 6 carbon atoms, or an alkyl group having from 1 to 6 carbon atoms); and
$L^2$ represents -O-.

Specific examples of the aryl group-containing compound include Synthesis Compounds (I-1) to (I-9), (I-24), (I-25), (I-30) to (I-32), (I-38) to (I-40), (I-47), and (I-48) that are synthesized in the below-described Examples.

(Alkyl Group-Containing Compound)

The lithium (*N*-carbonyl)sulfonamide compound (I) is preferably an alkyl group-containing compound.

When the nonaqueous electrolyte solution of the disclosure is an alkyl group-containing compound, the nonaqueous electrolyte solution can further inhibit an increase in the direct-current resistance and a decrease in the discharge capacity even when the lithium secondary battery is stored in a high-temperature environment.

The alkyl group-containing compound is represented by Formula (I) wherein

$R^1$ represents an alkyl group having from 1 to 10 carbon atoms (at least one hydrogen atom of the alkyl group is optionally substituted with a halogen atom);
$L^1$ represents a single bond;
$R^2$ represents an alkyl group having from 1 to 10 carbon atoms (at least one hydrogen atom of the alkyl group is optionally substituted with a halogen atom), an alkenyl group having from 2 to 10 carbon atoms (at least one hydrogen atom of the alkenyl group is optionally substituted with a halogen atom), an alkynyl group having from 2 to 10 carbon atoms (at least one hydrogen atom of the alkynyl group is optionally substituted with a halogen atom), an aryl group (at least one hydrogen atom of the aryl group is optionally substituted with a halogen atom, an alkoxy group having from 1 to 6 carbon atoms, or an alkyl group having from 1 to 6 carbon atoms), or an aralkyl group having from 7 to 16 carbon atoms (at least one hydrogen atom of an aromatic ring in the aralkyl group is optionally substituted with a halogen atom, an alkoxy group having from 1 to 6 carbon atoms, or an alkyl group having from 1 to 6 carbon atoms); and
$L^2$ represents -O-.

Specific examples of the alkyl group-containing compound include Synthesis Compounds (I-10) to (I-23), (I-26) to (I-29), (I-33) to (I-37), and (I-41) that are synthesized in the below-described Examples.

(Fluorine Atom-Containing Compound)

The lithium (*N*-carbonyl)sulfonamide compound (I) is preferably a fluorine atom-containing compound.
When the nonaqueous electrolyte solution of the disclosure contains a fluorine atom-containing compound, the nonaqueous electrolyte solution can further inhibit an increase in the direct-current resistance and a decrease in the discharge capacity even when the lithium secondary battery is stored in a high-temperature environment.
The fluorine atom-containing compound is represented by Formula (I) wherein

$R^1$ represents a fluorine atom;
$L^1$ represents a single bond;
$R^2$ represents an alkyl group having from 1 to 10 carbon atoms (at least one hydrogen atom of the alkyl group is optionally substituted with a halogen atom), an alkenyl group having from 2 to 10 carbon atoms (at least one hydrogen atom of the alkenyl group is optionally substituted with a halogen atom), an alkynyl group having from 2 to 10 carbon atoms (at least one hydrogen atom of the alkynyl group is optionally substituted with a halogen atom), an aryl group (at least one hydrogen atom of the aryl group is optionally substituted with a halogen atom, an alkoxy group having from 1 to 6 carbon atoms, or an alkyl group having from 1 to 6 carbon atoms), or an aralkyl group having from 7 to 16 carbon atoms (at least one hydrogen atom of an aromatic ring in the aralkyl group is optionally substituted with a halogen atom, an alkoxy group having from 1 to 6 carbon atoms, or an alkyl group having from 1 to 6 carbon atoms); and
$L^2$ represents -O-.

Specific examples of the fluorine atom-containing compound include Synthesis Compounds (I-42) to (I-46) that are synthesized in the below-described Examples.
From the standpoint of further improving the dissociation of an electrolyte and the ion mobility, the nonaqueous electrolyte solution preferably has an intrinsic viscosity of 10.0 mPa·s or less at 25°C.
When the nonaqueous electrolyte solution recovered by disassembling a lithium secondary battery is actually analyzed, the amount of the lithium (*N*-carbonyl)sulfonamide compound (I) therein may be less than the amount added to the nonaqueous electrolyte solution. Even in such a case, as long as the lithium (*N*-carbonyl)sulfonamide compound (I) is detected even in a small amount in the nonaqueous electrolyte solution extracted from the lithium secondary battery, the electrolyte solution of this lithium secondary battery is included in the scope of the nonaqueous electrolyte solution of the disclosure.
A content of the lithium (*N*-carbonyl)sulfonamide compound (I) is preferably from 0.01% by mass to 5.0% by mass, more preferably from 0.05% by mass to 3.0% by mass, still more preferably from 0.10% by mass to 1.5% by mass, particularly preferably from 0.20% by mass to 1.5% by mass, with respect to a total amount of the nonaqueous electrolyte solution.
As long as the content of the lithium (*N*-carbonyl)sulfonamide compound (I) is in this range, a lithium secondary battery can be operated without deterioration of the lithium cation conductivity caused by an SEI film. In addition, with the SEI film containing a phosphate structure, the battery characteristics of the lithium secondary battery are improved.
As long as the content of the lithium (*N*-carbonyl)sulfonamide compound (I) is in the above-described range, the SEI film contains a sufficient amount of a structure derived from the lithium (*N*-carbonyl)sulfonamide compound (I). This makes a thermally and chemically stable inorganic salt ormacromolecular structure more likely to be formed. Therefore, in a high-temperature environment, for example, elution of a component of the SEI film and modification of the SEI film, which impair the durability of the SEI film, are unlikely to occur. As a result, the durability of the SEI film and the characteristics of the lithium secondary battery after high-temperature storage are improved.

<Lithium Fluorophosphate Compound (II)>

The nonaqueous electrolyte solution of the disclosure preferably contains a compound (II) that is at least one selected from the group consisting of lithium monofluorophosphate and lithium difluorophosphate (hereinafter, may be referred to as "lithium fluorophosphate compound (II)").
Lithium difluorophosphate is represented by the following Formula (II-1), and lithium monofluorophosphate is represented by the following Formula (II-2).

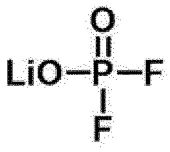

(II-1)

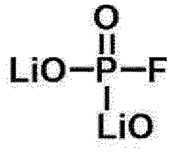

(II-2)

The nonaqueous electrolyte solution of the disclosure contains the lithium fluorophosphate compound (II) in addition to the lithium (*N*-carbonyl)sulfonamide compound (I), and thereby further inhibits a decrease in the discharge capacity and an increase in the direct-current resistance of a lithium secondary battery even in a charge-discharge cycle performed after storage of the lithium secondary battery in a high-temperature environment.

When the nonaqueous electrolyte solution contains the lithium fluorophosphate compound (II), a content thereof is preferably from 0.001% by mass to 5% by mass, more preferably from 0.01% by mass to 3% by mass, still more preferably from 0.1% by mass to 2% by mass, with respect to a total amount of the nonaqueous electrolyte solution.

As long as the content of the lithium fluorophosphate compound (II) is in this range, not only the solubility of lithium fluorophosphate in a nonaqueous solvent can be ensured, but also the direct-current resistance of a lithium secondary battery can be further reduced.

The nonaqueous electrolyte solution of the disclosure preferably contains a compound (III) represented by the following Formula (III) (hereinafter, referred to as "cyclic dicarbonyl compound (III)").

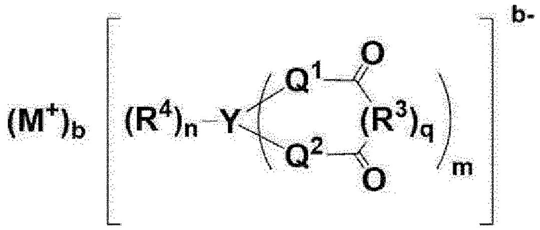

( III )

In Formula (III):

M represents an alkali metal,
Y represents a transition element, or an element of Group 13, 14, or 15 of the periodic table,
b represents an integer from 1 to 3,
m represents an integer from 1 to 4,
n represents an integer from 0 to 8,
q represents 0 or 1,
$R^3$ represents an alkylene group having from 1 to 10 carbon atoms, a halogenated alkylene group having from 1 to 10 carbon atoms, an arylene group having from 6 to 20 carbon atoms, or a halogenated arylene group having from 6 to 20 carbon atoms, the halogenated alkylene group, the arylene group and the halogenated arylene group each optionally containing a substituent or a heteroatom in a structure thereof and, when q is 1 and m is from 2 to 4, m instances of $R^3$ are optionally bound to each other,
$R^4$ represents a halogen atom, an alkyl group having from 1 to 10 carbon atoms, a halogenated alkyl group having from 1 to 10 carbon atoms, an aryl group having from 6 to 20 carbon atoms, or a halogenated aryl group having from 6 to 20 carbon atoms, the halogenated alkyl group, the aryl group and the halogenated aryl group each optionally containing a substituent or a heteroatom in a structure thereof and, when n is from 2 to 8, n instances of $R^4$ are optionally bound to each other to form a ring, and
each of $Q^1$ and $Q^2$ independently represents an oxygen atom or a carbon atom.

The nonaqueous electrolyte solution of the disclosure contains the cyclic dicarbonyl compound (III) in addition to the lithium (*N*-carbonyl)sulfonamide compound (I), and thereby further inhibits a decrease in the discharge capacity and an increase in the direct-current resistance of a lithium secondary battery even in a charge-discharge cycle performed after storage of the lithium secondary battery in a high-temperature environment.

It is presumed that this effect is exerted because of the following.

When the nonaqueous electrolyte solution contains the cyclic dicarbonyl compound (III) in addition to the lithium (*N*-carbonyl)sulfonamide compound (I), the SEI film and the like can contain therein bonds derived from the cyclic

dicarbonyl compound (III) in addition to the above-described reaction product and the like. This makes a thermally and chemically stable inorganic salt or macromolecular structure more likely to be formed. Therefore, in a high-temperature environment, for example, elution of a component of the SEI film and the like and modification of the SEI film and the like, which impair the durability of the SEI film and the like, are unlikely to occur. As a result, a decrease in the discharge capacity and an increase in the direct-current resistance of a lithium secondary battery are further inhibited even in a charge-discharge cycle performed after long-term storage of the lithium secondary battery in a high-temperature environment.

M represents an alkali metal. Examples of the alkali metal include lithium, sodium, and potassium. Thereamong, M is preferably lithium.

Y represents a transition element, or an element of Group 13, 14, or 15 of the periodic table. Y is preferably Al, B, V, Ti, Si, Zr, Ge, Sn, Cu, Y, Zn, Ga, Nb, Ta, Bi, P, As, Sc, Hf, or Sb, more preferably Al, B, or P. When Y is Al, B, or P, an anionic compound is synthesized relatively easily, so that the production cost can be reduced.

Further, b represents the valence of an anion or the number of cations, which is an integer from 1 to 3, preferably 1. When b is 3 or less, a salt of an anionic compound can be easily dissolved in a mixed organic solvent.

Each of m and n represents a value relating to the number of ligands, and is determined based on the type of M. The value of m is an integer from 1 to 4, and the value of n is an integer from 0 to 8.

Moreover, q represents 0 or 1. When q is 0, the chelate ring is a five-membered ring, while when q is 1, the chelate ring is a six-membered ring.

$R^3$ represents an alkylene group having from 1 to 10 carbon atoms, a halogenated alkylene group having from 1 to 10 carbon atoms, an arylene group having from 6 to 20 carbon atoms, or a halogenated arylene group having from 6 to 20 carbon atoms. These alkylene group, halogenated alkylene group, arylene group, and halogenated arylene group may each contain a substituent or a heteroatom in their structures. Specifically, $R^3$ may contain a substituent in place of a hydrogen atom of any of these groups. Examples of the substituent include a halogen atom, a linear or cyclic alkyl group, an aryl group, an alkenyl group, an alkoxy group, an aryloxy group, a sulfonyl group, an amino group, a cyano group, a carbonyl group, an acyl group, an amide group, and a hydroxy group. $R^3$ may have a structure in which a nitrogen atom, a sulfur atom, or an oxygen atom is introduced in place of a carbon atom of any of these groups. When q is 1 and m is from 2 to 4, m $R^3$s may be bound to each other. Examples of such a case include ligands such as ethylenediaminetetraacetic acid.

$R^4$ represents a halogen atom, an alkyl group having from 1 to 10 carbon atoms, a halogenated alkyl group having from 1 to 10 carbon atoms, an aryl group having from 6 to 20 carbon atoms, or a halogenated aryl group having from 6 to 20 carbon atoms. These alkyl group, halogenated alkyl group, aryl group, and halogenated aryl group, similarly to the case of $R^3$, may each contain a substituent or a heteroatom in their structures and, when n is from 2 to 8, n $R^4$s may be bound to each other to form a ring. $R^4$ is preferably an electron-withdrawing group, particularly preferably a fluorine atom. Each of $Q^1$ and $Q^2$ independently represents O or S. In other words, a ligand is bound to Y via these heteroatoms.

Specific examples of the cyclic dicarbonyl compound (III) include compounds represented by the following Formulae (III-1) and (III-2).

The compound represented by Formula (III-1) may be hereinafter referred to as "lithium bis(oxalato)borate (III-1)".

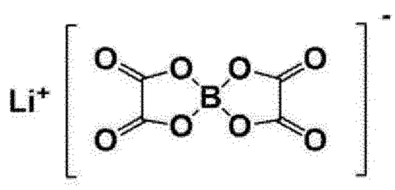

(III-1)

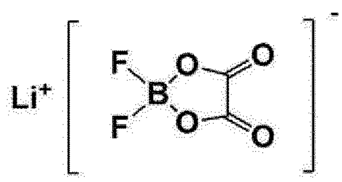

(III-2)

When the nonaqueous electrolyte solution contains the cyclic dicarbonyl compound (III), a content thereof is preferably from 0.01% by mass to 10% by mass, more preferably from 0.05% by mass to 5.0% by mass, still more preferably from 0.10% by mass to 3.0% by mass, and particularly preferably from 0.10% by mass to 2.0% by mass, with respect to a total amount of the nonaqueous electrolyte solution.

As long as the content of the cyclic dicarbonyl compound (III) is in this range, a lithium secondary battery can be operated without deterioration of the lithium cation conductivity caused by an SEI film and the like. In addition, with the SEI film and the like containing a cyclic dicarbonyl structure, the battery characteristics of the lithium secondary battery are improved.

As long as the content of the cyclic dicarbonyl compound (III) is in the above-described range, the SEI film and the like contain a sufficient amount of a structure mainly composed of the cyclic dicarbonyl structure. This makes a thermally and chemically stable inorganic salt or macromolecular structure more likely to be formed. Therefore, in a high-temperature environment, for example, elution of a component of the SEI film and the like and modification of the SEI film and the like, which impair the durability of the SEI film and the like, are unlikely to occur. As a result, the durability of the SEI film and the like, as well as the characteristics of the lithium secondary battery after high-temperature storage are improved.

The nonaqueous electrolyte solution of the disclosure preferably contains a compound (IV) represented by the following Formula (IV) (hereinafter, referred to as "cyclic sulfur-containing ester compound (IV)").

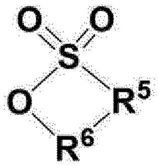 ( IV ) 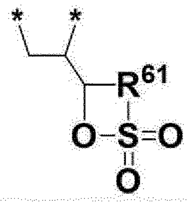 ( iv-1 ) 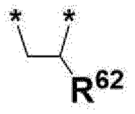 ( iv-2 )

In Formula (IV),

$R^5$ represents an oxygen atom, an alkylene group having from 1 to 6 carbon atoms, or an alkenylene group having from 2 to 6 carbon atoms;

$R^6$ represents an alkylene group having from 1 to 6 carbon atoms, an alkenylene group having from 2 to 6 carbon atoms, a group represented by Formula (iv-1), or a group represented by Formula (iv-2);

* represents a bonding position;

in Formula (iv-1), $R^{61}$ represents an oxygen atom, an alkylene group having from 1 to 6 carbon atoms, an alkenylene group having from 2 to 6 carbon atoms, or an oxymethylene group; and

in Formula (iv-2), $R^{62}$ represents an alkyl group having from 1 to 6 carbon atoms, or an alkenyl group having from 2 to 6 carbon atoms.

The nonaqueous electrolyte solution of the disclosure contains the cyclic sulfur-containing ester compound (IV) in addition to the lithium (*N*-carbonyl)sulfonamide compound (I), and can thereby inhibit a decrease in the discharge capacity and an increase in the direct-current resistance of a lithium secondary battery even when the lithium secondary battery is stored in a high-temperature environment over an extended period.

It is presumed that this effect is exerted because of the following.

When a lithium secondary battery is produced using the nonaqueous electrolyte solution of the disclosure, in the production process (e.g., the below-described aging step), a reaction product contains a product generated by a reaction between the cyclic sulfur-containing ester compound (IV) and a compound (e.g., LiF) generated from an electrolyte. By this, the stability of the lithium secondary battery in a high-temperature environment is improved. It is believed that, as a result, an increase in the direct-current resistance of the lithium secondary battery is further inhibited even when the lithium secondary battery is stored in a high-temperature environment. In addition, the progress of a decomposition reaction of the nonaqueous electrolyte solution is further inhibited. It is believed that, as a result, a decrease in the discharge capacity of the lithium secondary battery is made unlikely to occur even when the lithium secondary battery is stored in a high-temperature environment.

In Formula (IV), $R^5$ is preferably an alkylene group having from 2 to 3 carbon atoms, a vinylene group, or an oxygen atom, more preferably a trimethylene group, a vinylene group, or an oxygen atom, particularly preferably an oxygen atom.

In the cyclic sulfur-containing ester compound (IV), $R^5$ is preferably an oxygen atom. This makes a thermally and chemically stable inorganic salt structure more likely to be formed. Therefore, in a high-temperature environment, for example, elution of a component of the SEI film and the like and modification of the SEI film and the like, which impair the durability of the SEI film and the like, are unlikely to occur. As a result, the durability of the SEI film and the like, as well as the battery characteristics of the lithium secondary battery are improved.

In Formula (IV), $R^6$ is preferably a group represented by Formula (iv-1) or a group represented by Formula (iv-2).

In Formula (iv-1), $R^{61}$ is preferably an alkylene group having from 1 to 3 carbon atoms, an alkenylene group having from 1 to 3 carbon atoms, or an oxymethylene group, more preferably an oxymethylene group.

In Formula (iv-2), $R^{62}$ is preferably an alkyl group having from 1 to 3 carbon atoms or an alkenyl group having from 2 to 3 carbon atoms, more preferably a propyl group.

Specific examples of the cyclic sulfur-containing ester compound (IV) include compounds represented by Formulae (IV-1) to (IV-4).

Hereinafter, the compound represented by Formula (IV-1) may be referred to as "cyclic sulfur-containing ester compound (IV-1)".

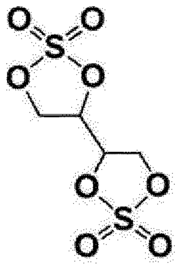

(IV-1)

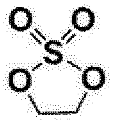

(IV-2)

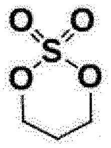

(IV-3)

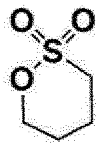

(IV-4)

The nonaqueous electrolyte solution may contain the cyclic sulfur-containing ester compound (IV) singly, or in combination of two or more kinds thereof.

When the nonaqueous electrolyte solution contains the cyclic sulfur-containing ester compound (IV), a content thereof is preferably from 0.01% by mass to 5.0% by mass, more preferably from 0.05% by mass to 3.0% by mass, still more preferably from 0.10% by mass to 2.0% by mass, with respect to a total amount of the nonaqueous electrolyte solution.

As long as the content of the cyclic sulfur-containing ester compound (IV) is in this range, a lithium secondary battery can be operated without deterioration of the lithium ion conductivity caused by an SEI film and the like. In addition, with the SEI film and the like containing a cyclic sulfur-containing ester structure, the battery characteristics of the lithium secondary battery are improved.

As long as the content of the cyclic sulfur-containing ester compound (IV) is in the above-described range, the SEI film and the like contain a sufficient amount of a cyclic sulfur-containing ester structure. This makes a thermally and chemically stable inorganic salt or macromolecular structure more likely to be formed. Therefore, in a high-temperature environment, for example, elution of a component of the SEI film and the like and modification of the SEI film and the like, which impair the durability of the SEI film and the like, are unlikely to occur. As a result, the durability of the SEI film and the like, as well as the battery characteristics of the lithium secondary battery are improved.

<Other Additives>

The nonaqueous electrolyte solution of the disclosure may also contain other additives.

The other additives are not particularly limited, and any known additives may be used as desired.

As the other additives, for example, the additives described in the paragraphs [0042] to [0055] of JP-A No. 2019-153443 can be used.

<Nonaqueous Solvent>

The nonaqueous electrolyte solution generally contains a nonaqueous solvent. The nonaqueous solvent can be selected as appropriate from various known solvents. The nonaqueous solvent may be contained singly, or in combination of two or more kinds thereof.

Examples of the nonaqueous solvent include cyclic carbonates, fluorine-containing cyclic carbonates, chain carbonates, fluorine-containing chain carbonates, aliphatic carboxylic acid esters, fluorine-containing aliphatic carboxylic acid esters, γ-lactones, fluorine-containing γ-lactones, cyclic ethers, fluorine-containing cyclic ethers, chain ethers, fluorine-containing chain ethers, nitriles, amides, lactams, nitromethane, nitroethane, sulfolane, trimethyl phosphate, dimethyl sulfoxide, and dimethyl sulfoxide phosphate.

Examples of the cyclic carbonates include ethylene carbonate (EC), propylene carbonate (PC), and butylene carbonate (BC).

Examples of the fluorine-containing cyclic carbonates include fluoroethylene carbonate (FEC).

Examples of the chain carbonates include dimethyl carbonate (DMC), diethyl carbonate (DEC), ethyl methyl carbonate (EMC), methyl propyl carbonate (MPC), ethyl propyl carbonate (EPC), and dipropyl carbonate (DPC).

Examples of the aliphatic carboxylic acid esters include methyl formate, methyl acetate, methyl propionate, methyl butyrate, methyl isobutyrate, methyl trimethylbutyrate, ethyl formate, ethyl acetate, ethyl propionate, ethyl butyrate, ethyl isobutyrate, and ethyl trimethylbutyrate.

Examples of the γ-lactones include γ-butyrolactone and γ-valerolactone.

Examples of the cyclic ethers include tetrahydrofuran, 2-methyltetrahydrofuran, tetrahydropyran, 1,3-dioxolane, 4-methyl-1,3-dioxolane, 1,3-dioxane, and 1,4-dioxane.

Examples of the chain ethers include 1,2-ethoxyethane (DEE), ethoxymethoxyethane (EME), diethyl ether, 1,2-dimethoxyethane, and 1,2-dibutoxyethane.

Examples of the nitriles include acetonitrile, glutaronitrile, adiponitrile, methoxyacetonitrile, and 3-methoxypropionitrile.

Examples of the amides include *N,N*-dimethylformamide.

Examples of the lactams include *N*-methylpyrrolidinone, *N*-methyloxazolidinone, and *N,N'*-dimethylimidazolidinone.

The nonaqueous solvent preferably contains at least one selected from the group consisting of cyclic carbonates, fluorine-containing cyclic carbonates, chain carbonates, and fluorine-containing chain carbonates.

In this case, a total ratio of the cyclic carbonates, the fluorine-containing cyclic carbonates, the chain carbonates, and the fluorine-containing chain carbonates is preferably from 50% by mass to 100% by mass, more preferably from 60% by mass to 100% by mass, still more preferably from 80% by mass to 100% by mass, with respect to a total amount of the nonaqueous solvent.

The nonaqueous solvent preferably contains at least one selected from the group consisting of cyclic carbonates and chain carbonates.

In this case, a total ratio of the cyclic carbonates and the chain carbonates in the nonaqueous solvent is preferably from 50% by mass to 100% by mass, more preferably from 60% by mass to 100% by mass, still more preferably from 80% by mass to 100% by mass, with respect to a total amount of the nonaqueous solvent.

A content of the nonaqueous solvent is preferably from 60% by mass to 99% by mass, more preferably from 70% by mass to 97% by mass, still more preferably from 70% by mass to 90% by mass, with respect to a total amount of the nonaqueous electrolyte solution.

From the standpoint of further improving the dissociation of an electrolyte and the ion mobility, the nonaqueous solvent preferably has an intrinsic viscosity of 10.0 mPa-s or less at 25°C.

<Electrolyte>

The nonaqueous electrolyte solution generally contains an electrolyte.

The "electrolyte" for a lithium secondary battery refers to a substance responsible for carrier transport between a positive electrode and a negative electrode. The electrolyte is highly soluble in the nonaqueous solvent, and has a high degree of dissociation in the nonaqueous solvent. As such an electrolyte, a lithium salt is used in many cases.

The electrolyte preferably contains at least one of a lithium salt containing fluorine (hereinafter, may be referred to as "fluorine-containing lithium salt") or a lithium salt not containing fluorine.

Examples of the fluorine-containing lithium salt include inorganic acid anion salts and organic acid anion salts.

Examples of the inorganic acid anion salts include lithium hexafluorophosphate ($LiPF_6$), lithium tetrafluoroborate ($LiBF_4$), lithium hexafluoroarsenate ($LiAsF_6$), and lithium hexafluorotantalate ($LiTaF_6$).

Examples of the organic acid anion salts include lithium trifluoromethane sulfonate ($LiCF_3SO_3$), lithium bis(trifluoromethanesulfonyl)imide ($Li(CF_3SO_2)_2N$), and lithium bis(pentafluoroethanesulfonyl)imide ($Li(C_2F_5SO_2)_2N$).

Thereamong, the fluorine-containing lithium salt is preferably a lithium salt other than the lithium (*N*-carbonyl)sulfonamide compound (I) represented by Formula (I). In other words, the fluorine-containing lithium salt is preferably at least one selected from the group consisting of lithium hexafluorophosphate ($LiPF_6$), lithium tetrafluoroborate ($LiBF_4$), lithium hexafluoroarsenate ($LiAsF_6$), lithium hexafluorotantalate ($LiTaF_6$), lithium trifluoromethane sulfonate ($LiCF_3SO_3$), lithium bis(trifluoromethanesulfonyl)imide ($Li(CF_3SO_2)_2N$), and lithium bis(pentafluoroethanesulfonyl)imide ($Li(C_2F_5SO_2)_2N$). The fluorine-containing lithium salt is particularly preferably lithium hexafluorophosphate ($LiPF_6$).

Examples of the lithium salt not containing fluorine include lithium perchlorate (LiClO4), lithium tetrachloroaluminate ($LiAlCl_4$), and lithium decachlorodecaborate ($Li_2B_{10}Cl_{10}$).

When the electrolyte contains a fluorine-containing lithium salt, a content ratio of the fluorine-containing lithium salt is preferably from 50% by mass to 100% by mass, more preferably from 60% by mass to 100% by mass, still more preferably from 80% by mass to 100% by mass or less, with respect to a total amount of the electrolyte.

When the fluorine-containing lithium salt contains lithium hexafluorophosphate ($LiPF_6$), a content ratio of lithium hexafluorophosphate ($LiPF_6$) is preferably from 50% by mass to 100% by mass, more preferably from 60% by mass to 100% by mass, still more preferably from 80% by mass to 100% by mass or less, with respect to a total amount of the electrolyte.

When the nonaqueous electrolyte solution contains an electrolyte, the concentration of the electrolyte in the nonaqueous electrolyte solution is preferably from 0.1 mol/L to 3 mol/L, more preferably from 0.5 mol/L to 2 mol/L.

When the nonaqueous electrolyte solution contains lithium hexafluorophosphate ($LiPF_6$), the concentration of

lithium hexafluorophosphate ($LiPF_6$) in the nonaqueous electrolyte solution is preferably from 0.1 mol/L to 3 mol/L, more preferably from 0.5 mol/L to 2 mol/L.

<Other Components>

The nonaqueous electrolyte solution may also contain other components if necessary. Examples of the other components include acid anhydrides.

[Lithium Secondary Battery Precursor]

Next, the lithium secondary battery precursor of the disclosure will be described.

The lithium secondary battery precursor of the disclosure includes a casing, a positive electrode, a negative electrode, a separator, and an electrolyte solution. The positive electrode, the negative electrode, the separator, and the electrolyte solution are housed in the casing. The positive electrode is configured to occlude and release lithium ions. The negative electrode is configured to occlude and release lithium ions. The electrolyte solution is the nonaqueous electrolyte solution of the disclosure.

The lithium secondary battery precursor represents a lithium secondary battery that has not yet been subjected to charging or discharging. In other words, in the lithium secondary battery precursor, the negative electrode does not include a negative electrode SEI film, and the positive electrode does not include a positive electrode SEI film.

<Casing>

The shape and the like of the casing are not particularly limited, and are selected as appropriate in accordance with the intended use and the like of the lithium secondary battery precursor of the disclosure. Examples of the casing include a casing that includes a laminated film, and a casing composed of a battery can and a battery can lid.

<Positive Electrode>

The positive electrode is configured to occlude and release lithium ions. The positive electrode preferably contains at least one positive electrode active material configured to occlude and release lithium ions.

The positive electrode includes a positive electrode current collector and a positive electrode mixture layer. The positive electrode mixture layer is arranged on at least a portion of the surface of the positive electrode current collector.

A material of the positive electrode current collector is, for example, a metal or an alloy. More particularly, examples of the material of the positive electrode current collector include aluminum, nickel, stainless steel (SUS), and copper. Thereamong, aluminum is preferred from the standpoint of the balance between the degree of conductivity and the cost. The term "aluminum" used herein means pure aluminum or an aluminum alloy. The positive electrode current collector is preferably an aluminum foil. A material of the aluminum foil is not particularly limited, and examples thereof include A1085 and A3003.

The positive electrode mixture layer contains a positive electrode active material and a binder.

The positive electrode active material is not particularly limited as long as it is a substance configured to occlude and release lithium ions, and may be adjusted as appropriate in accordance with the intended use and the like of the lithium secondary battery precursor.

Examples of the positive electrode active material include a first oxide and a second oxide. The first oxide contains lithium (Li) and nickel (Ni) as constituent metal elements. The second oxide contains Li, Ni, and at least one metal element other than Li and Ni as constituent metal elements. Examples of the metal element other than Li and Ni include transition metal elements and main-group metal elements. The second oxide contains the metal element other than Li and Ni preferably at a ratio equivalent to or lower than that of Ni in terms of the number of atoms. The metal element other than Li and Ni may be, for example, at least one selected from the group consisting of Co, Mn, Al, Cr, Fe, V, Mg, Ca, Na, Ti, Zr, Nb, Mo, W, Cu, Zn, Ga, In, Sn, La, and Ce. These positive electrode active materials may be used singly, or in combination of two or more kinds thereof.

The positive electrode active material preferably contains a lithium-containing composite oxide represented by the following Formula (C1) (hereinafter, may be referred to as "NCM"). The lithium-containing composite oxide (C1) is advantageous in that it has a high energy density per unit volume and excellent thermal stability.

$$LiNi_aCo_bMn_cO_2 \qquad (C1)$$

In Formula (C1), each of a, b, and c independently represents a number larger than 0 but smaller than 1, and

a sum of a, b, and c is from 0.99 to 1.00.

Specific examples of the NCM include $LiNi_{0.33}Co_{0.33}Mn_{0.33}O_2$, $LiNi_{0.5}Co_{0.3}Mn_{0.2}O_2$, $LiNi_{0.5}Co_{0.2}Mn_{0.3}O_2$, $LiNi_{0.6}Co_{0.2}Mn_{0.2}O_2$, and $LiNi_{0.8}Co_{0.1}Mn_{0.1}O_2$.

The positive electrode active material may also contain a lithium-containing composite oxide represented by the following Formula (C2) (hereinafter, may be referred to as "NCA").

$$Li_tNi_{1-x-y}Co_xAl_yO_2 \qquad (C2)$$

In Formula (C2), t represents a number of from 0.95 to 1.15, x represents a number of from 0 to 0.3, y represents a number of from 0.1 to 0.2, and a sum of x and y is less than 0.5.

Specific example of the NCA include $LiNi_{0.8}Co_{0.15}Al_{0.05}O_2$.

When the positive electrode in the lithium secondary battery precursor of the disclosure includes a positive electrode current collector and a positive electrode mixture layer that contains a positive electrode active material and a binder, a content of the positive electrode active material in the positive electrode mixture layer is preferably from 10% by mass to 99.9% by mass, more preferably from 30% by mass to 99.0% by mass, still more preferably from 50% by mass to 99.0% by mass, particularly preferably from 70% by mass to 99.0% by mass, with respect to a total amount of the positive electrode mixture layer.

Examples of the binder include polyvinyl acetate, polymethyl methacrylate, nitrocellulose, fluorine resins, and rubber particles. Examples of the fluorine resins include polytetrafluoroethylenes (PTFE), polyvinylidene fluorides (PVDF), tetrafluoroethylene-hexafluoropropylene copolymers (FEP), and vinylidene fluoride-hexafluoropropylene copolymers. Examples of the rubber particles include styrene-butadiene rubber particles and acrylonitrile rubber particles. Thereamong, from the standpoint of improving the oxidation resistance of the positive electrode mixture layer, the binder is preferably a fluorine resin. The above-described binders may be used singly, or in combination of two or more kinds thereof if necessary.

From the standpoint of satisfying both the physical properties of the positive electrode mixture layer (e.g., electrolyte solution permeability and peeling strength) and the battery performance, the content of the binder in the positive electrode mixture layer is preferably from 0.1% by mass to 4% by mass with respect to a total amount of the positive electrode mixture layer. When the content of the binder is 0.1 % by mass or more, the adhesion of the positive electrode mixture layer to the positive electrode current collector and the bindability between positive electrode active materials are further improved. When the content of the binder is 4% by mass or less, the amount of the positive electrode active material in the positive electrode mixture layer can be further increased, so that the discharge capacity is further improved.

The positive electrode mixture layer preferably contains a conductive aid.

As for a material of the conductive aid, any known conductive aid can be used. The known conductive aid is preferably a conductive carbon material. Examples of the conductive carbon material include graphites, carbon blacks, conductive carbon fibers, and fullerenes. These conductive carbon materials may be used singly, or in combination of two or more kinds thereof. Examples of the conductive carbon fibers include carbon nanotubes, carbon nanofibers, and carbon fibers. Examples of the graphites include artificial graphite and natural graphite. Examples of the natural graphite include flake graphite, bulk graphite, and earthy graphite.

The conductive aid may be a commercially available product. Examples of a commercially available carbon black include: TOKA BLACK #4300, #4400, #4500, #5500 and the like (furnace blacks manufactured by Tokai Carbon Co., Ltd.); PRINTEX L and the like (furnace blacks manufactured by Degussa-Hüls AG); RAVEN 7000, 5750, 5250, 5000 ULTRA III, 5000 ULTRA and the like, CONDUCTEX SC ULTRA, CONDUCTEX 975ULTRA and the like, PURE BLACK 100, 115, 205 and the like (furnace blacks manufactured by Columbian Chemicals Company, Inc.); #2350, #2400B, #2600B, #30050B, #3030B, #3230B, #3350B, #3400B, #5400B and the like (furnace blacks manufactured by Mitsubishi Chemical Corporation); MONARCH 1400, 1300, and 900, VULCAN XC-72R, BLACK PEARLS 2000, LITX-50, LITX-200 and the like (furnace blacks manufactured by Cabot Corporation); ENSACO 250G, ENSACO 260G, ENSACO 350G, and SUPER-P (manufactured by Timcal Ltd.); KETJEN BLACK EC-300J and EC-600JD (manufactured by AkzoNobel N.V.); and DENKA BLACK, DENKA BLACK HS-100, and FX-35 (acetylene blacks manufactured by Denka Co., Ltd.).

The positive electrode mixture layer may also contain other components. Examples of the other components include a thickening agent, a surfactant, a dispersant, a wetting agent, and an antifoaming agent.

<Negative Electrode>

The negative electrode is configured to occlude and release lithium ions. The negative electrode preferably contains at least one negative electrode active material configured to occlude and release lithium ions.

The negative electrode more preferably includes a negative electrode current collector and a negative electrode

mixture layer. The negative electrode mixture layer is arranged on at least a portion of the surface of the negative electrode current collector.

A material of the negative electrode current collector is not particularly limited, and any known material can be used as desired. The material of the negative electrode current collector is, for example, a metal or an alloy. More particularly, examples of the material of the negative electrode current collector include aluminum, nickel, stainless steel (SUS), nickel-plated steel, and copper. Thereamong, from the standpoint of workability, the material of the negative electrode current collector is preferably copper. The negative electrode current collector is preferably a copper foil.

The negative electrode mixture layer contains a negative electrode active material and a binder.

The negative electrode active material is not particularly limited as long as it is a substance configured to occlude and release lithium ions. The negative electrode active material is preferably, for example, at least one selected from the group consisting of metal lithium, lithium-containing alloys, metals and alloys that can be alloyed with lithium, oxides capable of doping and dedoping lithium ions, transition metal nitrides capable of doping and dedoping lithium ions, and carbon materials capable of doping and dedoping lithium ions. Thereamong, the negative electrode active material is preferably a carbon material capable of doping and dedoping lithium ions (hereinafter, simply referred to as "carbon material").

Examples of the carbon material include carbon black, activated charcoal, graphite materials, and amorphous carbon materials. These carbon materials may be used singly, or in combination of two or more kinds thereof as a mixture. The form of the carbon material is not particularly limited and may be, for example, any of a fibrous form, a spherical form, a potato-like form, and a flake form. The particle size of the carbon material is also not particularly limited, and it is preferably from 5 $\mu$m to 50 $\mu$m, more preferably from 20 $\mu$m to 30 $\mu$m.

Examples of the amorphous carbon materials include hard carbon, cokes, mesocarbon microbeads (MCMB) calcined at 1,500°C or lower, and mesophase pitch carbon fibers (MCF).

Examples of the graphite materials include natural graphite and artificial graphite. Examples of the artificial graphite include graphitized MCMB and graphitized MCF. The graphite materials may contain boron. The graphite materials may be coated with a metal or amorphous carbon as well. Examples of the metal used for coating the graphite materials include gold, platinum, silver, copper, and tin. The graphite materials may be mixtures of amorphous carbon and graphite.

The negative electrode mixture layer preferably contains a conductive aid. Examples of the conductive aid include the same ones as those exemplified above as the conductive aid that may be contained in the positive electrode mixture layer.

The negative electrode mixture layer may also contain other components in addition to the above-described components. Examples of the other components include a thickening agent, a surfactant, a dispersant, a wetting agent, and an antifoaming agent.

<Separator>

The separator is, for example, a porous resin plate. Examples of a material of the porous resin plate include resins and nonwoven fabrics containing the resins. Examples of the resins include polyethylene (PE), polypropylene (PP), polymethylpentene (PMP), polyester, cellulose, and polyamide.

Particularly, the separator is preferably a porous resin sheet having a monolayer or multilayer structure. The material of the porous resin sheet is mainly composed of one or more kinds of polyolefin resins. The thickness of the separator is preferably from 5 $\mu$m to 30 $\mu$m. The separator is preferably arranged between the positive electrode and the negative electrode.

[One Example of Lithium Secondary Battery Precursor]

Referring to FIG. 1, one example of a lithium secondary battery precursor 1 according to one embodiment of the disclosure will now be described concretely. FIG. 1 is a cross-sectional view of the lithium secondary battery precursor 1 according to one embodiment of the disclosure.

The lithium secondary battery precursor 1 is of a layered type. As illustrated in FIG. 1, in the lithium secondary battery precursor 1, a battery element 10 is enclosed in an outer package 30. The outer package 30 is formed of a laminated film. A positive electrode lead 21 and a negative electrode lead 22 are each attached to the battery element 10. The positive electrode lead 21 and the negative electrode lead 22 are drawn out in the opposite direction to each other from the inside of the outer package 30 to the outside.

In the battery element 10, as illustrated in FIG. 1, a positive electrode 11, a separator 13, and a negative electrode 12 are disposed in layers. In the positive electrode 11, a positive electrode mixture layer 11B is formed on both main surfaces of a positive electrode current collector 11A. In the negative electrode 12, a negative electrode mixture layer 12B is formed on both main surfaces of a negative electrode current collector 12A. The positive electrode

mixture layer 11B formed on one of the main surfaces of the positive electrode current collector 11A of the positive electrode 11 and the negative electrode mixture layer 12B formed on one of the main surfaces of the negative electrode current collector 12A of the negative electrode 12 adjacent to the positive electrode 11 face each other via the separator 13.

Inside the outer package 30 of the lithium secondary battery precursor 1, the nonaqueous electrolyte solution of the disclosure is injected. The nonaqueous electrolyte solution of the disclosure is impregnated into the positive electrode mixture layer 11B, the separator 13, and the negative electrode mixture layer 12B. In the lithium secondary battery precursor 1, a single cell layer 14 is formed by the positive electrode mixture layer 11B, the separator 13, and the negative electrode mixture layer 12B that are adjacent to one another. The positive electrode and the negative electrode may each have an active material layer formed on one side of the respective current collector.

It is noted here that, although the lithium secondary battery precursor 1 of the present embodiment is of a layered type, the disclosure is not limited thereto, and the lithium secondary battery precursor 1 may be of, for example, a wound type. The lithium secondary battery precursor 1 of a wound type is obtained by disposing the positive electrode, the separator, the negative electrode, and the separator on one another in this order, and winding the resultant in a layered form. The wound type encompasses a cylindrical shape and a prismatic shape.

In the present embodiment, as illustrated in FIG. 1, the directions in which the positive electrode lead and the negative electrode lead each protrude from the inside of the outer package 30 to the outside are opposite to each other with respect to the outer package 30; however, the disclosure is not limited to this mode. For example, the directions in which the positive electrode lead and the negative electrode lead each protrude from the inside of the outer package 30 to the outside may be the same with respect to the outer package 30.

One example of the below-described lithium secondary battery according to one embodiment of the disclosure is a lithium secondary battery of a mode in which a SEI film is formed on the surface of each of the positive electrode mixture layer 11B and the negative electrode mixture layer 12B in the lithium secondary battery precursor 1 by charging and discharging of the lithium secondary battery precursor 1.

Another example of the lithium secondary battery precursor of the disclosure is a coin-type battery.

FIG. 2 is a schematic cross-sectional view illustrating a coin-type battery, which is another example of the lithium secondary battery precursor of the disclosure.

In the coin-type battery illustrated in FIG. 2, a disc-shaped negative electrode 42, a separator 45 into which a nonaqueous electrolyte solution is injected, a disc-shaped positive electrode 41 and, as required, spacer plates 47 and 48 made of stainless steel, aluminum, or the like are disposed in layers in the order mentioned and housed between a positive electrode can 43 (hereinafter, also referred to as "battery can") and a sealing plate 44 (hereinafter, also referred to as "battery can lid"). The positive electrode can 43 and the sealing plate 44 are hermetically sealed by caulking via a gasket 46.

In this example, the nonaqueous electrolyte solution of the disclosure is used as the nonaqueous electrolyte solution injected into the separator 45.

[Lithium Secondary Battery]

Next, the lithium secondary battery according to one embodiment of the disclosure will be described.

The lithium secondary battery according to the present embodiment includes a casing, a positive electrode, a negative electrode, a separator, and an electrolyte solution. The positive electrode, the negative electrode, the separator, and the electrolyte solution are housed in the casing. The positive electrode is configured to occlude and release lithium ions. The negative electrode is configured to occlude and release lithium ions. The electrolyte solution is the nonaqueous electrolyte solution of the disclosure. The negative electrode includes a negative electrode SEI film. The positive electrode includes a positive electrode SEI film.

The lithium secondary battery according to the present embodiment is different from the lithium secondary battery precursor according to the present embodiment mainly in terms of a first point that the negative electrode includes a negative electrode SEI film, and a second point that the positive electrode includes a positive electrode SEI film. In other words, the lithium secondary battery according to the present embodiment is the same as the lithium secondary battery precursor according to the present embodiment, except for the first and the second points. Therefore, with regard to the lithium secondary battery according to the present embodiment, descriptions of constituent members other than the first and the second points are omitted below.

With regard to the first point, when the negative electrode includes a negative electrode current collector and a negative electrode mixture layer, the feature that "the negative electrode includes a negative electrode SEI film" encompasses a first negative electrode form and a second negative electrode form. The first negative electrode form represents a form in which the negative electrode SEI film is formed on at least a portion of the surface of the negative electrode mixture layer. The second negative electrode form represents a form in which the negative electrode SEI film is formed on the surface of a negative electrode active material that is a constituent of the negative electrode mixture layer.

With regard to the second point, when the positive electrode includes a positive electrode current collector and

a positive electrode mixture layer, the feature that "the positive electrode includes a positive electrode SEI film" encompasses a first positive electrode form and a second positive electrode form. The first positive electrode form represents a form in which the positive electrode SEI film is formed on at least a portion of the surface of the positive electrode mixture layer. The second positive electrode form represents a form in which the positive electrode SEI film is formed on the surface of a positive electrode active material that is a constituent of the positive electrode mixture layer.

These SEI films contain, for example, at least one selected from the group consisting of a decomposition product of the lithium (*N*-carbonyl)sulfonamide compound (I), a reaction product of the lithium (*N*-carbonyl)sulfonamide compound (I) and an electrolyte, and a decomposition product of the reaction product.

A component of the negative electrode SEI film and a component of the positive electrode SEI film may be the same or different from each other. The thickness of the negative electrode SEI film and that of the positive electrode SEI film may also be the same or different from each other.

The lithium secondary battery of the disclosure is obtained by charging and discharging the lithium secondary battery precursor of the disclosure. In other words, the lithium secondary battery of the disclosure is obtained by performing the below-described aging step.

[Method of Producing Lithium (*N*-carbonyl)sulfonamide Compound]

Next, a method of producing the lithium (*N*-carbonyl)sulfonamide compound of the disclosure will be described.

The method of producing the lithium (*N*-carbonyl)sulfonamide compound includes a below-described first step and a below-described second step. The first step and the second step are carried out in this order. As a result, the lithium (*N*-carbonyl)sulfonamide compound of the disclosure is obtained.

<First Step>

In the first step, a sulfonamide compound is allowed to react with a carboxylic acid chloride or a carboxylic anhydride in a solvent, the resulting salt is removed, and a (*N*-carbonyl)sulfonamide compound is obtained by column chromatography.

The sulfonamide compound, the carboxylic acid chloride, and the carboxylic anhydride are each selected as appropriate in accordance with the type of the (*N*-carbonyl)sulfonamide compound obtained as a product.

Examples of the sulfonamide compound include trifluoromethanesulfonamide, methanesulfonamide, phenoxymethyl sulfonamide, ethyl sulfamate, and 2,2,2-trifluoroethyl sulfamate.

Examples of the carboxylic acid chloride include methyl chloroformate, ethyl chloroformate, propyl chloroformate, isopropyl chloroformate, butyl chloroformate, phenyl chloroformate, and acetyl chloride.

Examples of the carboxylic anhydride include trifluoroacetic anhydride, acetic anhydride, trichloroacetic anhydride, di-*tert*-butyl dicarbonate, succinic anhydride, maleic anhydride, citraconic anhydride, itaconic anhydride, glutaric anhydride, 1,2-cyclohexenedicarboxylic anhydride, *n*-octadecylsuccinic anhydride, 5-norbornene-2,3-dicarboxylic anhydride, phthalic anhydride, trimellitic anhydride, pyromellitic anhydride, and naphthalic anhydride.

The solvent is, for example, a nonaqueous solvent. Examples of the nonaqueous solvent include tetrahydrofuran, diethyl ether, dimethoxyethane, 1,4-dioxane, acetone, ethyl acetate, acetonitrile, dimethyl carbonate, ethyl methyl carbonate, diethyl carbonate, pentane, hexane, heptane, octane, nonane, decane, toluene, xylene, ethylbenzene, butylbenzene, pentylbenzene, hexylbenzene, heptylbenzene, propylbenzene, isopropylbenzene (other name: cumene), cyclohexylbenzene, tetralin, mesitylene, methylcyclopentane, cyclohexane, methylcyclohexane, cycloheptane, cyclooctane, and cyclononane. Xylene includes *ortho*-xylene, *meta*-xylene, and *para*-xylene.

The above-described reaction in the first step can be carried out either under normal pressure or under reduced pressure. From the standpoint of preventing contamination with a component (e.g., water) that inhibits the generation of the (*N*-carbonyl)sulfonamide compound, the reaction in the first step is preferably carried out in an inert atmosphere. Examples of the component that inhibits the generation of the (*N*-carbonyl)sulfonamide compound include water. Examples of the inert atmosphere include a nitrogen atmosphere and an argon atmosphere.

The reaction temperature in the first step is preferably from -20°C to 60°C, more preferably from 0°C to 40°C, still more preferably from 10°C to 30°C. When the reaction temperature is 60°C or lower, decomposition of the sulfonamide used as a raw material and that of the carboxylic acid chloride or carboxylic anhydride used as a reagent are inhibited, so that the generation rate of the (*N*-carbonyl)sulfonamide compound is likely to be improved.

From the standpoint of allowing the reaction to proceed efficiently, the reaction time in the first step is preferably from 30 minutes to 12 hours, more preferably from 1 hour to 6 hours.

<Second Step>

In the second step, the (*N*-carbonyl)sulfonamide compound is allowed to react with a lithium salt compound in

a solvent. By this, a lithium (*N*-carbonyl)sulfonamide compound is obtained.

Examples of the lithium salt compound include lithium bis(trimethylsilyl)amide, lithium chloride, lithium carbonate, lithium hydroxide, lithium methoxide, lithium ethoxide, and lithium-*t*-butoxide. Thereamong, the lithium salt compound is preferably lithium bis(trimethylsilyl)amide, lithium chloride, lithium carbonate, or lithium hydroxide, more preferably lithium bis(trimethylsilyl)amide.

The above-described reaction in the second step can be carried out either under normal pressure or under reduced pressure. From the standpoint of preventing contamination with a component (e.g., water) that inhibits the generation of the lithium (*N*-carbonyl)sulfonamide compound, the reaction in this synthesis step is preferably carried out in an inert atmosphere. Examples of the component that inhibits the generation of the lithium (*N*-carbonyl)sulfonamide compound include water. Examples of the inert atmosphere include a nitrogen atmosphere and an argon atmosphere.

The reaction temperature in the second step is preferably from -20°C to 60°C, more preferably from 0°C to 40°C, still more preferably from 10°C to 30°C.

From the standpoint of allowing the reaction to proceed efficiently, the reaction time in the second step is preferably from 30 minutes to 12 hours, more preferably from 1 hour to 6 hours.

A method of recovering the lithium (*N*-carbonyl)sulfonamide compound from a product is not particularly limited, and can be adjusted as appropriate in accordance with the state of the obtained product. When only the lithium (*N*-carbonyl)sulfonamide compound is obtained, the lithium (*N*-carbonyl)sulfonamide compound is recovered without any special treatment. When a slurry in which the lithium (*N*-carbonyl)sulfonamide compound is dispersed in a solvent is the product, the lithium (*N*-carbonyl)sulfonamide compound is recovered by separating the solvent from the slurry and drying the resultant. When a solution in which the lithium (*N*-carbonyl)sulfonamide compound is dissolved in a solvent is the product, the lithium (*N*-carbonyl)sulfonamide compound is recovered by removing the solvent from the solution by distillation through heat-concentration or the like. When a solution in which the lithium (*N*-carbonyl)sulfonamide compound is dissolved in a solvent is the product, the lithium (*N*-carbonyl)sulfonamide compound is also recovered by adding a solvent, which does not dissolve the lithium (*N*-carbonyl)sulfonamide compound, to the solution so as to allow the lithium (*N*-carbonyl)sulfonamide compound to precipitate, subsequently separating the solvent from the solution, and then drying the resultant.

The lithium (*N*-carbonyl)sulfonamide compound recovered from the product may be subjected to a drying treatment. The drying treatment is not particularly limited, and examples of a method thereof include: a static drying method using a tray dryer; a fluid drying method using a conical dryer; a drying method using equipment such as a hot plate or an oven; and a method of supplying warm air or hot air using a drying machine such as a dryer.

The pressure at which the lithium (*N*-carbonyl)sulfonamide compound recovered from the product is dried may be either normal pressure or reduced pressure. The temperature at which the lithium (*N*-carbonyl)sulfonamide compound recovered from the product is dried is preferably from 20°C to 100°C, more preferably from 40°C to 80°C, still more preferably from 50°C to 70°C. When this drying temperature is 20°C or higher, excellent drying efficiency is obtained. When the drying temperature is 100°C or lower, decomposition of the generated lithium (*N*-carbonyl)sulfonamide compound is inhibited, so that the lithium (*N*-carbonyl)sulfonamide compound is likely to be recovered in a stable manner.

The lithium (*N*-carbonyl)sulfonamide compound recovered from the product may be used as is, or may be used after being, for example, dispersed or dissolved in a solvent, or mixed with other substance.

[Method of Producing Lithium (*N*-carbonyl)sulfonamide Compound (I)]

Next, a method of producing the lithium (*N*-carbonyl)sulfonamide compound (I) of the disclosure will be described.

The method of producing the lithium (*N*-carbonyl)sulfonamide compound (I) of the disclosure is carried out in the same manner as the above-described method of producing a lithium (*N*-carbonyl)sulfonamide compound (I), except that the sulfonamide compound, the carboxylic acid chloride, and the carboxylic anhydride may be selected such that $L^1$ and $L^2$ in Formula (1) are each a single bond. By this the lithium (*N*-carbonyl)sulfonamide compound (I) is obtained.

[Method of Producing Nonaqueous Electrolyte Solution]

Next, a method of producing the nonaqueous electrolyte solution of the disclosure will be described.

The method of producing the nonaqueous electrolyte solution of the disclosure includes the synthesis step, the dissolution step, and the mixing step. The dissolution step and the mixing step are carried out in this order. The synthesis step may be carried out prior to the mixing step.

In the synthesis step, a lithium (*N*-carbonyl)sulfonamide compound (I) is synthesized.

The synthesis step can be carried out in the same manner as the above-described method of producing a lithium (*N*-carbonyl)sulfonamide compound (I).

In the dissolution step, an electrolyte is dissolved in a nonaqueous solvent to obtain a solution. As compared

to the electroconductivity of this solution prior to the addition of the lithium (*N*-carbonyl)sulfonamide compound (I) thereto, the electroconductivity of the resulting nonaqueous electrolyte solution is preferably reduced.

In the mixing step, the lithium (*N*-carbonyl)sulfonamide compound (I) and, as required, other additives are added to the solution, followed by mixing. By this, a nonaqueous electrolyte solution is obtained. The nonaqueous electrolyte solution obtained by the method of producing a nonaqueous electrolyte solution according to the present embodiment more efFectively exerts an effect of reducing the direct-current resistance in a lithium secondary battery.

It is noted here that, although the method of producing the nonaqueous electrolyte solution of the disclosure includes the synthesis step, the dissolution step, and the mixing step, the disclosure is not limited to this mode.

[Method of Producing Lithium Secondary Battery Precursor]

Next, a method of producing the lithium secondary battery precursor of the disclosure will be described.

The method of producing the lithium secondary battery precursor of the disclosure includes: the first preparation step; the second preparation step; the third preparation step; the housing step; and the injection step. The housing step and the injection step are carried out in this order. The first preparation step, the second preparation step, and the third preparation step are each carried out prior to the housing step.

In the first preparation step, a positive electrode is prepared.

Examples of a method of preparing the positive electrode include a method of applying and then drying a positive electrode mixture slurry onto the surface of a positive electrode current collector. The positive electrode mixture slurry contains a positive electrode active material and a binder.

As a solvent contained in the positive electrode mixture slurry, an organic solvent is preferred. Examples of the organic solvent include *N*-methyl-2-pyrrolidone (NMP).

A method of applying the positive electrode mixture slurry is not particularly limited, and examples thereof include slot die coating, slide coating, curtain coating, and gravure coating. A method of drying the positive electrode mixture slurry is also not particularly limited, and examples thereof include drying with warm air, hot air, or low-humidity air; vacuum-drying; and drying by irradiation with infrared radiation (e.g., far-infrared radiation). The drying time is not particularly limited, and it is preferably from 1 minute to 30 minutes. The drying temperature is also not particularly limited, and it is preferably from 40°C to 80°C.

A dry product obtained by applying and drying the positive electrode mixture slurry on the positive electrode current collector is preferably subjected to a press treatment. By this, the porosity of the resulting positive electrode active material layer is reduced. As a method of the press treatment, for example, mold pressing or roll pressing can be employed.

In the second preparation step, a negative electrode is prepared.

Examples of a method of preparing the negative electrode include a method of applying and then drying a negative electrode mixture slurry onto the surface of a negative electrode current collector. The negative electrode mixture slurry contains a negative electrode active material and a binder.

Examples of a solvent contained in the negative electrode mixture slurry include water and a liquid medium compatible with water. When the solvent contained in the negative electrode mixture slurry contains a liquid medium compatible with water, the applicability of the mixture slurry to the negative electrode current collector can be improved. Examples of the liquid medium compatible with water include alcohols, glycols, cellosolves, aminoalcohols, amines, ketones, carboxylic acid amides, phosphoric acid amides, sulfoxides, carboxylic acid esters, phosphoric acid esters, ethers, and nitriles.

Examples of an application method, a drying method, and a press treatment of the negative electrode mixture slurry include the same methods as those exemplified above for the positive electrode mixture slurry.

In the third preparation step, a nonaqueous electrolyte solution is prepared. A method of preparing the nonaqueous electrolyte solution is the same as the above-described method of producing a nonaqueous electrolyte solution.

In the housing step, the positive electrode, the negative electrode, and a separator are housed in a casing.

In the housing step, for example, a battery element is produced using the positive electrode, the negative electrode, and the separator. Subsequently, the positive electrode current collector of the positive electrode and a positive electrode lead are electrically connected, and the negative electrode current collector of the negative electrode and a negative electrode lead are electrically connected. Thereafter, the resulting battery element is housed and immobilized in the casing.

A method of electrically connecting the positive electrode current collector and the positive electrode lead is not particularly limited, and examples thereof include ultrasonic welding and resistance welding. A method of electrically connecting the negative electrode current collector and the negative electrode lead is also not particularly limited, and examples thereof include ultrasonic welding and resistance welding.

A state where the positive electrode, the negative electrode, and the separator are housed in the casing is hereinafter referred to as "assembly".

In the injection step, the nonaqueous electrolyte solution of the disclosure is injected into the assembly. By this, the nonaqueous electrolyte solution is allowed to permeate into a positive electrode mixture layer, the separator, and a negative electrode mixture layer. As a result, a lithium secondary battery precursor is obtained.

[Method of Producing Lithium Secondary Battery]

Next, the method of producing a lithium secondary battery according to the disclosure will be described.

The method of producing a lithium secondary battery according to the disclosure includes the fourth preparation step and the aging step. The fourth preparation step and the aging step are carried out in this order.

In the fourth preparation step, a lithium secondary battery precursor is prepared. A method of preparing the lithium secondary battery precursor is the same as the above-described method of producing a lithium secondary battery precursor.

In the aging step, the lithium secondary battery precursor is subjected to an aging treatment. By this, a negative electrode SEI film and a positive electrode SEI film are formed. In other words, a lithium secondary battery is obtained.

The aging treatment includes charging and discharging the lithium secondary battery precursor in an environment of from 25°C to 70°C. Particularly, the aging treatment includes: a first charging phase; a first retention phase; a second charging phase; a second retention phase; and a charge-discharge phase.

In the first charging phase, the lithium secondary battery precursor is charged in an environment of from 25°C to 70°C. In the first retention phase, the lithium secondary battery precursor after the first charging phase is maintained in an environment of from 25°C to 70°C. In the second charging phase, the lithium secondary battery precursor after the first retention phase is charged in an environment of from 25°C to 70°C. In the second retention phase, the lithium secondary battery precursor after the second charging phase is maintained in an environment of from 25°C to 70°C. In the charge-discharge phase, the lithium secondary battery precursor after the second retention phase is subjected to a combination of charging and discharging at least once in an environment of from 25°C to 70°C.

In the lithium secondary battery obtained by the method of producing a lithium secondary battery according to the disclosure, an effect of inhibiting an increase in the direct-current resistance and a decrease in the discharge capacity is more efFectively exerted even when the lithium secondary battery is stored in a high-temperature environment.

EXAMPLES

Embodiments of the disclosure will now be described in detail referring to Examples. It is noted here, however, that the disclosure is not limited to the below-described Examples by any means.

[Synthesis of Lithium (*N*-carbonyl)sulfonamide Compound (I)]

Synthesis Compounds (I-1) to (I-48) represented by the following Formula (I) were synthesized as described below.

$R^1$, $R^2$, $L^1$, and $L^2$ in Formula (I) of each of Synthesis Compounds (I-1) to (I-48) are shown in Tables 1 and 2.

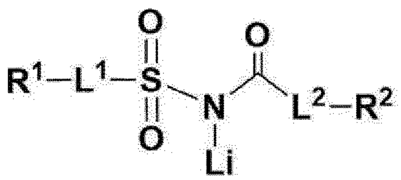

(I)

[Table 1]

| Synthesis Compound | $R^1$ | $L^1$ | $R^2$ | $L^2$ | Synthesis Example |
|---|---|---|---|---|---|
| I-1 | 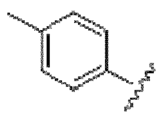 | single bond | Me | -O- | 3 |

(continued)

| Synthesis Compound | $R^1$ | $L^1$ | $R^2$ | $L^2$ | Synthesis Example |
|---|---|---|---|---|---|
| I-2 | 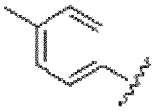 | single bond | Et | -O- | 4 |
| I-3 | 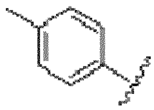 | single bond | *n*-Pr | -O- | 5 |
| I-4 | 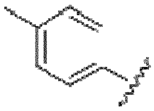 | single bond | *i*-Pr | -O- | 6 |
| I-5 | 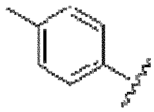 | single bond | *n*-Bu | -O- | 1 |
| I-6 | 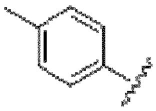 | single bond | *t*-Bu | -O- | 7 |
| I-7 | 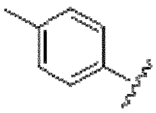 | single bond | $CH_2CF_3$ | -O- | 8 |
| I-8 | 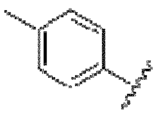 | single bond | Ph | -O- | 9 |
| I-9 | 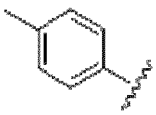 | single bond | $CH_2CH_2OCH_3$ | -O- | 10 |
| I-10 | $CF_3$ | single bond | Me | -O- | 2 |
| I-11 | $CF_3$ | single bond | Et | -O- | 11 |
| I-12 | $CF_3$ | single bond | *n*-Pr | -O- | 12 |
| I-13 | $CF_3$ | single bond | *i*-Pr | -O- | 13 |
| I-14 | $CF_3$ | single bond | *n*-Bu | -O- | 14 |
| I-15 | $CF_3$ | single bond | Ph | -O- | 15 |
| I-16 | $CH_3$ | single bond | Me | -O- | 16 |
| I-17 | $CH_3$ | single bond | *t*-Bu | -O- | 17 |
| I-18 | $CH_3$ | single bond | Ph | -O- | 18 |

[Table 2]

| Synthesis Compound | $R^1$ | $L^1$ | $R^2$ | $L^2$ | Synthesis Example |
|---|---|---|---|---|---|
| I-19 | Et | -O- | Me | single bond | 19 |

(continued)

| Synthesis Compound | $R^1$ | $L^1$ | $R^2$ | $L^2$ | Synthesis Example |
|---|---|---|---|---|---|
| I-20 | Et | -O- | $CF_3$ | single bond | 20 |
| I-21 | $CH_2CF_3$ | -O- | Et | single bond | 21 |
| I-22 | $CH_2CF_3$ | -O- | Ph | single bond | 22 |
| I-23 | Et | -O- | Ph | single bond | 23 |
| I-24 | Ph | -O- | Me | single bond | 24 |
| I-25 | Ph | -O- | Ph | single bond | 25 |
| I-26 | Et | -O- | Et | -O- | 26 |
| I-27 | $CH_2CF_3$ | -O- | Et | -O- | 27 |
| I-28 | $CH_2CF_3$ | -O- | $CH_2CF_3$ | -O- | 28 |
| I-29 | $CH_2CF_3$ | -O- | Ph | -O- | 29 |
| I-30 | 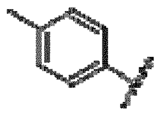 | -O- | Et | -O- | 30 |
| I-31 | 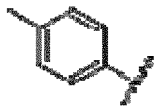 | -O- | $CH_2CF_3$ | -O- | 31 |
| I-32 | 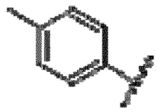 | -O- | 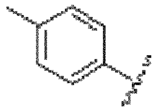 | -O- | 32 |

[Table 3]

| Synthesis Compound | $R^1$ | $L^1$ | $R^2$ | $L^2$ | Synthesis Example |
|---|---|---|---|---|---|
| I-33 | $CF_3$ | single bond | 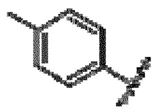 | -O- | 33 |
| I-34 | $CF_3$ | single bond | 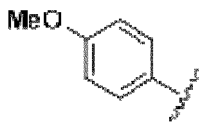 | -O- | 34 |
| I-35 | $CF_3$ | single bond | 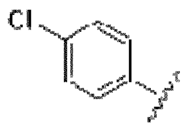 | -O- | 35 |
| I-36 | $CF_3$ | single bond | 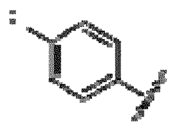 | -O- | 36 |
| I-37 | $CF_3$ | single bond | 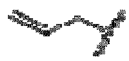 | -O- | 37 |

(continued)

| Synthesis Compound | $R^1$ | $L^1$ | $R^2$ | $L^2$ | Synthesis Example |
|---|---|---|---|---|---|
| I-38 | 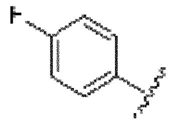 | single bond | n-Bu | -O- | 38 |
| I-39 | 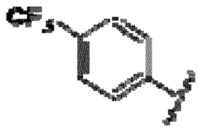 | single bond | Me | -O- | 39 |
| I-40 | 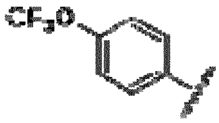 | single bond | Me | -O- | 40 |
| I-41 | Et | -O- | Ph | -O- | 41 |
| I-42 | F | single bond | Me | -O- | 42 |
| I-43 | F | single bond | Et | -O- | 43 |
| I-44 | F | single bond | *n*-Pr | -O- | 44 |
| I-45 | F | single bond | *n*-Bu | -O- | 45 |
| I-46 | F | single bond | 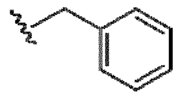 | -O- | 46 |
| I-47 | 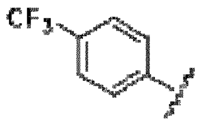 | single bond | *n*-Bu | -O- | 47 |
| I-48 | 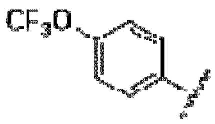 | single bond | *n*-Bu | -O- | 48 |

[Synthesis Example 1]

Lithium butoxycarbonyl tosylamide [Synthesis Compound (I-5)] represented by the following structural formula was synthesized in the following manner.

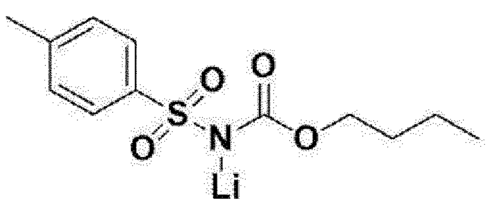

(I-5)

<First Step: Synthesis of Butoxycarbonyl Tosylamide>

To a nitrogen-purged 200-mL four-necked flask connected to a Dimroth condenser, p-toluene sulfonyl isocyanate (3.94 g, 20 mmol), dibutyl tin dilaurate (0.126 g, 0.2 mmol), and dichloromethane (100 mL) as a solvent were charged and, after maintaining this flask at room temperature, *n*-butanol (1.78 g, 24 mmol) was added, and a reaction was allowed to proceed with stirring at a reflux temperature (40°C). The reaction was terminated after 4 hours, followed

by cooling to room temperature.

Subsequently, the resulting reaction product was washed. In other words, the reaction solution was extracted and washed using a separatory funnel with addition of 100 mL of distilled water and 10 mL of ethyl acetate.

The above-described washing with water was repeated twice, and the resulting organic layer was dried with an addition of magnesium sulfate and then concentrated under reduced pressure.

The thus obtained crude product was separated and purified by flash column chromatography using a hexane/ethyl acetate solvent. The hexane/ethyl acetate solvent consisted of hexane and ethyl acetate.

In this manner, butoxycarbonyl tosylamide was obtained as a first white solid (5.27 g, 19.42 mmol, yield = 97%).

The result of measuring this first white solid by $^1$H-NMR (DMSO-d6) is shown below.

$^1$H-NMR: δ0.82 (t,J = 7.2 Hz, 3H), 1.10-1.30 (m,2H), 1.38-1.53 (m,2H), 2.40 (s,3H), 3.97 (t,J = 6.5 Hz, 2H), 7.44 (d,J = 8.1 Hz, 2H), 7.78 (d,J = 8.4 Hz, 2H), 11.89 (br,1H)

<Second Step: Synthesis of Lithium Butoxycarbonyl Tosylamide (I-5)>

To a nitrogen-purged 200-mL four-necked flask, butoxycarbonyl tosylamide (3.11 g, 11.46 mmol) and tetrahydrofuran (50 mL) as a solvent were charged. This flask was maintained at -20°C, and lithium bis(trimethylsilyl)amide (1.3M, 1.3 mol/dm$^3$) and a tetrahydrofuran solution (8.8 mL, 11.46 mmol) were added thereto over a period of 5 minutes, after which the flask was brought back to room temperature, and a reaction was allowed to proceed with stirring for 6 hours, as a result of which a second white solid was precipitated from the resulting reaction solution. This reaction solution was vacuum-filtered to remove the residual solvent of the thus obtained second white solid by vacuum distillation.

In this manner, lithium butoxycarbonyl tosylamide was obtained as the second white solid (2.17 g, 7.83 mmol, yield = 68%).

The result of measuring this second white solid by $^1$H-NMR (DMSO-d6) is shown below.

$^1$H-NMR: δ0.84 (t,J = 7.3 Hz, 3H), 1.16-1.45 (m,4H), 2.31 (s,3H), 3.64 (t,J = 6.5 Hz, 2H), 7.15 (d,J = 8.4 Hz, 2H), 7.58 (d,J = 8.1 Hz, 2H)

As described above, according to the results of Synthesis Example 1, lithium butoxy tosylamide [Synthesis Compound (I-5)] was obtained by the following reaction scheme.

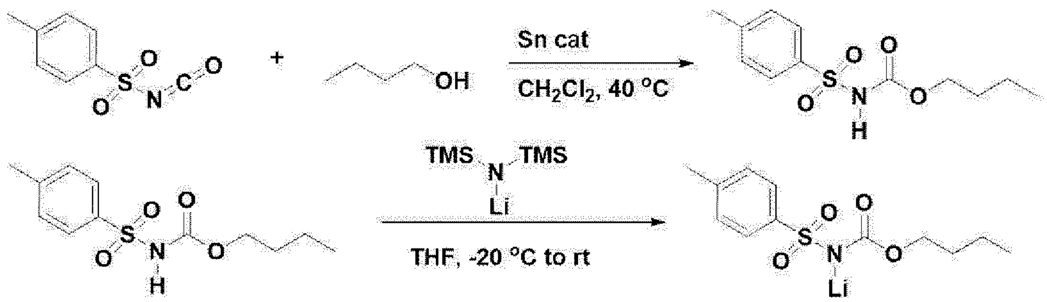


(I-5)

[Synthesis Example 2]

Lithium methoxycarbonyl trifluoromethyl sulfonamide [Synthesis Compound (I-10)] represented by the following structural formula was synthesized in the following manner.

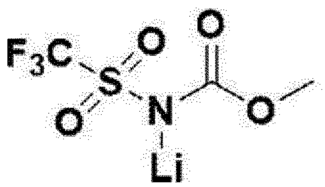

(I-10)

<First Step: Synthesis of Methoxycarbonyl Trifluoromethyl Sulfonamide>

To a nitrogen-purged 200-mL four-necked flask, trifluoromethanesulfonamide (5.01 g, 33.6 mmol), pyridine (6.38 g, 81 mmol), 4-dimethylaminopyridine (0.99 g, 8.1 mmol), and tetrahydrofuran (50 mL) as a solvent were charged. This flask was maintained at 0°C, and methyl chloroformate (7.62 g, 81 mmol) was added thereto over a period of 5 minutes, after which the flask was brought back to room temperature, and a reaction was allowed to proceed with stirring for 6 hours. Subsequently, the resulting reaction solution was filtered to remove hydrochloride, and the thus obtained

solution was washed. In other words, the filtrate was extracted and washed using a separatory funnel with addition of 100 mL of distilled water and 100 mL of ethyl acetate.

The above-described washing with water was repeated twice, and the resulting organic layer was dried with an addition of magnesium sulfate and then concentrated under reduced pressure.

The thus obtained crude product was separated and purified by flash column chromatography using a hexane/ethyl acetate solvent.

In this manner, methoxycarbonyl trifluoromethyl sulfonamide was obtained as a third white solid (3.12 g, 33.6 mmol, yield = 44.8%).

The result of measuring this third white solid by $^1$H-NMR (DMSO-d6) is shown below.

$^1$H-NMR: δ3.51 (s,3H), 10.00 (br,2H)

<Second Step: Synthesis of Lithium Methoxycarbonyl trifluoromethyl Sulfonamide (I-10)>

To a nitrogen-purged 200-mL four-necked flask, methoxycarbonyl trifluoromethyl sulfonamide (1.90 g, 9.17 mmol) and diethyl ether (50 mL) as a solvent were charged. This flask was maintained at -20°C, and a lithium bis(trimethylsilyl)amide (1.3M) tetrahydrofuran solution (7.1 mL, 9.17 mmol) was added thereto over a period of 5 minutes, after which the flask was brought back to room temperature, and a reaction was allowed to proceed with stirring for 3 hours. Subsequently, n-hexane (50 mL) was added at room temperature to allow a fourth white solid to precipitate from the resulting reaction solution. This reaction solution was vacuum-filtered to remove the residual solvent of the thus obtained fourth white solid by vacuum distillation.

In this manner, lithium methoxycarbonyl trifluoromethyl sulfonamide was obtained as the fourth white solid (0.99 g, 4.65 mmol, yield = 51%).

The result of measuring this fourth white solid by $^1$H-NMR (DMSO-d6) is shown below.

$^1$H-NMR: δ3.40 (s,3H)

As described above, according to the results of Synthesis Example 2, lithium methoxycarbonyl trifluoromethyl sulfonamide [Synthesis Compound (I-10)] was obtained by the following reaction scheme.

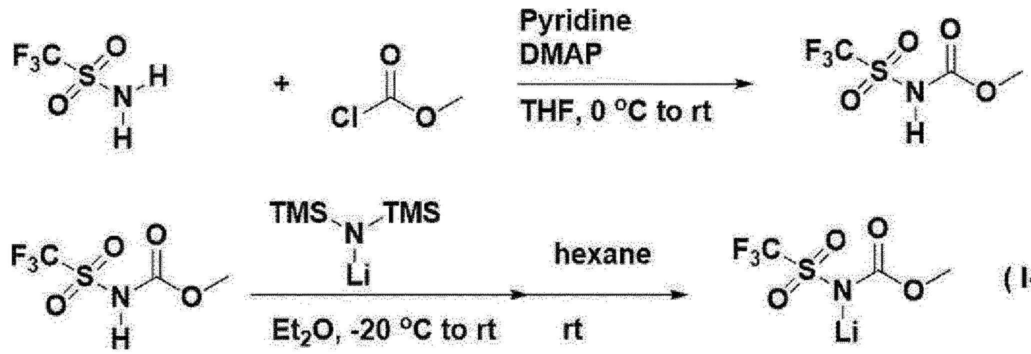


( I-10 )

[Synthesis Example 3]

Lithium methoxycarbonyl tosylamide [Synthesis Compound (I-1)] represented by the following structural formula was synthesized in the following manner.

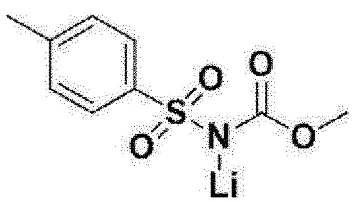

( I-1 )

<First Step: Synthesis of Methoxycarbonyl Tosylamide>

Methoxycarbonyl tosylamide was synthesized in the same manner as in the first step of Synthesis Example 1, except that n-butanol was changed to methanol (1.28 g, 40 mmol) in the first step of Synthesis Example 1 (Synthesis Compound (I-5)).

By this, methoxycarbonyl tosylamide was obtained as a fifth white solid (4.33 g, 18.89 mmol, yield = 94%).
The result of measuring this fifth white solid by $^1$H-NMR (DMSO-d6) is shown below.
$^1$H-NMR: δ2.31 (s,3H), 3.24 (s,3H), 7.15 (d,J = 7.8 Hz, 2H), 7.59 (d,J = 7.8 Hz, 2H)

<Second Step: Synthesis of Lithium Methoxycarbonyl Tosylamide (I-1)>

Using methoxycarbonyl tosylamide (1.52 g, 6.63 mmol), tetrahydrofuran (30 mL) as a solvent, and a lithium bis(trimethylsilyl)amide (1.3M) tetrahydrofuran solution (5.1 mL, 6.63 mmol), a reaction was carried out in the same manner as in the second step of Synthesis Example 1 (Synthesis Example 5).
By this, lithium methoxycarbonyl tosylamide was obtained as a sixth white solid (0.99 g, 4.20 mmol, yield = 63%).
The result of measuring this sixth white solid by $^1$H-NMR (DMSO-d6) is shown below.
$^1$H-NMR: δ2.31 (s,3H), 3.25 (s,3H), 7.16 (d,J = 7.8 Hz, 2H), 7.59 (d,J = 7.8 Hz, 2H)

[Synthesis Example 4]

Lithium ethoxycarbonyl tosylamide [Synthesis Compound (I-2)] represented by the following structural formula was synthesized in the following manner.

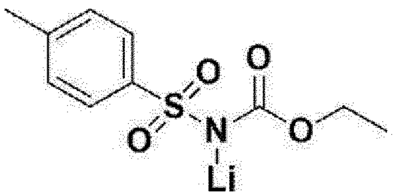

(I-2)

<First Step: Synthesis of Ethoxycarbonyl Tosylamide>

Ethoxycarbonyl tosylamide was synthesized in the same manner as in the first step of Synthesis Example 1, except that n-butanol was changed to ethanol (1.84 g, 40 mmol) in the first step of Synthesis Example 1 (Synthesis Compound (I-5)).
By this, ethoxycarbonyl tosylamide was obtained as a seventh white solid (4.47 g, 18.37 mmol, yield = 92%).
The result of measuring this seventh white solid by $^1$H-NMR (DMSO-d6) is shown below.
$^1$H-NMR: δ1.00 (t,J = 7.0 Hz, 3H), 2.23 (s,3H), 3.68 (q,J = 7.0 Hz, 2H), 7.15 (d,J = 8.1 Hz, 2H), 7.58 (d,J = 7.8 Hz, 2H)

<Second Step: Synthesis of Lithium Ethoxycarbonyl Tosylamide (I-2)>

Using ethoxycarbonyl tosylamide (3.24 g, 13.32 mmol), tetrahydrofuran (30 mL) as a solvent, and a lithium bis(trimethylsilyl)amide (1.3M) tetrahydrofuran solution (10.2 mL, 13.32 mmol), a reaction was carried out in the same manner as in the second step of Synthesis Example 1 (Synthesis Compound (I-5)).
By this, lithium ethoxycarbonyl tosylamide was obtained as an eighth white solid (1.14 g, 4.56 mmol, yield = 34%).
The result of measuring this eighth white solid by $^1$H-NMR (DMSO-d6) is shown below.
$^1$H-NMR: δ1.01 (t,J = 7.0 Hz, 3H), 2.31 (s,3H), 3.68 (q,J = 7.3 Hz, 2H), 7.15 (d,J = 7.8 Hz, 2H), 7.58 (d,J = 8.1 Hz, 2H)

[Synthesis Example 5]

Lithium propoxycarbonyl tosylamide [Synthesis Compound (I-3)] represented by the following structural formula was synthesized in the following manner.

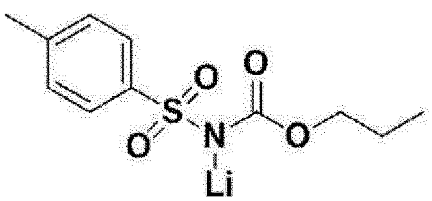

(I-3)

<First Step: Synthesis of Propylcarbonyl Tosylamide>

Propylcarbonyl tosylamide was synthesized in the same manner as in the first step of Synthesis Example 1, except that *p*-toluene sulfonyl isocyanate (4.75 g, 24.09 mmol) was used and n-butanol was changed to *n*-propanol (1.74 g, 28.9 mmol) in the first step of Synthesis Example 1 (Synthesis Compound (I-5)).
By this, propylcarbonyl tosylamide was obtained as a ninth white solid (5.70 g, 22.15 mmol, yield = 92%).
The result of measuring this ninth white solid by $^1$H-NMR (DMSO-d6) is shown below.
$^1$H-NMR: δ0.79 (t,J = 7.4 Hz, 3H), 1.32-1.48 (m,2H), 2.40 (s,3H), 3.92 (t,J = 6.8 Hz, 2H), 7.43 (d,J = 8.6 Hz, 2H), 7.78 (d,J = 8.4 Hz, 2H), 11.88 (br,1H)

<Second Step: Synthesis of Lithium Propoxycarbonyl Tosylamide (I-3)>

Using propylcarbonyl tosylamide (5.70 g, 22.15 mmol), tetrahydrofuran (50 mL) as a solvent, and a lithium bis(trimethylsilyl)amide (1.3M) tetrahydrofuran solution (17.0 mL, 22.15 mmol), a reaction was carried out in the same manner as in the second step of Synthesis Example 1 (Synthesis Compound (I-5)).
By this, lithium propoxycarbonyl tosylamide was obtained as a tenth white solid (3.66 g, 13.90 mmol, yield = 63%).
The result of measuring this tenth white solid by $^1$H-NMR (DMSO-d6) is shown below.
$^1$H-NMR: δ0.79 (t,J = 7.4 Hz, 3H), 1.32-1.48 (m,2H), 2.30 (s,3H), 3.58 (t,J = 6.8 Hz, 2H), 7.15 (d,J = 8.1 Hz, 2H), 7.58 (d,J = 8.4 Hz, 2H)

[Synthesis Example 6]

Lithium isopropoxycarbonyl tosylamide [Synthesis Compound (I-4)] represented by the following structural formula was synthesized in the following manner.

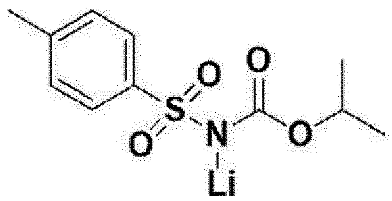

(I-4)

<First Step: Synthesis of Isopropylcarbonyl Tosylamide>

Isopropylcarbonyl tosylamide was synthesized in the same manner as in the first step of Synthesis Example 1, except that *n*-butanol was changed to isopropanol (2.40 g, 40 mmol) in the first step of Synthesis Example 1 (Synthesis Compound (I-5)).
By this, isopropylcarbonyl tosylamide was obtained as an 11th white solid (4.55 g, 17.68 mmol, yield = 88%).
The result of measuring this 11th white solid by $^1$H-NMR (DMSO-d6) is shown below.
$^1$H-NMR: δ1.10 (d,J = 5.94 Hz, 6H), 2.40 (s,3H), 4.72 (sept,J = 6.2 Hz, 1H), 7.43 (d,J = 8.4 Hz, 2H), 7.78 (d,J = 8.1 Hz, 2H), 11.77 (br,1H)

<Second Step: Synthesis of Lithium Isopropoxycarbonyl Tosylamide (I-4)>

Using isopropoxycarbonyl tosylamide (5.55 g, 21.57 mmol), tetrahydrofuran (50 mL) as a solvent, and a lithium bis(trimethylsilyl)amide (1.3M) tetrahydrofuran solution (16.6 mL, 21.57 mmol), a reaction was carried out in the same manner as in the second step of Synthesis Example 1 (Synthesis Compound (I-5)).
By this, lithium isopropoxycarbonyl tosylamide was obtained as a 12th white solid (5.46 g, 20.74 mmol, yield = 96%).
The result of measuring this 12th white solid by $^1$H-NMR (DMSO-d6) is shown below.
$^1$H-NMR: δ1.00 (d,J = 6.2 Hz, 3H), 2.31 (s,3H), 4.44 (sept,J = 6.1 Hz, 1H), 7.15 (d,J = 7.8 Hz, 2H), 7.58 (d,J = 8.4 Hz, 2H)

[Synthesis Example 7]

Lithium-*t*-butoxycarbonyl tosylamide [Synthesis Compound (I-6)] represented by the following structural formula was synthesized in the following manner.

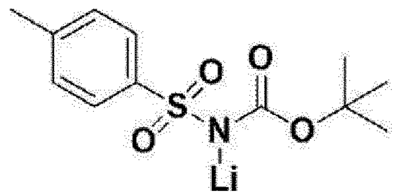

( I-6 )

<First Step: Synthesis of *t*-butylcarbonyl Tosylamide>

*T*-butylcarbonyl tosylamide was synthesized in the same manner as in the first step of Synthesis Example 1, except that *n*-butanol was changed to *t*-butylbutyl alcohol (1.78 g, 24 mmol) in the first step of Synthesis Example 1 (Synthesis Compound (I-5)).

By this, *t*-butylcarbonyl tosylamide was obtained as a 13th white solid (4.53 g, 16.70 mmol, yield = 83%).

The result of measuring this 13th white solid by $^1$H-NMR (DMSO-d6) is shown below.

$^1$H-NMR: δ1.29 (s,9H), 2.41 (s,3H), 7.44 (d,J = 8.4 Hz, 2H), 7.76 (d,J = 8.1 Hz, 2H), 11.53 (br,1H)

<Second Step: Synthesis of Lithium-*t*-Butoxycarbonyl Tosylamide (I-6)>

Using t-butylcarbonyl tosylamide (2.06 g, 7.59 mmol), tetrahydrofuran (30 mL) as a solvent, and a lithium bis(trimethylsilyl)amide (1.3M) tetrahydrofuran solution (5.8 mL, 7.59 mmol), a reaction was carried out in the same manner as in the second step of Synthesis Example 1 (Synthesis Compound (I-5)).

By this, lithium-*t*-butoxycarbonyl tosylamide was obtained as a 14th white solid (2.0 g, 7.21 mmol, yield = 95%).

The result of measuring this 14th white solid by $^1$H-NMR (DMSO-d6) is shown below.

$^1$H-NMR: δ1.20 (s,9H), 2.30 (s,3H), 7.14 (d,J = 8.1 Hz, 2H), 7.56 (d,J = 8.1 Hz, 2H)

[Synthesis Example 8]

Lithium-2,2,2-trifluoroethoxycarbonyl tosylamide [Synthesis Compound (I-7)] represented by the following structural formula was synthesized in the following manner.

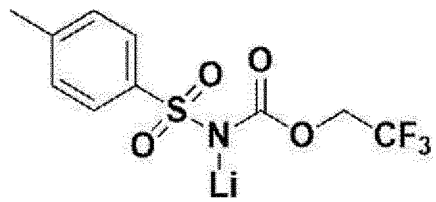

( I-7 )

<First Step: Synthesis of 2,2,2-trifluoroethoxycarbonyl Tosylamide>

2,2,2-trifluoroethoxycarbonyl tosylamide was synthesized in the same manner as in the first step of Synthesis Example 1, except that *p*-toluene sulfonyl isocyanate (4.73 g, 24.0 mmol) was used and *n*-butanol was changed to 2,2,2-trifluoroethanol (2.40 g, 24.0 mmol) in the first step of Synthesis Example 1 (Synthesis Compound (I-5)).

By this, 2,2,2-trifluoroethoxycarbonyl tosylamide was obtained as a 15th white solid (5.12 g, 17.22 mmol, yield = 72%).

The result of measuring this 15th white solid by $^1$H-NMR (DMSO-d6) is shown below.

$^1$H-NMR: δ2.41 (s,3H), 4.71 (q,J = 9.2 Hz, 2H), 7.45 (d,J = 8.4 Hz, 2H), 7.79 (d,J = 8.4 Hz, 2H)

<Second Step: Synthesis of Lithium-(2,2,2-trifluoroethoxy)tosylamide (I-7)>

Using 2,2,2-trifluoroethoxycarbonyl tosylamide (5.80 g, 19.51 mmol), tetrahydrofuran (50 mL) as a solvent, and a lithium bis(trimethylsilyl)amide (1.3M) tetrahydrofuran solution (15.0 mL, 19.51 mmol), a reaction was carried out in the same manner as in the second step of Synthesis Example 1 (Synthesis Compound (I-5)).

By this, lithium-(2,2,2-trifluoroethoxy)carbonyl tosylamide was obtained as a 16th white solid (4.98 g, 16.43 mmol, yield = 84%).

The result of measuring this 16th white solid by $^1$H-NMR (DMSO-d6) is shown below.

$^1$H-NMR: δ2.31 (s,3H), 4.29 (q,J = 9.5 Hz, 2H), 7.18 (d,J = 8.1 Hz, 2H), 7.60 (d,J = 8.1 Hz, 2H)

[Synthesis Example 9]

Lithium phenoxycarbonyl tosylamide [Synthesis Compound (I-8)] represented by the following structural formula was synthesized in the following manner.

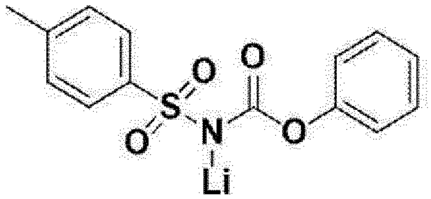

(I-8)

<First Step: Synthesis of Phenoxycarbonyl Tosylamide>

Phenoxycarbonyl tosylamide was synthesized in the same manner as in the first step of Synthesis Example 1, except that *p*-toluene sulfonyl isocyanate (8.32 g, 42.2 mmol) was used and *n*-butanol was changed to phenol (4.40 g, 46.7 mmol) in the first step of Synthesis Example 1 (Synthesis Compound (I-5)).

By this, phenoxycarbonyl tosylamide was obtained as a 17th white solid (10.33 g, 35.50 mmol, yield = 84%).

The result of measuring this 17th white solid by $^1$H-NMR (DMSO-d6) is shown below.

$^1$H-NMR: δ2.41 (s,3H), 7.05 (d,J = 8.6 Hz, 2H), 7.20-7.30 (m,1H), 7.36 (d,J = 7.6 Hz, 2H), 7.46 (d,J = 7.8 Hz, 2H), 7.83 (d,J = 7.8 Hz, 2H)

<Second Step: Synthesis of Lithium Phenoxycarbonyl Tosylamide (I-8)>

Using phenoxycarbonyl tosylamide (2.70 g, 9.27 mmol), tetrahydrofuran (50 mL) as a solvent, and a lithium bis(trimethylsilyl)amide (1.3M) tetrahydrofuran solution (7.1 mL, 9.27 mmol), a reaction was carried out in the same manner as in the second step of Synthesis Example 1 (Synthesis Compound (I-5)).

By this, lithium phenoxycarbonyl tosylamide was obtained as an 18th white solid (2.0 g, 6.80 mmol, yield = 73%).

The result of measuring this 18th white solid by $^1$H-NMR (DMSO-d6) is shown below.

$^1$H-NMR: δ2.32 (s,3H), 6.88 (d,J = 8.1 Hz, 2H), 7.03 (t,J = 4.1, 1H), 7.10-7.30 (m,4H), 7.63 (d,J = 8.1 Hz, 2H)

[Synthesis Example 10]

Lithium 2-methoxyethylcarbonyl tosylamide [Synthesis Compound (I-9)] represented by the following structural formula was synthesized in the following manner.

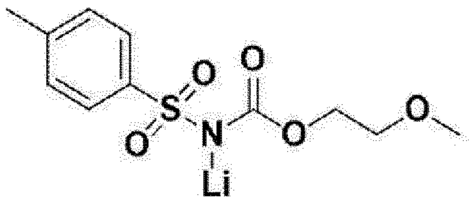

(I-9)

<First Step: Synthesis of 2-methoxyethylcarbonyl Tosylamide>

2-methoxyethylcarbonyl tosylamide was synthesized in the same manner as in the first step of Synthesis Example 1, except that *p*-toluene sulfonyl isocyanate (4.14 g, 21.0 mmol) was used and *n*-butanol was changed to 2-methoxyethanol (1.60 g, 21.0 mmol) in the first step of Synthesis Example 1 (Synthesis Compound (I-5)).

By this, 2-methoxyethylcarbonyl tosylamide was obtained as a 19th white solid (5.63 g, 20.60 mmol, yield = 98%).

The result of measuring this 19th white solid by $^1$H-NMR (DMSO-d6) is shown below.

$^1$H-NMR: δ2.40 (s,3H), 3.35-3.50 (m,2H), 4.00-4.15 (m,2H), 7.43 (d,J = 8.1 Hz, 2H), 7.78 (d,J = 8.4 Hz, 2H), 11.99 (br,1H)

<Second Step: Synthesis of Lithium 2-methoxyethylcarbonyl Tosylamide (I-9)>

Using 2-methoxyethylcarbonyl tosylamide (5.63 g, 20.60 mmol), tetrahydrofuran (100 mL) as a solvent, and a lithium bis(trimethylsilyl)amide (1.3M) tetrahydrofuran solution (15.8 mL, 20.60 mmol), a reaction was carried out in the

same manner as in the second step of Synthesis Example 1 (Synthesis Compound (I-5)).

By this, lithium 2-methoxyethylcarbonyl tosylamide was obtained as a 20th white solid (5.45 g, 19.52 mmol, yield = 95%).

The result of measuring this 20th white solid by $^1$H-NMR (DMSO-d6) is shown below.

$^1$H-NMR: δ2.31 (s,3H), 3.21 (s,3H), 3.30-3.40 (m,2H), 3.70-3.80 (m,2H), 7.16 (d,J = 8.1 Hz, 2H), 7.59 (d,J = 7.8 Hz, 2H)

[Synthesis Example 11]

Lithium ethoxycarbonyl trifluoromethyl sulfonamide [Synthesis Compound (1-11)] represented by the following structural formula was synthesized in the following manner.

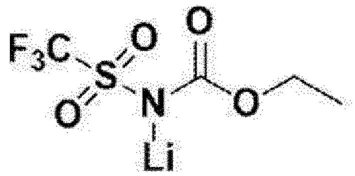

( I-11 )

<First Step: Synthesis of Ethoxycarbonyl Trifluoromethyl Sulfonamide>

Ethoxycarbonyl trifluoromethyl sulfonamide was synthesized in the same manner as in the first step of Synthesis Example 2, except that methyl chloroformate was changed to ethyl chloroformate (8.56 g, 79 mmol) in the first step of Synthesis Example 2 (Synthesis Compound (1-10)).

By this, ethoxycarbonyl trifluoromethyl sulfonamide was obtained as a 21st white solid (3.12 g, 33.6 mmol, yield = 45%).

The result of measuring this 21st white solid by $^1$H-NMR (DMSO-d6) is shown below.

$^1$H-NMR: δ1.28 (t,J = 4.2 Hz, 3H), 4.32 (q,J = 4.3 Hz, 2H), 12.04 (br,2H)

<Second Step: Synthesis of Lithium Ethoxycarbonyl Trifluoromethyl Sulfonamide (I-11)>

Using ethoxycarbonyl trifluoromethyl sulfonamide (2.01 g, 9.04 mmol), diethyl ether (30 mL) as a solvent, and a lithium bis(trimethylsilyl)amide (1.3M) tetrahydrofuran solution (7.0 mL, 9.04 mmol), a reaction was carried out in the same manner as in the second step of Synthesis Example 2 (Synthesis Compound (I-10)).

By this, lithium ethoxycarbonyl trifluoromethyl sulfonamide was obtained as a 22nd white solid (1.43 g, 6.30 mmol, yield = 70%).

The result of measuring this 22nd white solid by $^1$H-NMR (DMSO-d6) is shown below.

$^1$H-NMR: δ1.10 (t,J = 7.0 Hz, 3H), 3.83 (q,J = 7.0 Hz, 2H)

[Synthesis Example 12]

Lithium propoxycarbonyl trifluoromethyl sulfonamide [Synthesis Compound (I-12)] represented by the following structural formula was synthesized in the following manner.

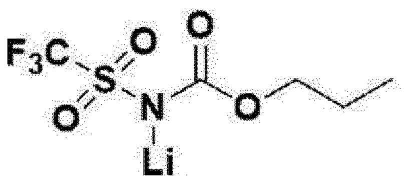

( I-12 )

<First Step: Synthesis of Propoxycarbonyl Trifluoromethyl Sulfonamide>

Propoxycarbonyl trifluoromethyl sulfonamide was synthesized in the same manner as in the first step of Synthesis Example 2, except that trifluoromethanesulfonamide (4.30 g, 28.8 mmol), pyridine (4.56 g, 57.7 mmol), 4-dimethylaminopyridine (0.71 g, 5.8 mmol), and tetrahydrofuran (50 mL) as a solvent were used, and methyl chloroformate was changed to propyl chloroformate (7.07 g, 57.7 mmol) in the first step of Synthesis Example 2 (Synthesis Compound (1-10)).

By this, propoxycarbonyl trifluoromethyl sulfonamide was obtained as a 23rd white solid (6.40 g, 27.2 mmol, yield = 94%).

The result of measuring this 23rd white solid by $^1$H-NMR (DMSO-d6) is shown below.
$^1$H-NMR: δ0.87 (t,J = 7.3 Hz, 3H), 1.30-1.55 (m,2H), 3.86 (t,J = 6.3 Hz, 2H)

<Second Step: Synthesis of Lithium Propoxycarbonyl Trifluoromethyl Sulfonamide (I-12)>

Using propoxycarbonyl trifluoromethyl sulfonamide (3.28 g, 13.95 mmol), diethyl ether (30 mL) as a solvent, and a lithium bis(trimethylsilyl)amide (1.3M) tetrahydrofuran solution (10.7 mL, 13.95 mmol), a reaction was carried out in the same manner as in the second step of Synthesis Example 2 (Synthesis Compound (1-10)).

By this, lithium propoxycarbonyl trifluoromethyl sulfonamide was obtained as a 24th white solid (2.13 g, 8.83 mmol, yield = 63%).

The result of measuring this 24th white solid by $^1$H-NMR (DMSO-d6) is shown below.
$^1$H-NMR: δ0.85 (t,J = 7.3 Hz, 3H), 1.35-1.60 (m,2H), 3.75 (t,J = 6.6 Hz, 2H)

[Synthesis Example 13]

Lithium isopropoxycarbonyl trifluoromethyl sulfonamide [Synthesis Compound (I-13)] represented by the following structural formula was synthesized in the following manner.

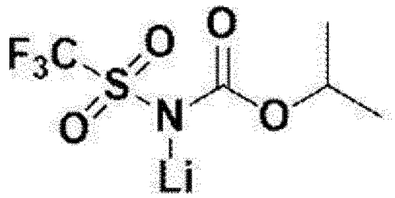

( I-13 )

<First Step: Synthesis of Isopropoxycarbonyl Trifluoromethyl Sulfonamide>

Isopropoxycarbonyl trifluoromethyl sulfonamide was synthesized in the same manner as in the first step of Synthesis Example 2, except that trifluoromethanesulfonamide (4.30 g, 28.8 mmol), pyridine (4.56 g, 57.7 mmol), 4-dimethylaminopyridine (0.71 g, 5.8 mmol), and tetrahydrofuran (50 mL) as a solvent were used, and methyl chloroformate was changed to isopropyl chloroformate (7.07 g, 57.7 mmol) in the first step of Synthesis Example 2 (Synthesis Compound (I-10)).

By this, isopropoxycarbonyl trifluoromethyl sulfonamide was obtained as a 25th white solid (5.56 g, 23.6 mmol, yield = 82%).

The result of measuring this 25th white solid by $^1$H-NMR (DMSO-d6) is shown below.
$^1$H-NMR: δ1.14 (d,J = 4.3 Hz, 3H), 1.16 (d,J = 4.3 Hz, 3H), 4.60-4.80 (m,1H)

<Second Step: Synthesis of Lithium Isopropoxycarbonyl Trifluoromethyl Sulfonamide (I-13)>

Using isopropoxycarbonyl trifluoromethyl sulfonamide (3.38 g, 14.37 mmol), diethyl ether (30 mL) as a solvent, and a lithium bis(trimethylsilyl)amide (1.3M) tetrahydrofuran solution (11.0 mL, 14.37 mmol), a reaction was carried out in the same manner as in the second step of Synthesis Example 2 (Synthesis Compound (1-10)).

By this, lithium isopropoxycarbonyl trifluoromethyl sulfonamide was obtained as a 26th white solid (2.55 g, 10.58 mmol, yield = 74%).

The result of measuring this 26th white solid by $^1$H-NMR (DMSO-d6) is shown below.
$^1$H-NMR: δ1.10 (d,J = 6.2 Hz,6H)

[Synthesis Example 14]

Lithium butoxycarbonyl trifluoromethyl sulfonamide [Synthesis Compound (I-14)] represented by the following structural formula was synthesized in the following manner.

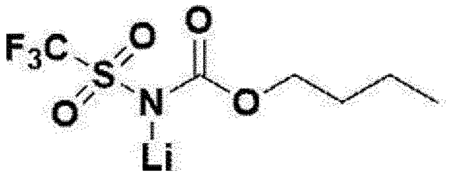

(I-14)

<First Step: Synthesis of Butoxycarbonyl Trifluoromethyl Sulfonamide>

Butoxycarbonyl trifluoromethyl sulfonamide was synthesized in the same manner as in the first step of Synthesis Example 2, except that trifluoromethanesulfonamide (5.0 g, 33.5 mmol), pyridine (5.31 g, 67.1 mmol), 4-dimethylaminopyridine (0.82 g, 6.7 mmol), and tetrahydrofuran (50 mL) as a solvent were used, and methyl chloroformate was changed to butyl chloroformate (9.16 g, 67.1 mmol) in the first step of Synthesis Example 2 (Synthesis Compound (I-10)).

By this, butoxycarbonyl trifluoromethyl sulfonamide was obtained as a 27th white solid (7.32 g, 29.4 mmol, yield = 88%).

The result of measuring this 27th white solid by $^1$H-NMR (DMSO-d6) is shown below.

$^1$H-NMR: $\delta$0.88 (t,J = 4.6 Hz, 3H), 1.12-1.14 (m,2H), 1.40-1.60 (m,2H), 3.88 (t,J = 6.6 Hz, 2H), 8.94 (br,1H)

<Second Step: Synthesis of Lithium Butoxycarbonyl Trifluoromethyl Sulfonamide (I-14)>

Using butoxycarbonyl trifluoromethyl sulfonamide (2.50 g, 10.03 mmol), diethyl ether (30 mL) as a solvent, and a lithium bis(trimethylsilyl)amide (1.3M) tetrahydrofuran solution (7.7 mL, 10.03 mmol), a reaction was carried out in the same manner as in the second step of Synthesis Example 2 (Synthesis Compound (I-10)).

By this, lithium butoxycarbonyl trifluoromethyl sulfonamide was obtained as a 28th white solid (2.21 g, 8.66 mmol, yield = 86%).

The result of measuring this 28th white solid by $^1$H-NMR (DMSO-d6) is shown below.

$^1$H-NMR: $\delta$0.87 (t,J = 7.2 Hz, 3H), 1.15-1.55 (m,4H), 3.79 (t,J = 6.6 Hz, 2H)

[Synthesis Example 15]

Lithium phenoxycarbonyl trifluoromethyl sulfonamide [Synthesis Compound (I-15)] represented by the following structural formula was synthesized in the following manner.

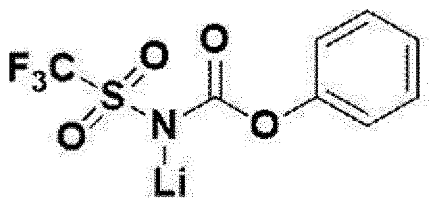

(I-15)

<First Step: Synthesis of Phenoxycarbonyl Trifluoromethyl Sulfonamide>

Phenoxycarbonyl trifluoromethyl sulfonamide was synthesized in the same manner as in the first step of Synthesis Example 2, except that trifluoromethanesulfonamide (3.0 g, 20.1 mmol), pyridine (3.18 g, 40.2 mmol), 4-dimethylaminopyridine (0.49 g, 4.0 mmol), and tetrahydrofuran (30 mL) as a solvent were used, and methyl chloroformate was changed to phenyl chloroformate (3.15 g, 20.1 mmol) in the first step of Synthesis Example 2 (Synthesis Compound (I-10)).

By this, phenoxycarbonyl trifluoromethyl sulfonamide was obtained as a 29th white solid (1.83 g, 6.8 mmol, yield = 34%).

The result of measuring this 29th white solid by $^1$H-NMR (DMSO-d6) is shown below.

$^1$H-NMR: $\delta$6.70-6.85 (m,3H), 7.16 (t,J = 7.8 Hz, 2H), 9.33 (br,1H)

<Second Step: Synthesis of Lithium Phenoxycarbonyl Trifluoromethyl Sulfonamide (I-15)>

Using phenoxycarbonyl trifluoromethyl sulfonamide (1.73 g, 6.43 mmol), diethyl ether (30 mL) as a solvent, and a lithium bis(trimethylsilyl)amide (1.3M) tetrahydrofuran solution (4.9 mL, 6.43 mmol), a reaction was carried out in the same manner as in the second step of Synthesis Example 2 (Synthesis Compound (I-10)).

By this, lithium phenoxycarbonyl trifluoromethyl sulfonamide was obtained as a 30th white solid (0.78 g, 2.83

mmol, yield = 44%).

The result of measuring this 30th white solid by $^1$H-NMR (DMSO-d6) is shown below.

$^1$H-NMR: δ5.98 (t,J = 6.9 Hz, 1H), 6.23 (d,J = 7.6 Hz, 2H), 6.76 (t,J = 7.4 Hz, 2H)

[Synthesis Example 16]

Lithium methoxycarbonylmethyl sulfonamide [Synthesis Compound (I-16)] represented by the following structural formula was synthesized in the following manner.

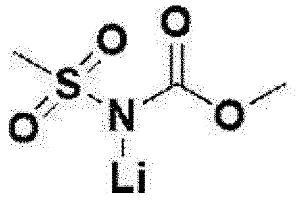

(I-16)

<First Step: Synthesis of Methoxycarbonylmethyl Sulfonamide>

Methoxycarbonylmethyl sulfonamide was synthesized in the same manner as in the first step of Synthesis Example 2, except that methyl chloroformate (19.87 g, 210 mmol), triethylamine (21.28 g, 210 mmol), 4-dimethylaminopyridine (2.57 g, 21 mmol), and tetrahydrofuran (200 mL) as a solvent were used, and trifluoromethanesulfonamide was changed to methanesulfonamide (10 g, 105 mmol) in the first step of Synthesis Example 2 (Synthesis Compound (I-10)).

By this, methoxycarbonylmethyl sulfonamide was obtained as a 31st white solid (0.65 g, 4.24 mmol, yield = 4%).

The result of measuring this 31st white solid by $^1$H-NMR (CD3OD-d4) is shown below.

$^1$H-NMR: δ3.24 (s,3H), 3.77 (s,3H)

<Second Step: Synthesis of Lithium Methoxycarbonylmethyl Sulfonamide (I-16)>

Using methoxycarbonylmethyl sulfonamide (0.50 g, 3.25 mmol), tetrahydrofuran (20 mL) as a solvent, and a lithium bis(trimethylsilyl)amide (1.3M) tetrahydrofuran solution (2.5 mL, 3.25 mmol), a reaction was carried out in the same manner as in the second step of Synthesis Example 2 (Synthesis Compound (I-10)).

By this, lithium methoxycarbonylmethyl sulfonamide was obtained as a 32nd white solid (0.31 g, 1.96 mmol, yield = 60%).

The result of measuring this 32nd white solid by $^1$H-NMR (DMSO-d6) is shown below.

$^1$H-NMR: δ2.61 (s,3H), 3.27 (s,3H)

[Synthesis Example 17]

Lithium-t-butoxycarbonylmethyl sulfonamide [Synthesis Compound (I-17)] represented by the following structural formula was synthesized in the following manner.

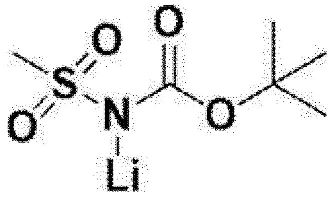

(I-17)

<First Step: Synthesis of t-butoxycarbonylmethyl Sulfonamide>

T-butoxycarbonylmethyl sulfonamide was synthesized in the same manner as in the first step of Synthesis Example 16, except that methanesulfonamide (4.0 g, 42.1 mmol), triethylamine (5.11 g, 210 mmol), 4-dimethylaminopyridine (0.62 g, 5.1 mmol), and tetrahydrofuran (50 mL) as a solvent were used, and methyl chloroformate was changed to dit-butyl dioxide (9.18 g, 42.1 mmol) in the first step of Synthesis Example 16 (Synthesis Compound (I-16)).

By this, *t*-butoxycarbonylmethyl sulfonamide was obtained as a 33rd white solid (5.22 g, 26.7 mmol, yield = 64%).

The result of measuring this 33rd white solid by $^1$H-NMR (DMSO-d6) is shown below.
$^1$H-NMR: δ1.44 (s,9H), 3.20 (s,3H)

<Second Step: Synthesis of Lithium-*t*-butoxycarbonylmethyl Sulfonamide (I-17)>

Using *t*-butoxycarbonylmethyl sulfonamide (5.0 g, 25.6 mmol), tetrahydrofuran (50 mL) as a solvent, and a lithium bis(trimethylsilyl)amide (1.3M) tetrahydrofuran solution (19.7 mL, 25.6 mmol), a reaction was carried out in the same manner as in the second step of Synthesis Example 2 (Synthesis Compound (I-10)).

By this, lithium-*t*-butoxycarbonyl trifluoromethyl sulfonamide was obtained as a 34th white solid (4.91 g, 24.5 mmol, yield = 95%).

The result of measuring this 34th white solid by $^1$H-NMR (DMSO-d6) is shown below.
$^1$H-NMR: δ1.30 (s,9H), 2.64 (s,3H)

[Synthesis Example 18]

Lithium phenoxycarbonylmethyl sulfonamide [Synthesis Compound (1-18)] represented by the following structural formula was synthesized in the following manner.

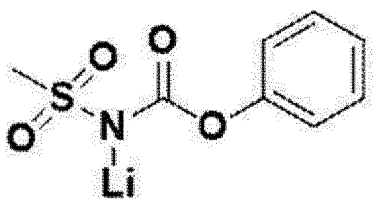

( I-18 )

<First Step: Synthesis of Phenoxycarbonylmethyl Sulfonamide>

Phenoxycarbonylmethyl sulfonamide was synthesized in the same manner as in the first step of Synthesis Example 2, except that methanesulfonamide (3.0 g, 31.5 mmol), pyridine (4.99 g, 63.1 mmol), 4-dimethylaminopyridine (0.77 g, 6.3 mmol), and tetrahydrofuran (30 mL) as a solvent were used, and methyl chloroformate was changed to phenyl chloroformate (5.93 g, 37.8 mmol) in the first step of Synthesis Example 2 (Synthesis Compound (1-10)).

By this, phenoxycarbonylmethyl sulfonamide was obtained as a 35th white solid (3.25 g, 15.1 mmol, yield = 48%).

The result of measuring this 35th white solid by $^1$H-NMR (DMSO-d6) is shown below.
$^1$H-NMR: δ3.31 (s,3H), 7.22 (d,J = 8.4 Hz, 2H), 7.25-7.50 (m,3H)

<Second Step: Synthesis of Lithium Phenoxycarbonylmethyl Sulfonamide (I-18)>

Using phenoxycarbonylmethyl sulfonamide (2.11 g, 9.8 mmol), tetrahydrofuran (30 mL) as a solvent, and a lithium bis(trimethylsilyl)amide (1.3M) tetrahydrofuran solution (7.5 mL, 9.8 mmol), a reaction was carried out in the same manner as in the second step of Synthesis Example 2 (Synthesis Compound (I-10)).

By this, lithium phenoxycarbonylmethyl sulfonamide was obtained as a 36th white solid (1.96 g, 8.9 mmol, yield = 90%).

The result of measuring this 36th white solid by $^1$H-NMR (DMSO-d6) is shown below.
$^1$H-NMR: δ3.31 (s,3H), 6.97 (d,J = 8.6 Hz, 2H), 7.06 (t,J = 7.3 Hz, 1H), 7.28 (t,J = 7.8 Hz, 2H)

[Synthesis Example 19]

Lithium acetylethoxy sulfonamide [Synthesis Compound (1-19)] represented by the following structural formula was synthesized in the following manner.

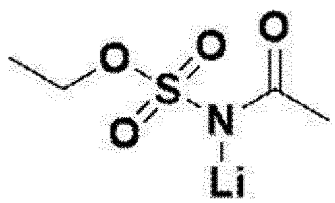

( I-19 )

<First Step: Synthesis ofAcetylethoxy Sulfonamide>

Acetylethoxy sulfonamide ethyl was synthesized in accordance with Patent Document (WO 2017/156179).

To a nitrogen-purged 200-mL four-necked flask, chlorosulfonyl isocyanate (7.08 g, 50 mmol) and dichloromethane (100 mL) as a solvent were charged. This flask was maintained at 0°C, and acetic acid (3.0 g, 50 mmol) was added thereto, after which the flask was brought back to room temperature, and a reaction was allowed to proceed with stirring for 6 hours. After the completion of the reaction, the solvent was removed by concentration to obtain a white solid. Tetrahydrofuran (50 mL) was charged to this four-necked flask to prepare a first reaction solution, which was then maintained at 0°C.

Separately, ethanol (2.92 g, 63.5 mmol), pyridine (5.02 g, 63.5 mmol), and 4-dimethylaminopyridine (0.78 g, 6.4 mmol) were added to tetrahydrofuran (50 mL) to prepare a second reaction solution. This second reaction solution was added to the four-necked flask containing the first reaction solution at 0°C over a period of 10 minutes, after which the flask was brought back to room temperature, and a reaction was allowed to proceed with stirring for 6 hours. Subsequently, the resulting reaction solution was filtered to remove hydrochloride, and the thus obtained solution was washed. In other words, the filtrate was extracted and washed using a separatory funnel with addition of 100 mL of distilled water and 100 mL of ethyl acetate.

The above-described washing with water was repeated twice, and the resulting organic layer was dried with an addition of magnesium sulfate and then concentrated under reduced pressure.

The thus obtained crude product was separated and purified by flash column chromatography using a hexane/ethyl acetate solvent.

In this manner, acetylethoxy sulfonamide was obtained as a 37th white solid (2.87 g, 17.2 mmol, yield = 56%).

The result of measuring this 37th white solid by $^1$H-NMR (DMSO-d6) is shown below.

$^1$H-NMR: $\delta$1.28 (d,J = 4.3 Hz, 3H), 2.02 (s,3H), 4.27 (q,J = 4.3 Hz, 2H), 12.04 (br,1H)

<Second Step: Synthesis of Lithium Acetylethoxy Sulfonamide (I-19)>

To a nitrogen-purged 200-mL four-necked flask, acetylethoxy sulfonamide (1.74 g, 10.4 mmol) and tetrahydrofuran (30 mL) as a solvent were charged. This flask was maintained at -20°C, and a lithium bis(trimethylsilyl)amide (1.3M) tetrahydrofuran solution (8.0 mL, 10.4 mmol) was added thereto over a period of 5 minutes, after which the flask was brought back to room temperature, and a reaction was allowed to proceed with stirring for 3 hours. Subsequently, *n*-hexane (30 mL) was added to allow a 38th white solid to precipitate from the resulting reaction solution. This reaction solution was vacuum-filtered to remove the residual solvent of the thus obtained 38th white solid by vacuum distillation.

In this manner, lithium acetylethoxy sulfonamide was obtained as the 38th white solid (0.22 g, 1.27 mmol, yield = 12%).

The result of measuring this 38th white solid by $^1$H-NMR (DMSO-d6) is shown below.

$^1$H-NMR: $\delta$1.13 (t,J = 7.2 Hz, 3H), 1.71 (s,3H), 3.86 (q,J = 7.3 Hz, 2H)

[Synthesis Example 20]

Lithium ethoxysulfonyl-2,2,2-trifluoroacetylamide [Synthesis Compound (I-20)] represented by the following structural formula was synthesized in the following manner.

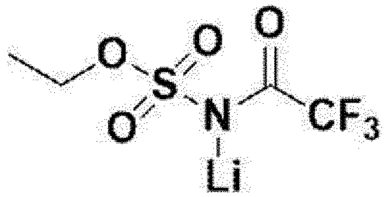

( I-20 )

<First Step: Synthesis of Ethoxysulfonyl-(2,2,2-trifluoroacetyl)amide>

Ethyl sulfamate (6.45 g, 51.5 mmol, yield = 62%) was synthesized in accordance with Non-Patent Document (Journal of the Chemical Society. Perkin transactions I, 1982, p. 677-680).

To a nitrogen-purged 200-mL four-necked flask, ethyl sulfamate (2.05 g, 16.4 mmol), pyridine (1.56 g, 19.7 mmol), 4-dimethylaminopyridine (0.24 g, 2.0 mmol), and tetrahydrofuran (30 mL) as a solvent were charged. This flask was maintained at 0°C, and trifluoroacetic anhydride (4.13 g, 19.7 mmol) was added thereto over a period of 10 minutes, after which the flask was brought back to room temperature, and a reaction was allowed to proceed with stirring for 6

hours. Subsequently, the resulting reaction solution was filtered to remove hydrochloride, and the thus obtained solution was washed. In other words, the filtrate was extracted and washed using a separatory funnel with addition of 100 mL of distilled water and 100 mL of ethyl acetate.

The above-described washing with water was repeated twice, and the resulting organic layer was dried with an addition of magnesium sulfate and then concentrated under reduced pressure.

The thus obtained crude product was separated and purified by flash column chromatography using a hexane/ethyl acetate solvent.

In this manner, ethoxysulfonyl-(2,2,2-trifluoroacetyl)amide was obtained as the 39th white solid (1.22 g, 5.52 mmol, yield = 34%).

The result of measuring this 39th white solid by $^{1}$H-NMR (DMSO-d6) is shown below.

$^{1}$H-NMR: $\delta$1.18 (t,J = 4.5 Hz, 3H), 3.98 (q,J = 7.0 Hz, 2H), 8.38 (br,1H)

<Second Step: Synthesis of Lithium Ethoxysulfonyl-(2,2,2-trifluoroacetyl)amide (1-20)>

To a nitrogen-purged 200-mL four-necked flask, ethoxysulfonyl-(2,2,2-trifluoroacetyl)amide (1.0 g, 4.5 mmol) and tetrahydrofuran (20 mL) as a solvent were charged. This flask was maintained at -20°C, and a lithium bis(trimethylsilyl)amide (1.3M) tetrahydrofuran solution (3.5 mL, 4.5 mmol) was added thereto over a period of 5 minutes, after which the flask was brought back to room temperature, and a reaction was allowed to proceed with stirring for 3 hours. Subsequently, *n*-hexane (20 mL) was added to allow a white solid to precipitate from the resulting reaction solution. This reaction solution was vacuum-filtered to remove the residual solvent of the thus obtained solid by vacuum distillation.

In this manner, lithium ethoxysulfonyl-(2,2,2-trifluoroacetyl)amide was obtained as a 40th white solid (0.12 g, 0.53 mmol, yield = 12%).

The result of measuring this 40th white solid by $^{1}$H-NMR (DMSO-d6) is shown below.

$^{1}$H-NMR: $\delta$1.08 (dt,J = 7.0 Hz, 1.1 Hz, H), 3.68 (dq,J = 7.0 Hz, 1.1 Hz, 2H)

[Synthesis Example 21]

Lithium propionyl-(2,2,2-trifluoroethoxy)sulfonamide [Synthesis Compound (I-21)] represented by the following structural formula was synthesized in the following manner.

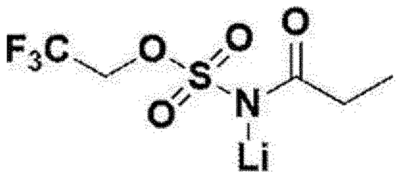

(I-21)

<First Step: Synthesis of Propionyl-(2,2,2-trifluoroethoxy)sulfonamide>

Propionyl-(2,2,2-trifluoroethoxy)sulfonamide was synthesized in accordance with Patent Document (WO 2017/156179).

To a nitrogen-purged 200-mL four-necked flask, chlorosulfonyl isocyanate (14.15 g, 100 mmol) and dichloromethane (100 mL) as a solvent were charged. This flask was maintained at 0°C, and propionic acid (7.41 g, 100 mmol) was added thereto, after which the flask was brought back to room temperature, and a reaction was allowed to proceed with stirring for 6 hours. After the completion of the reaction, the solvent was removed by concentration to obtain a white solid. Tetrahydrofuran (100 mL) was charged to this four-necked flask to prepare a third reaction solution, which was then maintained at 0°C.

Separately, 2,2,2-trifluoroethanol (12.0 g, 120 mmol), triethylamine (20.24 g, 200 mmol), and 4-dimethylaminopyridine (2.44 g, 20 mmol) were added to tetrahydrofuran (100 mL) to prepare a fourth reaction solution. This fourth reaction solution was added to the four-necked flask containing the third reaction solution at 0°C over a period of 10 minutes, after which the flask was brought back to room temperature, and a reaction was allowed to proceed with stirring for 6 hours. Subsequently, the resulting reaction solution was filtered to remove hydrochloride, and the thus obtained solution was washed. In other words, the filtrate was extracted and washed using a separatory funnel with addition of 100 mL of distilled water and 100 mL of ethyl acetate.

The above-described washing with water was repeated twice, and the resulting organic layer was dried with an addition of magnesium sulfate and then concentrated under reduced pressure.

The thus obtained crude product was separated and purified by flash column chromatography using a hexane/ethyl acetate solvent.

In this manner, propionyl-(2,2,2-trifluoroethoxy)sulfonamide was obtained as a 41st white solid (11.63 g, 49.5 mmol, yield = 50%).

The result of measuring this 41st white solid by $^1$H-NMR (DMSO-d6) is shown below.

$^1$H-NMR: δ1.00 (t,J = 7.4 Hz, 3H), 2.32 (q,J = 7.8 Hz, 2H), 4.93 (q,J = 8.6 Hz, 2H)

<Second Step: Synthesis of Lithium Propionyl-(2,2,2-trifluoroethoxy)sulfonamide (1-21)>

To a nitrogen-purged 200-mL four-necked flask, propionyl-(2,2,2-trifluoroethoxy)sulfonamide (1.75 g, 7.4 mmol) and tetrahydrofuran (30 mL) as a solvent were charged. This flask was maintained at -20°C, and a lithium bis(trimethylsilyl)amide (1.3M) tetrahydrofuran solution (5.7 mL, 7.4 mmol) was added thereto over a period of 5 minutes, after which the flask was brought back to room temperature, and a reaction was allowed to proceed with stirring for 3 hours. Subsequently, n-hexane (30 mL) was added to allow a 42nd white solid to precipitate from the resulting reaction solution. This reaction solution was vacuum-filtered to remove the residual solvent of the thus obtained 42nd white solid by vacuum distillation.

In this manner, lithium propionyl-(2,2,2-trifluoroethoxy)sulfonamide was obtained as the 42nd white solid (0.79 g, 3.28 mmol, yield = 44%).

The result of measuring this 42nd white solid by $^1$H-NMR (DMSO-d6) is shown below.

$^1$H-NMR: δ0.91 (t,J = 7.6 Hz, 3H), 1.98 (q,J = 7.6 Hz, 2H), 4.44 (q,J = 6.5 Hz, 2H)

[Synthesis Example 22]

Lithium benzoyl-(2,2,2-trifluoroethoxy)sulfonamide [Synthesis Compound (I-22)] represented by the following structural formula was synthesized in the following manner.

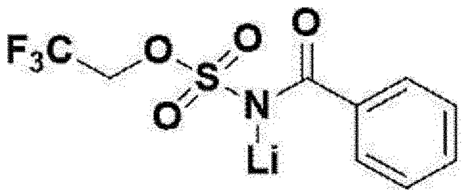

( I-22 )

<First Step: Synthesis of Benzoyl-(2,2,2-trifluoroethoxy)sulfonamide>

2,2,2-trifluoroethyl sulfamate (12.4 g, 69.2 mmol, yield = 69%) was synthesized in accordance with Non-Patent Document (Journal of the Chemical Society. Perkin transactions I, 1982, p. 677-680).

To a nitrogen-purged 200-mL four-necked flask, 2,2,2-trifluoroethylethyl sulfamate (2.50 g, 14.0 mmol), triethylamine (2.82 g, 27.9 mmol), 4-dimethylaminopyridine (0.34 g, 2.8 mmol), and tetrahydrofuran (50 mL) as a solvent were charged. This flask was maintained at 0°C, and benzoyl chloride (2.35 g, 16.8 mmol) was added thereto over a period of 5 minutes, after which the flask was brought back to room temperature, and a reaction was allowed to proceed with stirring for 6 hours. Subsequently, the resulting reaction solution was filtered to remove hydrochloride, and the thus obtained solution was washed. In other words, the filtrate was extracted and washed using a separatory funnel with addition of 100 mL of distilled water and 100 mL of ethyl acetate.

The above-described washing with water was repeated twice, and the resulting organic layer was dried with an addition of magnesium sulfate and then concentrated under reduced pressure.

The thus obtained crude product was separated and purified by flash column chromatography using a hexane/ethyl acetate solvent.

In this manner, benzoyl-(2,2,2-trifluoroethoxy)sulfonamide was obtained as a 43rd white solid (3.22 g, 11.37 mmol, yield = 81%).

The result of measuring this 43rd white solid by $^1$H-NMR (DMSO-d6) is shown below.

$^1$H-NMR: δ5.01 (q,J = 8.6 Hz, 2H), 7.52 (t,J = 7.8 Hz, 2H), 7.65 (t,J = 7.0 Hz, 1H), 7.94 (d,J = 8.4 Hz, 2H)

<Second Step: Synthesis of Lithium Benzoyl-(2,2,2-trifluoroethoxy)sulfonamide (I-22)>

To a nitrogen-purged 200-mL four-necked flask, benzoyl-(2,2,2-trifluoroethoxy)sulfonamide (2.06 g, 7.3 mmol) and tetrahydrofuran (20 mL) as a solvent were charged. This flask was maintained at -20°C, and a lithium bis(trimethylsilyl)amide (1.3M) tetrahydrofuran solution (5.6 mL, 7.3 mmol) was added thereto over a period of 5 minutes, after which the flask was brought back to room temperature, and a reaction was allowed to proceed with stirring for 3 hours.

Subsequently, *n*-hexane (20 mL) was added to allow a 44th white solid to precipitate from the resulting reaction solution. This reaction solution was vacuum-filtered to remove the residual solvent of the thus obtained 44th white solid by vacuum distillation.

In this manner, lithium benzoyl-(2,2,2-trifluoroethoxy)sulfonamide was obtained as the 44th white solid (1.13 g, 3.9 mmol, yield = 54%).

The result of measuring this 44th white solid by $^1$H-NMR (DMSO-d6) is shown below.

$^1$H-NMR: δ4.58 (q,J = 8.9 Hz, 2H), 7.52 (t,J = 7.8 Hz, 2H), 7.25-7.50 (m,3H), 7.93 (d,J = 7.6 Hz, 2H)

[Synthesis Example 23]

Lithium benzoylethoxy sulfonamide [Synthesis Compound (I-23)] represented by the following structural formula was synthesized in the following manner.

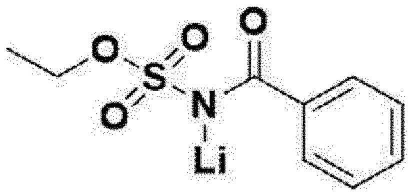

( I-23 )

<First Step: Synthesis of Benzoylethoxy Sulfonamide>

Benzoylethoxy sulfonamide was synthesized in the same manner as in the first step of Synthesis Example 20, except that ethyl sulfamate (2.02 g, 16.1 mmol), 4-dimethylaminopyridine (0.39 g, 3.2 mmol), and tetrahydrofuran (30 mL) as a solvent were used, and trifluoroacetic anhydride and pyridine were changed to benzoyl chloride (2.72 g, 19.4 mmol) and triethylamine (3.27 g, 32.3 mmol), respectively, in the first step of Synthesis Example 20 (Synthesis Compound (I-20)).

By this, benzoylethoxy sulfonamide was obtained as a 45th white solid (1.95 g, 16.14 mmol, yield = 53%).

The result of measuring this 45th white solid by $^1$H-NMR (DMSO-d6) is shown below.

$^1$H-NMR: δ1.32 (t,J = 7.2 Hz, 2H), 4.38 (q,J = 7.3 Hz, 2H), 7.53 (t,J = 7.6 Hz, 2H), 7.60 (t,J = 7.4 Hz, 2H), 7.92 (d,J = 7.4 Hz, 2H), 12.48 (br,1H)

<Second Step: Synthesis of Lithium Benzoylethoxy Sulfonamide (I-23)>

To a nitrogen-purged 200-mL four-necked flask, benzoylethoxy sulfonamide (1.74 g, 7.6 mmol) and tetrahydrofuran (20 mL) as a solvent were charged. This flask was maintained at -20°C, and a lithium bis(trimethylsilyl)amide (1.3M) tetrahydrofuran solution (5.8 mL, 7.6 mmol) was added thereto over a period of 5 minutes, after which the flask was brought back to room temperature, and a reaction was allowed to proceed with stirring for 3 hours. Subsequently, *n*-hexane (20 mL) was added to allow a 46th white solid to precipitate from the resulting reaction solution. This reaction solution was vacuum-filtered to remove the residual solvent of the thus obtained 46th white solid by vacuum distillation.

In this manner, lithium benzoylethoxy sulfonamide was obtained as the 46th white solid (1.29 g, 5.5 mmol, yield = 72%).

The result of measuring this 46th white solid by $^1$H-NMR (DMSO-d6) is shown below.

$^1$H-NMR: δ1.17 (t,J = 7.2 Hz, 2H), 3.96 (t,J = 7.0 Hz, 2H), 7.25-7.45 (m,3H), 7.91 (d,J = 7.0 Hz, 2H)

[Synthesis Example 24]

Lithium acetylphenoxy sulfonamide [Synthesis Compound (I-24)] represented by the following structural formula was synthesized in the following manner.

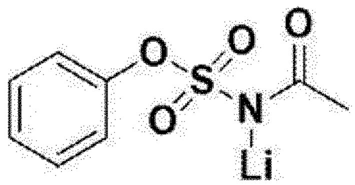

( I-24 )

<First Step: Synthesis of Phenyl Sulfamate>

Sulfamoyl chloride was synthesized in accordance with Non-Patent Document (Journal of the Chemical Society. Perkin transactions I, 1982, p. 677-680).

To a nitrogen-purged 200-mL four-necked flask, sulfamoyl chloride (32.1 g, 278 mmol) and tetrahydrofuran (300 mL) as a solvent were charged to prepare a fifth reaction solution, which was then maintained at 0°C.

Separately, phenol (26.2 g, 278 mmol) and sodium hydride (6.67 g, 278 mmol) were added to tetrahydrofuran (100 mL) at 0°C to prepare a sixth reaction solution. This sixth reaction solution was added to the four-necked flask containing the fifth reaction solution at 0°C over a period of 30 minutes, after which the flask was brought back to room temperature, and a reaction was allowed to proceed with stirring for 6 hours. Subsequently, the resulting reaction solution was filtered to remove salts, and the thus obtained solution was washed. In other words, the filtrate was extracted and washed using a separatory funnel with addition of 100 mL of distilled water and 100 mL of ethyl acetate.

The above-described washing with water was repeated twice, and the resulting organic layer was dried with an addition of magnesium sulfate and then concentrated under reduced pressure.

The thus obtained crude product was separated and purified by flash column chromatography using a hexane/ethyl acetate solvent.

In this manner, phenyl sulfamate was obtained as a 47th white solid (18.5 g, 107 mmol, yield = 38%).

The result of measuring this 47th white solid by $^1$H-NMR (DMSO-d6) is shown below.

$^1$H-NMR: δ1.41 (t,J = 7.0 Hz, 3H), 4.29 (q,J = 7.3 Hz, 2H), 4.91 (br,2H)

The reaction formula in the first step of Synthesis Example 24 is as follows.

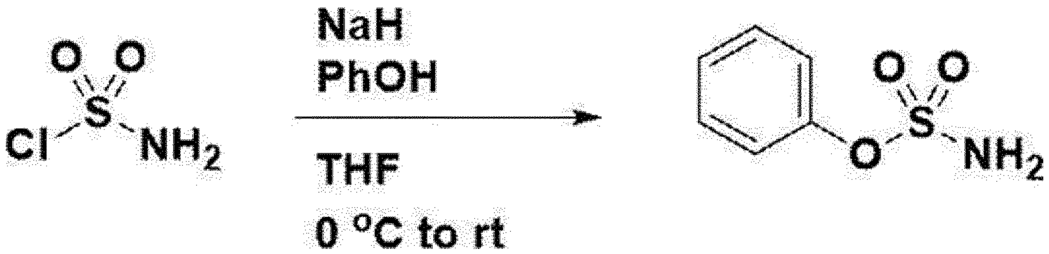


<Second Step: Synthesis of Acetylphenoxy Sulfonamide>

Acetylphenoxy sulfonamide was synthesized in the same manner as in the first step of Synthesis Example 2, except that pyridine (4.99 g, 63.1 mmol), 4-dimethylaminopyridine (0.77 g, 6.3 mmol), and tetrahydrofuran (30 mL) as a solvent were used, and methanesulfonamide and methyl chloroformate were changed to phenyl sulfamate (2.68 g, 15.5 mmol) and acetyl chloride (1.46 g, 18.6 mmol), respectively, in the first step of Synthesis Example 2 (Synthesis Compound (I-10)).

By this, acetylphenoxy sulfonamide was obtained as a black solid (2.11 g, 9.8 mmol, yield = 63%).

The result of measuring this black solid by $^1$H-NMR (DMSO-d6) is shown below.

$^1$H-NMR: δ2.05 (s,3H), 7.27 (t,J = 8.6 Hz, 2H), 7.35-7.60 (m,3H), 12.44 (br,1H)

<Third Step: Synthesis of Lithium Acetylphenoxy Sulfonamide (I-24)>

To a nitrogen-purged 200-mL four-necked flask, acetylphenoxy sulfonamide (2.11 g, 9.8 mmol) and tetrahydrofuran (20 mL) as a solvent were charged. This flask was maintained at -20°C, and a lithium bis(trimethylsilyl)amide (1.3M) tetrahydrofuran solution (7.5 mL, 9.8 mmol) was added thereto over a period of 5 minutes, after which the flask was brought back to room temperature, and a reaction was allowed to proceed with stirring for 3 hours. Subsequently, *n*-hexane (20 mL) was added to allow a 48th white solid to precipitate from the resulting reaction solution. This reaction solution was vacuum-filtered to remove the residual solvent of the thus obtained 48th white solid by vacuum distillation.

In this manner, lithium acetylphenoxy sulfonamide was obtained as the 48th white solid (1.83 g, 8.27 mmol, yield = 84%).

The result of measuring this 48th white solid by $^1$H-NMR (DMSO-d6) is shown below.

$^1$H-NMR: δ1.72 (s,3H), 7.05-7.20 (m,3H), 7.30 (t,J = 5.1 Hz, 2H)

[Synthesis Example 25]

Lithium benzoylphenoxy sulfonamide [Synthesis Compound (I-25)] represented by the following structural formula was synthesized in the following manner.

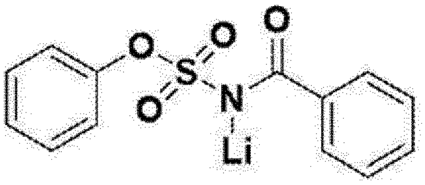

( I-25 )

<First Step: Synthesis of Phenyl Sulfamate>

Phenyl sulfamate was obtained in the same manner as in the first step of Synthesis Example 24 (Synthesis Compound (I-24)).

<Second Step: Synthesis of Benzoylphenoxy Sulfonamide>

A 49th white solid was synthesized in the same manner as in the first step of Synthesis Example 24, except that phenyl sulfamate (4.10 g, 23.7 mmol), 4-dimethylaminopyridine (0.58 g, 5.8 mmol), and tetrahydrofuran (50 mL) as a solvent were used, and acetyl chloride and pyridine were changed to benzoyl chloride (3.99 g, 28.4 mmol) and triethylamine (4.79 g, 47.3 mmol), respectively, in the second step of Synthesis Example 24 (Synthesis Compound (I-24)).
By this, benzoylphenoxy sulfonamide was obtained as the 49th white solid (4.93 g, 17.8 mmol, yield = 75%).
The result of measuring this 49th white solid by $^1$H-NMR (DMSO-d6) is shown below.
$^1$H-NMR: $\delta$7.29 (d,J = 8.4 Hz, 2H), 7.30-7.70 (m,6H), 7.89 (d,J = 7.3 Hz, 2H)

<Third Step: Lithium Benzoylphenoxy Sulfonamide (I-25)>

To a nitrogen-purged 200-mL four-necked flask, benzoylphenoxy sulfonamide (3.12 g, 11.25 mmol) and tetrahydrofuran (30 mL) as a solvent were charged. This flask was maintained at -20°C, and a lithium bis(trimethylsilyl)amide (1.3M) tetrahydrofuran solution (8.7 mL, 11.25 mmol) was added thereto over a period of 5 minutes, after which the flask was brought back to room temperature, and a reaction was allowed to proceed with stirring for 3 hours. Subsequently, *n*-hexane (30 mL) was added to allow a 50th white solid to precipitate from the resulting reaction solution. This reaction solution was vacuum-filtered to remove the residual solvent of the thus obtained 50th white solid by vacuum distillation.
In this manner, lithium benzoylphenoxy sulfonamide was obtained as the 50th white solid (2.04 g, 7.20 mmol, yield = 64%).
The result of measuring this 50th white solid by $^1$H-NMR (DMSO-d6) is shown below.
$^1$H-NMR: $\delta$7.05-7.45 (m,8H), 7.90 (d,J = 7.6 Hz, 2H)

[Synthesis Example 26]

Lithium ethoxycarbonylethoxy sulfonamide [Synthesis Compound (I-26)] represented by the following structural formula was synthesized in the following manner.

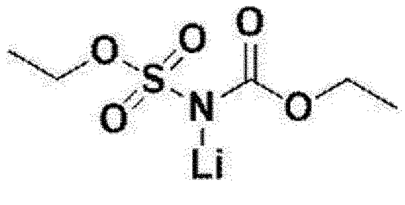

( I-26 )

<First Step: Synthesis of Ethoxycarbonylethoxy Sulfonamide>

To a nitrogen-purged 500-mL four-necked flask connected to a Dimroth condenser, chlorosulfonyl isocyanate (4.00 g, 28.3 mmol) and dichloromethane (100 mL) as a solvent were charged and, after maintaining this flask at room temperature, ethanol (3.26 g, 70.8 mmol) and triethylamine (2.86 g, 28.3 mmol) were added, and a reaction was allowed to proceed with stirring at a reflux temperature. The reaction was terminated after 4 hours, followed by cooling to room temperature. Subsequently, the resulting reaction product was washed. In other words, the reaction solution was extracted and washed using a separatory funnel with addition of 100 mL of distilled water and 100 mL of ethyl acetate.

The above-described washing with water was repeated twice, and the resulting organic layer was dried with an addition of magnesium sulfate and then concentrated under reduced pressure.

The thus obtained crude product was separated and purified by flash column chromatography using a hexane/ethyl acetate solvent.

In this manner, ethoxycarbonylethoxy sulfonamide was obtained as a 51st white solid (5.27 g, 11.3 mmol, yield = 40%).

The result of measuring this 51st white solid by $^1$H-NMR ($CDCl_3$) is shown below.

1H-NMR: δ1.32 (t,J = 7.0 Hz, 3H), 1.44 (t,J = 7.0 Hz, 3H), 4.28 (q,J = 7.0 Hz, 3H), 4.47 (q,J = 7.0 Hz, 3H), 7.53 (br,1H)

The reaction formula in the first step of Synthesis Example 26 is as follows.

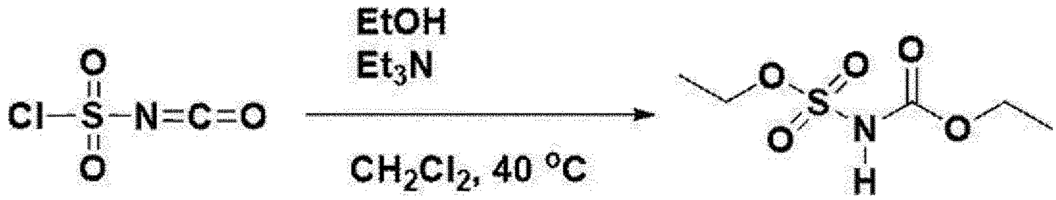


<Second Step: Synthesis of Lithium Ethoxycarbonylethoxy sulfonamide (I-26)>

Using ethoxycarbonylethoxy sulfonamide (1.02 g, 5.17 mmol), tetrahydrofuran (20 mL) as a solvent, and a lithium bis(trimethylsilyl)amide (1.3 M) tetrahydrofuran solution (3.50 mL, 4.55 mmol), a reaction was carried out in the same manner as in the second step of Synthesis Example 1 (Synthesis Compound (I-5)).

By this, lithium ethoxycarbonylethoxy sulfonamide was obtained as a 52nd white solid (258 mg, 1.29 mmol, yield = 25%).

The result of measuring this 52nd white solid by $^1$H-NMR (DMSO-d6) is shown below.

$^1$H-NMR: δ1.07 (t,J = 7.3 Hz, 3H), 1.13 (t,J = 7.3 Hz, 3H), 3.77 (q, J = 7.3 Hz, 3H), 3.85 (q, J = 7.3 Hz, 3H)

[Synthesis Example 27]

Lithium ethoxycarbonyl-2,2,2-trifluoroethoxy sulfonamide [Synthesis Compound (I-27)] represented by the following structural formula was synthesized in the following manner.

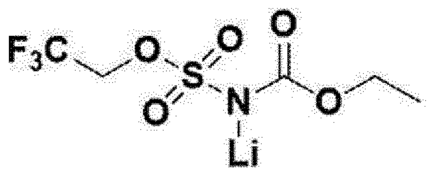
( I-27 )

<First Step: Synthesis of Ethoxycarbonyl-(2,2,2-trifluoroethoxy)sulfonamide>

2,2,2-trifluoroethyl sulfamate (12.4 g, 69.2 mmol, yield = 69%) was synthesized in accordance with Non-Patent Document (Journal of the Chemical Society. Perkin transactions I, 1982, p. 677-680).

A 53rd white solid was synthesized in the same manner as in the first step of Synthesis Example 22, except that 2,2,2-trifluoroethyl sulfamate (2.55 g, 14.2 mmol), 4-dimethylaminopyridine (0.35 g, 2.85 mmol), and tetrahydrofuran (30 mL) as a solvent were used, and benzoyl chloride and triethylamine were changed to ethyl chloroformate (3.09 g, 28.5 mmol) and pyridine (2.25 g, 28.5 mmol), respectively, in the first step of Synthesis Example 22 (Synthesis Compound (I-22)).

By this, ethoxycarbonyl-(2,2,2-trifluoroethoxy)sulfonamide was obtained as the 53rd white solid (2.28 g, 9.08 mmol, yield = 64%).

The result of measuring the 53rd white solid by $^1$H-NMR (DMSO-d6) is shown below.

$^1$H-NMR: δ1.22 (t,J = 7.0 Hz, 3H), 4.17 (q,J = 6.8 Hz, 2H), 4.93 (q,J = 8.4 Hz, 2H)

<Second Step: Synthesis of Lithium Ethoxycarbonyl-(2,2,2-trifluoroethoxy)sulfonamide (I-27)>

To a nitrogen-purged 200-mL four-necked flask, ethoxycarbonyl-(2,2,2-trifluoroethoxy)sulfonamide (2.0 g, 7.96 mmol) and tetrahydrofuran (30 mL) as a solvent were charged. This flask was maintained at -20°C, and lithium bis(trimethylsilyl)amide (1.3M) and a tetrahydrofuran solution (6.1 mL, 7.96 mmol) was added thereto over a period of 5 minutes, after which the flask was brought back to room temperature, and a reaction was allowed to proceed with stirring for 3 hours. Subsequently, *n*-hexane (30 mL) was added to allow a 54th white solid to precipitate from the resulting reaction solution. This reaction solution was vacuum-filtered to remove the residual solvent of the thus obtained 54th white solid by vacuum distillation.

In this manner, lithium ethoxycarbonyl-(2,2,2-trifluoroethoxy)sulfonamide was obtained as the 54th white solid (1.95 g, 7.58 mmol, yield = 95%).

The result of measuring this 54th white solid by $^1$H-NMR (DMSO-d6) is shown below.

$^1$H-NMR: $\delta$1.08 (t,J = 4.5 Hz, 3H), 3.81 (q,J = 6.8 Hz, 2H), 4.40 (q,J = 9.2 Hz, 2H)

[Synthesis Example 28]

Lithium-(2,2,2-trifluoroethoxy)carbonyl-(2,2,2-trifluoroethoxy)sulfonamide [Synthesis Compound (I-28)] represented by the following structural formula was synthesized in the following manner.

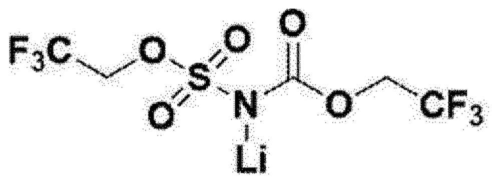

( I-28 )

<First Step: Synthesis of 2,2,2-trifluoroethoxycarbonyl-(2,2,2-trifluoroethoxy)sulfonamide>

To a nitrogen-purged 100-mL four-necked flask connected to a Dimroth condenser, 2,2,2-trifluoroethanol (5.73 g, 57.3 mmol) and chlorobenzene (13 mL) as a solvent were charged and, after maintaining this flask at room temperature, chlorosulfonyl isocyanate (4.05 g, 28.6 mmol) was added, and a reaction was allowed to proceed with stirring at a reflux temperature. The reaction was terminated after 12 hours, followed by cooling to room temperature. The resulting reaction solution was concentrated under reduced pressure, and the thus obtained crude product was separated and purified by flash column chromatography using a hexane/ethyl acetate solvent.

In this manner, 2,2,2-trifluoroethoxycarbonyl-(2,2,2-trifluoroethoxy)sulfonamide was obtained as a first colorless transparent oil (7.34 g, 24.1 mmol, yield = 84%).

The result of measuring this first colorless transparent oil by $^1$H-NMR ($CDCl_3$) is shown below.

$^1$H-NMR: $\delta$4.59 (d,J = 8.1 Hz, 2H), 4.73 (d,J = 7.6 Hz, 2H), 8.28 (br,1H)

<Second Step: Synthesis of Lithium-(2,2,2-trifluoroethoxy)carbonyl-(2,2,2-trifluoroethoxy)sulfonamide (I-28)>

Using 2,2,2-trifluoroethoxycarbonyl-(2,2,2-trifluoroethoxy)sulfonamide (1.50 g, 4.92 mmol), tetrahydrofuran (20 mL) as a solvent, and a lithium bis(trimethylsilyl)amide (1.3 M) tetrahydrofuran solution (3.75 mL, 4.88 mmol), a reaction was carried out in the same manner as in the second step of Synthesis Example 1 (Synthesis Compound (I-5)).

By this, lithium-(2,2,2-trifluoroethoxy)carbonyl-(2,2,2-trifluoroethoxy)sulfonamide was obtained as a 55th white solid (1.51 g, 4.85 mmol, yield = 99%).

The result of measuring this 55th white solid by $^1$H-NMR (DMSO-d6) is shown below.

$^1$H-NMR: $\delta$4.43 (q,J = 9.5 Hz, 2H), 4.44 (q,J = 9.0 Hz, 2H)

[Synthesis Example 29]

Lithium benzoyl-(2,2,2-trifluoroethoxy)sulfonamide [Synthesis Compound (I-29)] represented by the following structural formula was synthesized in the following manner.

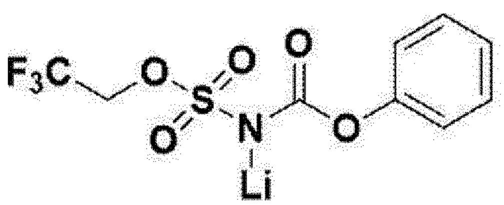

( I-29 )

<First Step: Synthesis of Phenoxycarbonyl-(2,2,2-trifluoroethoxy)sulfonamide>

2,2,2-trifluoroethyl sulfamate (12.4 g, 69.2 mmol, yield = 69%) was synthesized in accordance with Non-Patent Document (Journal of the Chemical Society. Perkin transactions I, 1982, p. 677-680).

A 56th white solid was synthesized in the same manner as in the first step of Synthesis Example 27, except that 2,2,2-trifluoroethyl sulfamate (3.19 g, 17.81 mmol), pyridine (2.82 g, 35.6 mmol), 4-dimethylaminopyridine (0.44 g, 3.56 mmol), and tetrahydrofuran (30 mL) as a solvent were used, and ethyl chloroformate was changed to phenyl chloroformate (3.35 g, 21.4 mmol) in the first step of Synthesis Example 27 (Synthesis Compound (I-27)).

By this, phenoxycarbonyl-(2,2,2-trifluoroethoxy)sulfonamide was obtained as the 56th white solid (1.97 g, 6.58 mmol, yield = 37%).

The result of measuring this 56th white solid by $^1$H-NMR (DMSO-d6) is shown below.
$^1$H-NMR: δ4.66 (q,J = 8.6 Hz, 2H), 6.75 (d,J = 8.6 Hz, 2H), 7.00-7.40 (m,3H), 8.00 (br,1H)

<Second Step: Synthesis of Lithium Phenoxycarbonyl-(2,2,2-trifluoroethoxy)sulfonamide (I-29)>

To a nitrogen-purged 200-mL four-necked flask, phenoxycarbonyl-(2,2,2-trifluoroethoxy)sulfonamide (1.97 g, 6.58 mmol), and tetrahydrofuran (30 mL) as a solvent were charged. This flask was maintained at -20°C, and a lithium bis(trimethylsilyl)amide (1.3M) tetrahydrofuran solution (5.1 mL, 6.58 mmol) was added thereto over a period of 5 minutes, after which the flask was brought back to room temperature, and a reaction was allowed to proceed with stirring for 3 hours. Subsequently, *n*-hexane (30 mL) was added to allow a 57th white solid to precipitate from the resulting reaction solution. This reaction solution was vacuum-filtered to remove the residual solvent of the thus obtained 57th white solid by vacuum distillation.

In this manner, lithium phenoxycarbonyl-(2,2,2-trifluoroethoxy)sulfonamide was obtained as the 57th white solid (1.71 g, 5.60 mmol, yield = 85%).

The result of measuring this 57th white solid by $^1$H-NMR (DMSO-d6) is shown below.

$^1$H-NMR: δ4.47 (q,J = 8.9 Hz, 2H), 6.99 (d,J = 8.4 Hz, 2H), 7.11 (t,J = 7.6 Hz, 2H), 7.31 (t,J = 5.0 Hz, 2H)

[Synthesis Example 30]

Lithium ethoxycarbonyl-p-tolyloxy sulfonamide [Synthesis Compound (I-30)] represented by the following structural formula was synthesized in the following manner.

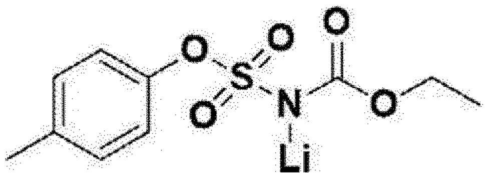

(I-30)

<First Step: Synthesis of *p*-tolyloxycarbonylchlorosulfonamide>

Referring to Non-Patent Document (Picard, J.A. et al., J. Med. Chem., 1996, 39, 1243.), *p*-tolyloxycarbonylchlorosulfonamide was synthesized.

To a nitrogen-purged 100-mL four-necked flask connected to a Dimroth condenser, *p*-cresol (7.04 g, 65.1 mmol) and chlorobenzene (20 mL) as a solvent were charged and, after maintaining this flask at room temperature, chlorosulfonyl isocyanate (9.21 g, 65.1 mmol) was added, and a reaction was allowed to proceed with stirring for 1 hour at room temperature, as a result of which a 58th white solid was precipitated from the resulting reaction solution. This reaction solution was vacuum-filtered to remove the residual solvent of the thus obtained 58th white solid by vacuum distillation.

In this manner, p-tolyloxycarbonylchlorosulfonamide was obtained as the 58th white solid (14.6 g, 58.5 mmol, yield = 90%).

The result of measuring this 58th white solid by $^1$H-NMR ($CDCl_3$) is shown below.
$^1$H-NMR: δ2.37 (s,3H), 7.09 (d,J = 8.5 Hz, 2H), 7.22 (d,J = 8.5 Hz, 2H)

<Second Step: Synthesis of Ethoxycarbonyl-*p*-tolyloxy Sulfonamide>

Referring to Non-Patent Document (Picard, J.A. et al., J. Med. Chem., 1996, 39, 1243.), ethoxycarbonyl-*p*-tolyloxy sulfonamide was synthesized.

To a nitrogen-purged 100-mL four-necked flask connected to a Dimroth condenser, tolyloxycarbonylchlorosulfonamide (5.00 g, 20.0 mmol) and chlorobenzene (30 mL) as a solvent, and these materials were allowed to react with stirring for 12 hours at a reflux temperature. Subsequently, the resultant was cooled to room temperature, and ethanol (1.85 g, 40.2 mmol) was added thereto and allowed to react with stirring at room temperature. The reaction was terminated after 2 hours, and the resulting reaction product was washed. In other words, the reaction solution was extracted and washed using a separatory funnel with addition of 30 mL of distilled water and 30 mL of ethyl acetate.

The above-described washing with water was repeated twice, and the resulting organic layer was dried with an addition of magnesium sulfate and then concentrated under reduced pressure.

The thus obtained crude product was separated and purified by flash column chromatography using a hexane/ethyl acetate solvent.

In this manner, ethoxycarbonyl-*p*-tolyloxy sulfonamide was obtained as a second colorless transparent oil (4.03 g, 15.5 mmol, yield = 78%).

The result of measuring this second colorless transparent oil by $^1$H-NMR (DMSO-d6) is shown below.

$^1$H-NMR: $\delta$1.23 (t,J = 7.0 Hz, 3H), 2.32 (s,3H), 4.20 (q,J = 7.0 Hz, 2H), 7.14 (d,J = 8.4 Hz, 2H), 7.30 (d,J = 8.4 Hz, 2H)

<Third Step: Synthesis of Lithium Ethoxycarbonyl-*p*-tolyloxy sulfonamide (I-30)>

Using ethoxycarbonyl-*p*-tolyloxy sulfonamide (2.54 g, 9.80 mmol), tetrahydrofuran (50 mL) as a solvent, and a lithium bis(trimethylsilyl)amide (1.3 M) tetrahydrofuran solution (7.30 mL, 9.49 mmol), a reaction was carried out in the same manner as in the second step of Synthesis Example 1 (Synthesis Compound (I-5)).

By this, lithium ethoxycarbonyl-*p*-tolyloxy sulfonamide was obtained as a 59th white solid (1.11 g, 4.19 mmol, yield = 43%).

The result of measuring this 59th white solid by $^1$H-NMR (DMSO-d6) is shown below.

$^1$H-NMR: $\delta$1.07 (t,J = 7.0 Hz, 3H), 2.26 (s,3H), 3.80 (q,J = 7.0 Hz, 2H), 7.02 (d,J = 8.6 Hz, 2H), 7.09 (d,J = 8.6 Hz, 2H)

As described above, according to the results of Synthesis Example 30, lithium ethoxycarbonyl-*p*-tolyloxy sulfonamide [Synthesis Compound (I-30)] was obtained by the following reaction scheme.

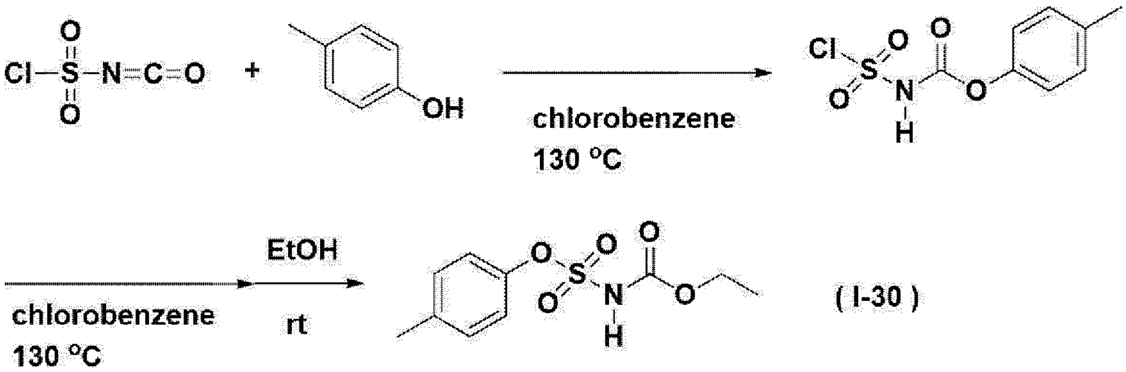


(I-30)

[Synthesis Example 31]

Lithium-*p*-tolyloxysulfonyl-(2,2,2-trifluoroethoxy)carbonylamide [Synthesis Compound (1-31)] represented by the following structural formula was synthesized in the following manner.

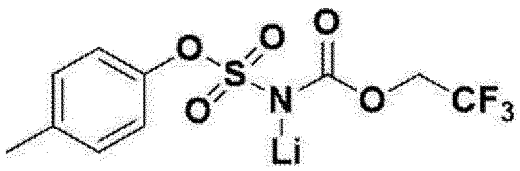

(I-31)

<First Step: Synthesis of *p*-tolyloxycarbonylchlorosulfonamide>

*P*-tolyloxycarbonylchlorosulfonamide was synthesized in the same manner as in the first step of Synthesis Example 30 (Synthesis Compound (I-30)).

<Second Step: Synthesis of *p*-tolyloxysulfonyl-(2,2,2-trifluoroethoxy)carbonylamide>

Using *p*-tolyloxycarbonylchlorosulfonamide (1.50 g, 6.01 mmol), chlorobenzene (10 mL) as a solvent, and 2,2,2-trifluoroethanol (0.60 g, 6.00 mmol), a reaction was carried out in the same manner as in the second step of Synthesis Example 30 (Synthesis Compound (I-30)).
By this, *p*-tolyloxysulfonyl-(2,2,2-trifluoroethoxy)carbonylamide was obtained as a third colorless transparent oil (1.01 g, 3.22 mmol, yield = 54%).
The result of measuring this third colorless transparent oil by $^1$H-NMR ($CDCl_3$) is shown below.
$^1$H-NMR: δ2.37 (s,3H), 4.62 (q,J = 8.1 Hz, 2H), 7.20 (m,4H)

<Third Step: Synthesis of Lithium-*p*-tolyloxysulfonyl-(2,2,2-trifluoroethoxy)carbonylamide (1-31)>

Using *p*-tolyloxysulfonyl-(2,2,2-trifluoroethoxy)carbonylamide (1.01 g, 3.22 mmol), tetrahydrofuran (15 mL) as a solvent, and a lithium bis(trimethylsilyl)amide (1.3 M) tetrahydrofuran solution (2.30 mL, 2.99 mmol), a reaction was carried out in the same manner as in the second step of Synthesis Example 1 (Synthesis Compound (I-5)).
By this, lithium-*p*-tolyloxysulfonyl-(2,2,2-trifluoroethoxy)carbonylamide was obtained as a 60th white solid (350 mg, 1.10 mmol, yield = 34%).
The result of measuring this 60th white solid by $^1$H-NMR (DMSO-d6) is shown below.
$^1$H-NMR: δ2.26 (s,3H), 4.43 (q,J = 9.2 Hz, 2H), 7.03 (d,J = 8.6 Hz, 2H), 7.10 (d,J = 8.6 Hz, 2H)

[Synthesis Example 32]

Lithium *p*-tolyloxycarbonyl-*p*-tolyloxy sulfonamide [Synthesis Compound (I-32)] represented by the following structural formula was synthesized in the following manner.

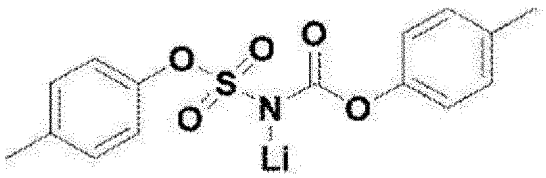

(I-32)

<First Step: Synthesis of *p*-tolyloxycarbonyl-*p*-tolyloxy Sulfonamide>

A fourth colorless transparent oil was synthesized in the same manner as in the first step of Synthesis Example 28, except that trifluoroethanol was changed to *p*-cresol (6.20 g, 57.3 mmol) in the first step of Synthesis Example 28 (Synthesis Compound (I-28)).
By this, *p*-tolyloxycarbonyl-*p*-tolyloxy sulfonamide was obtained as the fourth colorless transparent oil (9.10 g, 28.3 mmol, yield = 99%).
The result of measuring this fourth colorless transparent oil by $^1$H-NMR ($CDCl_3$) is shown below.
$^1$H-NMR: δ2.37 (s,3H), 2.38 (s,3H), 7.05 (d,J = 6.8 Hz, 2H), 7.21 (m,6H)

<Second Step: Synthesis of Lithium-*p*-tolyloxycarbonyl-*p*-tolyloxy sulfonamide (I-32)>

Using *p*-tolyloxycarbonyl-*p*-tolyloxy sulfonamide (1.20 g, 3.73 mmol), tetrahydrofuran (20 mL) as a solvent, a lithium bis(trimethylsilyl)amide (1.3 M), and a tetrahydrofuran solution (2.60 mL, 3.38 mmol), a reaction was carried out in the same manner as in the second step of Synthesis Example 1 (Synthesis Compound (I-5)).
By this, lithium *p*-tolyloxycarbonyl *p*-tolyloxy sulfonamide was obtained as a 61st white solid (798 mg, 2.42 mmol, yield = 65%).
The result of measuring this 61st white solid by $^1$H-NMR (DMSO-d6) is shown below.
$^1$H-NMR: δ2.27 (s,3H), 2.28 (s,3H), 6.82 (d,J = 8.6 Hz, 2H), 7.10 (m,6H)

[Synthesis Example 33]

Lithium-4-methylphenoxycarbonyl trifluoromethyl sulfonamide [Synthesis Compound (I-33)] represented by the following structural formula was synthesized in the following manner.

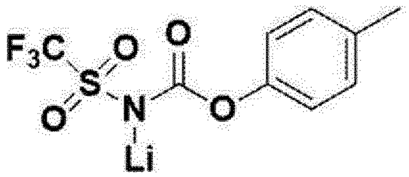

(I-33)

<First Step: Synthesis of 4-methylphenoxycarbonyl Trifluoromethyl Sulfonamide>

4-methylphenoxycarbonyl trifluoromethyl sulfonamide was synthesized in the same manner as in the first step of Synthesis Example 2, except that trifluoromethanesulfonamide (2.0 g, 13.4 mmol), pyridine (2.12 g, 26.8 mmol), 4-dimethylaminopyridine (0.33 g, 2.7 mmol), and tetrahydrofuran (30 mL) as a solvent were used, and methyl chloroformate was changed to 4-methylphenyl chloroformate (2.52 g, 14.8 mmol) in the first step of Synthesis Example 2 (Synthesis Compound (I-10)).

By this, 4-methylphenoxycarbonyl trifluoromethyl sulfonamide was obtained as a fifth colorless transparent oil (1.02 g, 3.6 mmol, yield = 27%).

The result of measuring this fifth colorless transparent oil by $^1$H-NMR (DMSO-d6) is shown below.

$^1$H-NMR: $\delta$6.61-6.66 (m,2H), 6.92-6.97 (m,2H), 9.06 (br,1H)

<Second Step: Synthesis of Lithium-4-methylphenoxycarbonyl Trifluoromethyl Sulfonamide (I-33)>

Using 4-methylphenoxycarbonyl trifluoromethyl sulfonamide (1.02 g, 3.60 mmol), diethyl ether (20 mL) as a solvent, and a lithium bis(trimethylsilyl)amide (1.3M) tetrahydrofuran solution (2.8 mL, 3.60 mmol), a reaction was carried out in the same manner as in the second step of Synthesis Example 2 (Synthesis Compound (I-10)).

By this, lithium-4-methylphenoxycarbonyl trifluoromethyl sulfonamide was obtained as a 62nd white solid (0.76 g, 2.63 mmol, yield = 73%).

The result of measuring this 62nd white solid by $^1$H-NMR (DMSO-d6) is shown below.

$^1$H-NMR: $\delta$6.18-6.34 (m,2H), 6.58-6.66 (m,2H)

[Synthesis Example 34]

Lithium-4-methoxyphenoxycarbonyl trifluoromethyl sulfonamide [Synthesis Compound (I-34)] represented by the following structural formula was synthesized in the following manner.

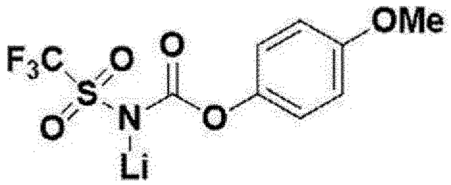

(I-34)

<First Step: Synthesis of 4-methoxyphenoxycarbonyl Trifluoromethyl Sulfonamide>

4-methoxyphenoxycarbonyl trifluoromethyl sulfonamide was synthesized in the same manner as in the first step of Synthesis Example 2, except that trifluoromethanesulfonamide (1.60 g, 10.7 mmol), pyridine (1.70 g, 21.5 mmol), 4-dimethylaminopyridine (0.26 g, 2.1 mmol), and tetrahydrofuran (30 mL) as a solvent were used, and methyl chloroformate was changed to 4-methoxyphenyl chloroformate (2.40 g, 12.9 mmol) in the first step of Synthesis Example 2 (Synthesis Compound (1-10)).

By this, 4-methoxyphenoxycarbonyl trifluoromethyl sulfonamide was obtained as a 63rd white solid (1.55 g, 5.2 mmol, yield = 48%).

The result of measuring this 63rd white solid by $^1$H-NMR (DMSO-d6) is shown below.

$^1$H-NMR: $\delta$3.65 (s,3H), 6.55-6.78 (m,4H), 8.86 (br,1H)

<Second Step: Synthesis of Lithium-4-methoxyphenoxycarbonyl Trifluoromethyl Sulfonamide (I-34)>

Using 4-methoxyphenoxycarbonyl trifluoromethyl sulfonamide (0.95 g, 3.16 mmol), diethyl ether (20 mL) as a solvent, and a lithium bis(trimethylsilyl)amide (1.3M) tetrahydrofuran solution (2.4 mL, 3.16 mmol), a reaction was carried out in the same manner as in the second step of Synthesis Example 2 (Synthesis Compound (I-10)).

By this, lithium-4-methoxyphenoxycarbonyl trifluoromethyl sulfonamide was obtained as a 64th white solid (0.79 g, 2.58 mmol, yield = 82%).

The result of measuring this 64th white solid by $^1$H-NMR (DMSO-d6) is shown below.
$^1$H-NMR: δ3.55 (s,3H), 6.13-6.28 (m,2H), 6.43-6.52 (m,2H)

[Synthesis Example 35]

Lithium-4-chlorophenoxycarbonyl trifluoromethyl sulfonamide [Synthesis Compound (I-35)] represented by the following structural formula was synthesized in the following manner.

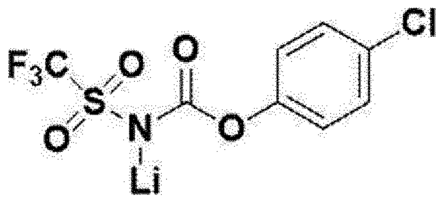

( I-35 )

<First Step: Synthesis of 4-chlorophenoxycarbonyl Trifluoromethyl Sulfonamide>

4-chlorophenoxycarbonyl trifluoromethyl sulfonamide was synthesized in the same manner as in the first step of Synthesis Example 2, except that trifluoromethanesulfonamide (2.0 g, 13.4 mmol), pyridine (2.12 g, 26.8 mmol), 4-dimethylaminopyridine (0.33 g, 2.7 mmol), and tetrahydrofuran (30 mL) as a solvent were used, and methyl chloroformate was changed to 4-chlorophenyl chloroformate (3.07 g, 16.1 mmol) in the first step of Synthesis Example 2 (Synthesis Compound (I-10)).

By this, 4-chlorophenoxycarbonyl trifluoromethyl sulfonamide was obtained as a sixth colorless transparent oil (1.54 g, 5.1 mmol, yield = 38%).

The result of measuring this sixth colorless transparent oil by $^1$H-NMR (DMSO-d6) is shown below.
$^1$H-NMR: δ6.74-6.79 (m,2H), 7.17-7.22 (m,2H)

<Second Step: Synthesis of Lithium-4-chlorophenoxycarbonyl Trifluoromethyl Sulfonamide (I-35)>

Using 4-chlorophenoxycarbonyl trifluoromethyl sulfonamide (1.54 g, 5.07 mmol), diethyl ether (20 mL) as a solvent, and a lithium bis(trimethylsilyl)amide (1.3M) tetrahydrofuran solution (3.9 mL, 5.07 mmol), a reaction was carried out in the same manner as in the second step of Synthesis Example 2 (Synthesis Compound (I-10)).

By this, lithium-4-chlorophenoxycarbonyl trifluoromethyl sulfonamide was obtained as a 65th yellow solid (0.65 g, 2.10 mmol, yield = 41%).

The result of measuring this 65th yellow solid by $^1$H-NMR (DMSO-d6) is shown below.
$^1$H-NMR: δ6.40-6.46 (m,2H), 6.84-6.90 (m,2H)

[Synthesis Example 36]

Lithium-4-fluorophenoxycarbonyl trifluoromethyl sulfonamide [Synthesis Compound (I-36)] represented by the following structural formula was synthesized in the following manner.

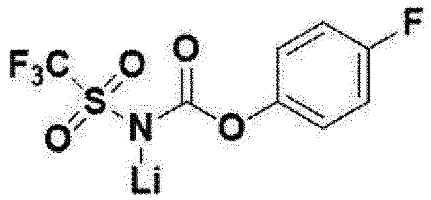

( I-36)

<First Step: Synthesis of 4-fluorophenoxycarbonyl Trifluoromethyl Sulfonamide>

4-fluorophenoxycarbonyl trifluoromethyl sulfonamide was synthesized in the same manner as in the first step of Synthesis Example 2, except that trifluoromethanesulfonamide (1.94 g, 13.0 mmol), pyridine (2.06 g, 26.0 mmol), 4-dimethylaminopyridine (0.32 g, 2.6 mmol), and tetrahydrofuran (30 mL) as a solvent were used, and methyl chloroformate

was changed to 4-fluorophenyl chloroformate (2.50 g, 14.3 mmol) in the first step of Synthesis Example 2 (Synthesis Compound (I-10)).

By this, 4-fluorophenoxycarbonyl trifluoromethyl sulfonamide was obtained as a seventh colorless transparent oil (2.02 g, 7.0 mmol, yield = 54%).

The result of measuring this seventh colorless transparent oil by $^1$H-NMR (DMSO-d6) is shown below.
$^1$H-NMR: δ6.71-6.76 (m,2H), 6.94-7.01 (m,2H), 9.34 (br,1H)

<Second Step: Synthesis of Lithium-4-fluorophenoxycarbonyl Trifluoromethyl Sulfonamide (I-36)>

Using 4-fluorophenoxycarbonyl trifluoromethyl sulfonamide (2.02 g, 7.03 mmol), diethyl ether (20 mL) as a solvent, and a lithium bis(trimethylsilyl)amide (1.3M) tetrahydrofuran solution (5.4 mL, 7.03 mmol), a reaction was carried out in the same manner as in the second step of Synthesis Example 2 (Synthesis Compound (I-10)).

By this, lithium-4-fluorophenoxycarbonyl trifluoromethyl sulfonamide was obtained as a 66th white solid (0.91 g, 3.10 mmol, yield = 44%).

The result of measuring this 66th white solid by $^1$H-NMR (DMSO-d6) is shown below.
$^1$H-NMR: δ6.21-6.31 (m,2H), 6.58-6.68 (m,2H)

[Synthesis Example 37]

Lithium allyloxycarbonyl trifluoromethyl sulfonamide [Synthesis Compound (I-37)] represented by the following structural formula was synthesized in the following manner.

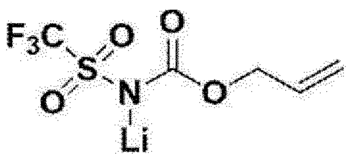

(I-37)

<First Step: Synthesis of Allyloxycarbonyl Trifluoromethyl Sulfonamide>

Allyloxycarbonyl trifluoromethyl sulfonamide was synthesized in the same manner as in the first step of Synthesis Example 2, except that trifluoromethanesulfonamide (2.50 g, 16.8 mmol), pyridine (2.65 g, 33.5 mmol), 4-dimethylaminopyridine (0.41 g, 3.4 mmol), and tetrahydrofuran (30 mL) as a solvent were used, and methyl chloroformate was changed to ally chloroformate (2.43 g, 20.1 mmol) in the first step of Synthesis Example 2 (Synthesis Compound (I-10)).

By this, allyloxycarbonyl trifluoromethyl sulfonamide was obtained as a 67th white solid (2.46 g, 10.6 mmol, yield = 63%).

The result of measuring this 67th white solid by $^1$H-NMR (DMSO-d6) is shown below.
$^1$H-NMR: δ4.25-4.50 (m,2H), 4.95-5.32 (m,2H), 5.70-5.98 (m,1H), 9.66 (br,1H)

<Second Step: Synthesis of Lithium Allyloxycarbonyl Trifluoromethyl Sulfonamide (I-37)>

Using allyloxycarbonyl trifluoromethyl sulfonamide (2.20 g, 9.44 mmol), diethyl ether (20 mL) as a solvent, and a lithium bis(trimethylsilyl)amide (1.3M) tetrahydrofuran solution (7.3 mL, 9.44 mmol), a reaction was carried out in the same manner as in the second step of Synthesis Example 2 (Synthesis Compound (I-10)).

By this, lithium allyloxycarbonyl trifluoromethyl sulfonamide was obtained as a 68th white solid (1.58 g, 6.63 mmol, yield = 70%).

The result of measuring this 68th white solid by $^1$H-NMR (DMSO-d6) is shown below.
$^1$H-NMR: δ4.25-4.40 (m,2H), 5.00-5.30 (m,2H), 5.85-5.96 (m,1H)

[Synthesis Example 38]

Lithium butoxycarbonyl-4-fluorophenyl sulfonamide [Synthesis Compound (I-38)] represented by the following structural formula was synthesized in the following manner.

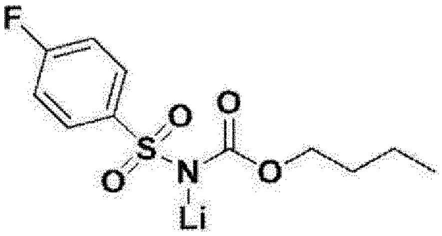

( I-38 )

<First Step: Synthesis of Butoxycarbonyl-4-fluorophenyl Sulfonamide>

Butoxycarbonyl-4-fluorophenyl sulfonamide was synthesized in the same manner as in the first step of Synthesis Example 14, except that butyl chloroformate (2.34 g, 17.1 mmol), 4-dimethylaminopyridine (0.41 g, 3.4 mmol), and tetrahydrofuran (30 mL) as a solvent were used, and trifluoromethanesulfonamide and pyridine were changed to 4-fluorobenzenesulfonamide (2.50 g, 14.3 mmol) and triethylamine (2.89 g, 28.5 mmol), respectively, in the first step of Synthesis Example 14 (Synthesis Compound (I-14)).

By this, butoxycarbonyl-4-fluorophenyl sulfonamide was obtained as a 69th white solid (1.13 g, 4.1 mmol, yield = 29%).

The result of measuring this 69th white solid by $^1$H-NMR (DMSO-d6) is shown below.

$^1$H-NMR: $\delta$0.82 (t,J = 7.0 Hz, 3H), 1.05-1.30 (m,2H), 1.35-1.55 (m,2H), 3.98 (t,J = 6.6 Hz, 2H), 7.38-7.58 (m,2H), 7.84-8.04 (m,2H), 12.06 (br,1H)

<Second Step: Synthesis of Lithium Butoxycarbonyl-4-fluorophenyl Sulfonamide (I-38)>

Using butoxycarbonyl-4-fluorophenyl sulfonamide (1.01 g, 3.67 mmol), diethyl ether (20 mL) as a solvent, and a lithium bis(trimethylsilyl)amide (1.3M) tetrahydrofuran solution (2.8 mL, 3.67 mmol), a reaction was carried out in the same manner as in the second step of Synthesis Example 2 (Synthesis Compound (I-10)).

By this, lithium butoxycarbonyl-4-fluorophenyl sulfonamide was obtained as a 70th white solid (0.77 g, 2.72 mmol, yield = 74%).

The result of measuring this 70th white solid by $^1$H-NMR (DMSO-d6) is shown below.

$^1$H-NMR: $\delta$0.84 (t,J = 7.2 Hz, 3H), 1.15-1.30 (m,2H), 1.30-1.45 (m,2H), 3.65 (t,J = 6.6 Hz, 2H), 7.10-7.25 (m,2H), 7.65-7.80 (m,2H)

[Synthesis Example 39]

Lithium methoxycarbonyl-4-trifluoromethylphenyl sulfonamide [Synthesis Compound (I-39)] represented by the following structural formula was synthesized in the following manner.

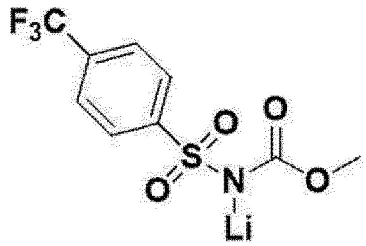

( I-39 )

<First Step: Synthesis of Methoxycarbonyl-4-(trifluoromethyl)phenyl Sulfonamide>

Methoxycarbonyl-4-(trifluoromethyl)phenyl sulfonamide was synthesized in the same manner as in the first step of Synthesis Example 2, except that methyl chloroformate (2.64 g, 28.0 mmol), 4-dimethylaminopyridine (0.61 g, 5.0 mmol), and tetrahydrofuran (100 mL) as a solvent were used, and trifluoromethanesulfonamide and pyridine were changed to 4-(trifluoromethyl)benzenesulfonamide (5.24 g, 23.3 mmol) and triethylamine (2.82 g, 28.0 mmol), respectively, in the first step of Synthesis Example 2 (Synthesis Compound (I-10)).

By this, methoxycarbonyl-4-(trifluoromethyl)phenyl sulfonamide was obtained as a 71st white solid (5.30 g, 18.7 mmol, yield = 80%).

The result of measuring this 71st white solid by $^1$H-NMR (DMSO-d6) is shown below.

$^1$H-NMR: $\delta$3.65 (s,3H), 8.04 (d,J = 9.1 Hz, 2H), 8.18 (d,J = 9.1 Hz, 2H)

<Second Step: Synthesis of Lithium Methoxycarbonyl-4-(trifluoromethyl)phenyl Sulfonamide (I-39)>

Using methoxycarbonyl-4-(trifluoromethyl)phenyl sulfonamide (1.80 g, 6.36 mmol), tetrahydrofuran (30 mL) as a solvent, and a lithium bis(trimethylsilyl)amide (1.3M) tetrahydrofuran solution (4.64 mL, 6.03 mmol), a reaction was carried out in the same manner as in the second step of Synthesis Example 2 (Synthesis Compound (I-10)).
By this, lithium methoxycarbonyl-4-(trifluoromethyl)phenyl sulfonamide was obtained as a 72nd white solid (0.44 g, 1.52 mmol, yield = 24%).
The result of measuring this 72nd white solid by $^1$H-NMR (DMSO-d6) is shown below.
$^1$H-NMR: δ2.31 (s,3H), 7.65 (d,J = 8.9 Hz, 2H), 7.70 (d,J = 8.9 Hz, 2H)

[Synthesis Example 40]

Lithium methoxycarbonyl-4-(trifluoromethoxy)phenyl sulfonamide [Synthesis Compound (I-40)] represented by the following structural formula was synthesized in the following manner.

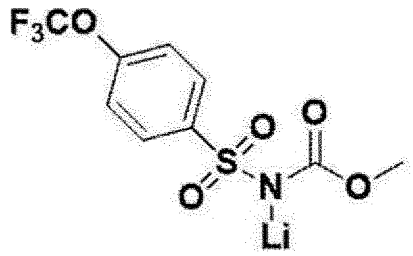

( I-40 )

<First Step: Synthesis of Methoxycarbonyl-4-(trifluoromethoxy)phenyl Sulfonamide>

Methoxycarbonyl-4-(trifluoromethoxy)phenyl sulfonamide was synthesized in the same manner as in the first step of Synthesis Example 2, except that methyl chloroformate (1.18 g, 12.5 mmol), 4-dimethylaminopyridine (0.13 g, 1.0 mmol), and tetrahydrofuran (50 mL) as a solvent were used, and trifluoromethanesulfonamide and pyridine were changed to 4-(trifluoromethoxy)benzenesulfonamide (2.50 g, 10.4 mmol) and triethylamine (1.26 g, 12.5 mmol), respectively, in the first step of Synthesis Example 2 (Synthesis Compound (I-10)).
By this, methoxycarbonyl-4-(trifluoromethoxy)phenyl sulfonamide was obtained as a 73rd white solid (3.10 g, 10.4 mmol, yield = 100%).
The result of measuring this 73rd white solid by $^1$H-NMR (DMSO-d6) is shown below.
$^1$H-NMR: δ3.65 (s,3H), 7.63 (d,J = 9.3 Hz, 2H), 8.10 (d,J = 9.3 Hz, 2H), 8.32 (br,1H)

<Second Step: Synthesis of Lithium Methoxycarbonyl-4-(trifluoromethoxy)phenyl Sulfonamide (I-40)>

Using methoxycarbonyl-4-(trifluoromethoxy)phenyl sulfonamide (3.10 g, 10.4 mmol), tetrahydrofuran (40 mL) as a solvent, and a lithium bis(trimethylsilyl)amide (1.3M) tetrahydrofuran solution (7.57 mL, 9.84 mmol), a reaction was carried out in the same manner as in the second step of Synthesis Example 2 (Synthesis Compound (I-10)).
By this, lithium methylcarbonyl-4-(trifluoromethoxy)phenylamide was obtained as a 74th white solid (0.40 g, 1.31 mmol, yield = 13%).
The result of measuring this 74th white solid by $^1$H-NMR (DMSO-d6) is shown below.
$^1$H-NMR: δ2.36 (s,3H), 7.42 (d,J = 9.0 Hz, 2H), 7.74 (d,J = 9.0 Hz, 2H)

[Synthesis Example 41]

Lithium phenoxycarbonylethoxy sulfonamide [Synthesis Compound (I-41)] represented by the following structural formula was synthesized in the following manner.

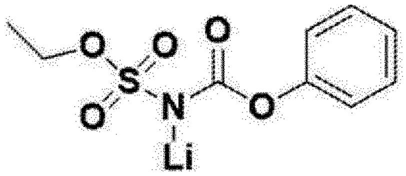

( I-41 )

<First Step: Synthesis of Phenoxycarbonylethoxy Sulfonamide>

Phenoxycarbonylethoxy sulfonamide was synthesized in the same manner as in the first step of Synthesis Example 20, except that ethyl sulfamate(1.80 g, 14.4 mmol), 4-dimethylaminopyridine (0.39 g, 3.2 mmol), and tetrahydrofuran (30 mL) as a solvent were used, and trifluoroacetic anhydride and pyridine were changed to phenyl chloroformate (2.70 g, 17.3 mmol) and triethylamine (2.91 g, 28.8 mmol), respectively, in the first step of Synthesis Example 20 (Synthesis Compound (I-20)).

By this, phenoxycarbonylethoxy sulfonamide was obtained as a 75th white solid (2.19 g, 8.93 mmol, yield = 62%).

The result of measuring this 75th white solid by $^1$H-NMR (DMSO-d6) is shown below.

$^1$H-NMR: δ1.43 (t,J = 7.2 Hz, 3H), 4.52 (q,J = 7.2 Hz, 2H), 7.12-7.46 (m,5H)

<Second Step: Synthesis of Lithium Phenoxycarbonylethoxy Sulfonamide (I-41)>

To a nitrogen-purged 200-mL four-necked flask, phenoxycarbonylethoxy sulfonamide (2.19 g, 8.9 mmol), and tetrahydrofuran (20 mL) as a solvent were charged. This flask was maintained at -20°C, and a lithium bis(trimethylsilyl)amide (1.3M) tetrahydrofuran solution (6.9 mL, 8.9 mmol) was added thereto over a period of 5 minutes, after which the flask was brought back to room temperature, and a reaction was allowed to proceed with stirring for 3 hours. Subsequently, n-hexane (20 mL) was added to allow a 76th white solid to precipitate from the resulting reaction solution. This reaction solution was vacuum-filtered to remove the residual solvent of the thus obtained 76th white solid by vacuum distillation.

In this manner, lithium phenoxycarbonylethoxy sulfonamide was obtained as a 76th white solid (2.11 g, 8.4 mmol, yield = 94%).

The result of measuring this 76th white solid by $^1$H-NMR (DMSO-d6) is shown below.

$^1$H-NMR: δ1.16 (t,J = 7.0 Hz, 3H), 3.93 (q,J = 7.3 Hz, 2H), 6.92-7.12 (m,3H), 7.24-7.34 (m,2H)

[Synthesis Example 42]

Lithium fluorosulfonylmethoxycarbonylamide [Synthesis Compound (I-42)] represented by the following structural formula was synthesized in the following manner.

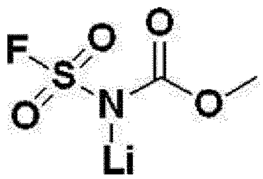

(I-42)

<First Step: Synthesis of Fluorosulfonylmethoxycarbonylamide>

To a nitrogen-purged 100-mL four-necked flask connected to a Dimroth condenser, chlorosulfonyl isocyanate (9.72 g, 68.7 mmol) and acetonitrile (25 mL) as a solvent were charged, and the flask was cooled to 0°C. Methanol (2.00 g, 62.4 mmol) was added thereto and allowed to react with stirring for 1 hour at room temperature, after which potassium hydrogen difluoride (5.85 g, 74.9 mmol) was added, and the resultant was allowed to further react with stirring. The reaction was terminated after 2 hours.

Subsequently, the resulting reaction product was washed. In other words, the reaction solution was extracted and washed using a separatory funnel with addition of 50 mL of distilled water and 50 mL of hexane/ethyl acetate.

The above-described washing with water was repeated twice, and all of the resulting organic phases were combined and washed with saturated saline (20 mL). The thus obtained organic phase was dried with an addition of magnesium sulfate and then concentrated under reduced pressure. By this, a crude product, fluorosulfonylmethoxycarbonylamide (9.63 g), was obtained.

<Second Step: Synthesis of Lithium Fluorosulfonylmethoxycarbonylamide>

To a nitrogen-purged 100-mL four-necked flask, the thus obtained crude product fluorosulfonylmethoxycarbonylamide (4.50 g) and methanol (25 mL) as a solvent were charged and, after adding thereto lithium carbonate (2.33 g, 31.5 mmol), the resultant was allowed to react with stirring for 1 hour. The resulting reaction solution was concentrated under reduced pressure, and ethyl acetate (20 mL) was added thereto to prepare a suspension. This suspension was filtered through Celite, and the resulting filtrate was concentrated under reduced pressure. Dichloromethane (20 mL)

was added, and the resultant was stirred for 1 hour at room temperature, as a result of which a 77th white solid was precipitated. This solution was vacuum-filtered to remove the residual solvent of the thus obtained 77th white solid by vacuum distillation. In this manner, lithium fluorosulfonylmethoxycarbonylamide was obtained as the 77th white solid (3.30 g, 20.2 mmol, second step yield = 70%).

The result of measuring this 77th white solid by $^1$H-NMR (DMSO-d6) is shown below.
$^1$H-NMR: δ3.41 (s,3H)

As described above, according to the results of Synthesis Example 42, lithium fluorosulfonylmethoxycarbonylamide [Synthesis Compound (I-42)] was obtained by the following reaction scheme.

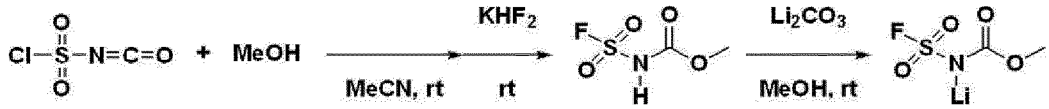


[Synthesis Example 43]

Lithium fluorosulfonylethoxycarbonylamide [Synthesis Compound (I-43)] represented by the following structural formula was synthesized in the following manner.

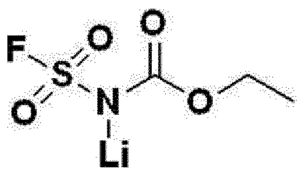

( I-43 )

<First Step: Synthesis of Fluorosulfonylethoxycarbonylamide>

Fluorosulfonylethoxycarbonylamide was synthesized in the same manner as in Synthesis Example 42, except that chlorosulfonyl isocyanate (8.45 g, 59.7 mmol), potassium hydrogen difluoride (5.09 g, 65.2 mmol), and acetonitrile (25 mL) as a solvent were used, and methanol was changed to ethanol (2.50 g, 54.3 mmol) in the first step of Synthesis Example 42 (Synthesis Compound (I-42)). By this, a crude product, fluorosulfonylethoxycarbonylamide (9.29 g), was obtained.

<Second Step: Synthesis of Lithium Fluorosulfonylethoxycarbonylamide>

Using the thus obtained crude product fluorosulfonylethoxycarbonylamide (4.50 g), lithium carbonate (2.14 g, 29.0 mmol), and methanol (25 mL) as a solvent a reaction was carried out in the same manner as in the second step of Synthesis Example 42 (Synthesis Compound (I-42)).

By this, lithium fluorosulfonylethoxycarbonylamide was obtained as a 78th white solid (2.29 g, 12.9 mmol, second step yield = 49%).

The result of measuring this 78th white solid by $^1$H-NMR (DMSO-d6) is shown below.
$^1$H-NMR: δ1.10 (t,J = 6.8 Hz, 3H), 3.84 (q,J = 6.8 Hz, 2H)

[Synthesis Example 44]

Lithium fluorosulfonylpropoxycarbonylamide [Synthesis Compound (I-44)] represented by the following structural formula was synthesized in the following manner.

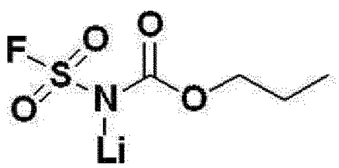

( I-44 )

<First Step: Synthesis of Fluorosulfonylpropoxycarbonylamide>

Fluorosulfonylpropoxycarbonylamide was synthesized in the same manner as in Synthesis Example 42, except that chlorosulfonyl isocyanate (7.77 g, 54.9 mmol), potassium hydrogen difluoride (4.68 g, 59.9 mmol), and acetonitrile (25 mL) as a solvent were used, and methanol was changed to n-propanol (3.00 g, 49.9 mmol) in the first step of Synthesis Example 42 (Synthesis Compound (I-42)). By this, a crude product, fluorosulfonylpropoxycarbonylamide (9.24 g), was obtained.

<Second Step: Synthesis of Lithium Fluorosulfonylpropoxycarbonylamide>

Using the crude product fluorosulfonylpropoxycarbonylamide obtained in the first step (4.00 g), lithium carbonate (1.76 g, 23.8 mmol), and methanol (20 mL) as a solvent a reaction was carried out in the same manner as in the second step of Synthesis Example 42 (Synthesis Compound (I-42)).

By this, lithium fluorosulfonylpropoxycarbonylamide was obtained as a 79th white solid (1.76 g, 9.21 mmol, second step yield = 43%).

The result of measuring this 79th white solid by $^1$H-NMR (DMSO-d6) is shown below.

$^1$H-NMR: δ0.88 (t,J = 7.6 Hz, 3H), 1.46-1.55 (m,2H), 3.76 (t,J = 6.8 Hz, 2H)

[Synthesis Example 45]

Lithium fluorosulfonylbutoxycarbonylamide [Synthesis Compound (I-45)] represented by the following structural formula was synthesized in the following manner.

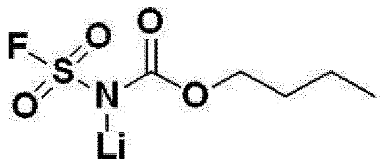

( I-45 )

<First Step: Synthesis of Fluorosulfonylbutoxycarbonylamide>

Fluorosulfonylbutoxycarbonylamide was synthesized in the same manner as in Synthesis Example 42, except that chlorosulfonyl isocyanate (5.11 g, 36.1 mmol), potassium hydrogen difluoride (3.08 g, 39.4 mmol), and acetonitrile (15 mL) as a solvent were used, and methanol was changed to n-butanol (2.43 g, 32.8 mmol) in the first step of Synthesis Example 42 (Synthesis Compound (I-42)). By this, a crude product, fluorosulfonylbutoxycarbonylamide (6.53 g), was obtained.

<Second Step: Synthesis of Lithium Fluorosulfonylbutoxycarbonylamide>

Using the thus obtained crude product fluorosulfonylbutoxycarbonylamide (3.39 g), lithium carbonate (1.38 g, 18.7 mmol), and methanol (15 mL) as a solvent, a reaction was carried out in the same manner as in the second step of Synthesis Example 42 (Synthesis Compound (I-42)).

By this, lithium fluorosulfonylbutoxycarbonylamide was obtained as an 80th white solid (2.50 g, 12.2 mmol, second step yield = 72%).

The result of measuring this 80th white solid by $^1$H-NMR (DMSO-d6) is shown below.

$^1$H-NMR: δ0.88 (t,J = 7.6 Hz, 3H), 1.26-1.35 (m,2H), 1.43-1.50 (m,2H), 3.80 (t,J = 6.4 Hz, 3H)

[Synthesis Example 46]

Lithium fluorosulfonylbenzyloxycarbonylamide [Synthesis Compound (I-46)] represented by the following structural formula was synthesized in the following manner.

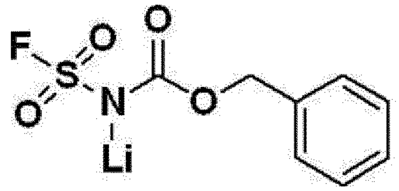

( I-46 )

<First Step: Synthesis of Fluorosulfonylbenzyloxycarbonylamide>

Fluorosulfonylbenzyloxycarbonylamide was synthesized in the same manner as in Synthesis Example 42, except that chlorosulfonyl isocyanate (3.97 g, 28.1 mmol), potassium hydrogen difluoride (2.34 g, 30.0 mmol), and acetonitrile (15 mL) as a solvent were used, and methanol was changed to benzyl alcohol (2.70 g, 25.0 mmol) in the first step of Synthesis Example 42 (Synthesis Compound (I-42)). By this, a crude product, fluorosulfonylbenzyloxycarbonylamide (5.82 g), was obtained.

<Second Step: Synthesis of Lithium Fluorosulfonylbenzyloxycarbonylamide>

Using the thus obtained crude product fluorosulfonylbenzyloxycarbonylamide (3.52 g), lithium carbonate (2.03 g, 27.5 mmol), and methanol (15 mL) as a solvent, a reaction was carried out in the same manner as in the second step of Synthesis Example 42 (Synthesis Compound (I-42)).

By this, lithium fluorosulfonylbenzyloxycarbonylamide was obtained as an 81st white solid (2.67 g, 11.2 mmol, second step yield = 74%).

The result of measuring this 81st white solid by $^1$H-NMR (DMSO-d6) is shown below.

$^1$H-NMR: $\delta$4.91 (s,2H), 7.25-7.37 (m,5H)

[Synthesis Example 47]

Lithium butoxycarbonyl-4-trifluoromethylphenyl sulfonamide [Synthesis Compound (I-47)] represented by the following structural formula was synthesized in the following manner.

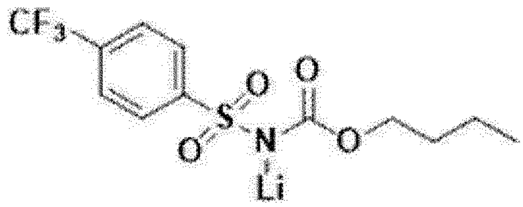

( I-47 )

<First Step: Synthesis of Butoxycarbonyl-4-(trifluoromethyl)phenyl Sulfonamide>

Butoxycarbonyl-4-(trifluoromethyl)phenyl sulfonamide was synthesized in the same manner as in the first step of Synthesis Example 39, except that 4-(trifluoromethyl)benzenesulfonamide (2.40 g, 10.7 mmol), triethylamine (1.30 g, 12.79 mmol), 4-dimethylaminopyridine (0.16 g, 1.28 mmol), and tetrahydrofuran (50 mL) as a solvent were used, and methyl chloroformate was changed to butyl chloroformate (1.46 g, 10.7 mmol) in the first step of Synthesis Example 39 (Synthesis Compound (I-39)).

By this, butoxycarbonyl-4-(trifluoromethyl)phenyl sulfonamide was obtained as an 82nd white solid (1.80 g, 5.53 mmol, yield = 52%).

The result of measuring this 82nd white solid by $^1$H-NMR (DMSO-d6) is shown below.

$^1$H-NMR: $\delta$0.80 (t,J = 7.4 Hz, 3H), 1.15-1.21 (m,2H), 1.40-1.50 (m,2H), 4.00 (t,J = 6.6 Hz, 3H), 8.02-8.15 (m,4H)

<Second Step: Synthesis of Lithium Butoxycarbonyl-4-(trifluoromethyl)phenyl Sulfonamide (I-47)>

Using butoxycarbonyl-4-(trifluoromethyl)phenyl sulfonamide (1.64 g, 5.03 mmol), tetrahydrofuran (20 mL) as a solvent, and a lithium bis(trimethylsilyl)amide (1.3M) tetrahydrofuran solution (3.87 mL, 5.03 mmol), a reaction was carried out in the same manner as in the second step of Synthesis Example 39 (Synthesis Compound (I-39)).

By this, lithium butoxycarbonyl-4-(trifluoromethyl)phenyl sulfonamide was obtained as an 83rd white solid (1.22 g, 3.68 mmol, yield = 73%).

The result of measuring this 83rd white solid by $^1$H-NMR (DMSO-d6) is shown below.
$^1$H-NMR: δ0.82 (d,J = 7.2 Hz, 3H), 1.15-1.28 (m,2H), 1.32-1.41 (m,2H), 3.65 (t,J = 6.6 Hz, 2H), 7.72-7.78 (m,2H), 7.87-7.93 (m,2H)

[Synthesis Example 48]

Lithium butoxycarbonyl-4-(trifluoromethoxy)phenyl sulfonamide [Synthesis Compound (I-48)] represented by the following structural formula was synthesized in the following manner.

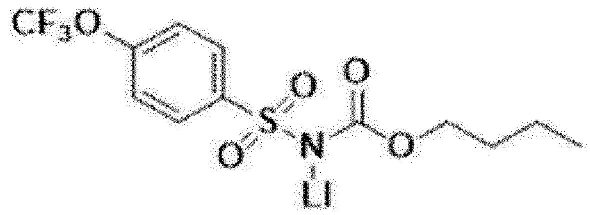

(I-48)

<First Step: Synthesis of Butoxycarbonyl-4-(trifluoromethoxy)phenyl Sulfonamide>

Butoxycarbonyl-4-(trifluoromethoxy)phenyl sulfonamide was synthesized in the same manner as in the first step of Synthesis Example 47, except that butyl chloroformate (1.59 g, 11.6 mmol), triethylamine (1.41 g, 13.9 mmol), 4-dimethylaminopyridine (0.17 g, 1.40 mmol), and tetrahydrofuran (30 mL) as a solvent were used, and 4-(trifluoromethyl)benzenesulfonamide was changed to 4-(trifluoromethoxy)benzenesulfonamide (2.80 g, 11.6 mmol) in the first step of Synthesis Example 47 (Synthesis Compound (I-47)).
By this, butoxycarbonyl-4-(trifluoromethoxy)phenyl sulfonamide was obtained as an 84th white solid (3.06 g, 8.97 mmol, yield = 77%).
The result of measuring this 84th white solid by $^1$H-NMR (DMSO-d6) is shown below.
$^1$H-NMR: δ0.81 (d,J = 7.2 Hz, 3H), 1.12-1.24 (m,2H), 1.40-1.50 (m,2H), 3.99 (t,J = 6.6 Hz, 2H), 7.62-7.68 (m,2H), 8.00-8.06 (m,2H)

<Second Step: Synthesis of Lithium Butoxycarbonyl-4-(trifluoromethoxy)phenyl Sulfonamide (I-40)>

Using butoxycarbonyl-4-(trifluoromethoxy)phenyl sulfonamide (3.06 g, 8.97 mmol), tetrahydrofuran (50 mL) as a solvent, and a lithium bis(trimethylsilyl)amide (1.3M) tetrahydrofuran solution (6.90 mL, 8.97 mmol), a reaction was carried out in the same manner as in the second step of Synthesis Example 47 (Synthesis Compound (I-47)).
By this, lithium butoxycarbonyl-4-(trifluoromethoxy)phenylamide was obtained as an 85th white solid (2.66 g, 7.66 mmol, yield = 85%).
The result of measuring this 85th white solid by $^1$H-NMR (DMSO-d6) is shown below.
$^1$H-NMR: δ0.83 (d,J = 7.4 Hz, 3H), 1.17-1.27 (m,2H), 1.32-1.42 (m,2H), 3.65 (t,J = 6.6 Hz, 2H), 7.32-7.38 (m,2H), 7.78-7.85 (m,2H)

[Example 1]

A nonaqueous electrolyte solution was obtained in the following manner.

<Preparation of Nonaqueous Electrolyte Solution>

Ethylene carbonate (hereinafter, "EC"), dimethyl carbonate (hereinafter, "DMC"), and ethyl methyl carbonate (hereinafter, "EMC") were mixed at EC: DMC: EMC = 30:35:35 (volume ratio), whereby a mixed solvent was obtained as a nonaqueous solvent.
In the thus obtained mixed solvent, $LiPF_6$ was dissolved as an electrolyte such that the concentration thereof in a nonaqueous electrolyte solution to be eventually obtained would be 1 mol/L, whereby an electrolyte solution was obtained.
The thus obtained electrolyte solution is hereinafter referred to as "basic electrolyte solution".
As an additive, lithium butoxycarbonyl tosylamide (I-5) represented by the following Formula (I-5), which was synthesized in Synthesis Example 1, was added to the basic electrolyte solution such that the content (% by mass) of this additive with respect to a total amount of a nonaqueous electrolyte solution to be eventually obtained would be as shown in Table 4, whereby a nonaqueous electrolyte solution was obtained.

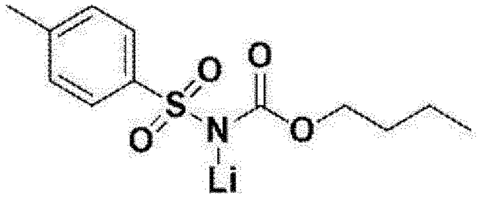

(I-5)

A coin-type battery (hereinafter, also simply referred to as "battery") was produced as a lithium secondary battery precursor in the following manner.

<Production of Positive Electrode>

A mixture was obtained by adding $Li(Ni_{0.5}Co_{0.2}Mn_{0.3}O_2)$ (94% by mass) as a positive electrode active material, carbon black (3% by mass) as a conductive aid, and polyvinylidene fluoride (PVdF) (3% by mass) as a binder. The thus obtained mixture was dispersed in an N-methylpyrrolidone solvent to obtain a positive electrode mixture slurry.

A 20 μm-thick aluminum foil was prepared as a positive electrode current collector.

The above-obtained positive electrode mixture slurry was applied and dried onto the aluminum foil, and the resultant was subsequently roll-pressed using a press machine to obtain a sheet-form positive electrode. This positive electrode consisted of the positive electrode current collector and a positive electrode active material layer.

<Production of Negative Electrode>

A negative electrode mixture slurry was obtained by mixing graphite (96% by mass) as a negative electrode active material, carbon black (1% by mass) as a conductive aid, sodium carboxymethyl cellulose dispersed in pure water as a thickening agent in an amount of 1% by mass in terms of solid content, and a styrene-butadiene rubber (SBR) dispersed in pure water as a binder in an amount of 2% by mass in terms of solid content.

A 10 μm-thick copper foil was prepared as a negative electrode current collector.

The above-obtained obtained slurry was applied and dried onto the copper foil, and the resultant was subsequently roll-pressed using a press machine to obtain a sheet-form negative electrode. This negative electrode consisted of the negative electrode current collector and a negative electrode active material layer.

The nonaqueous electrolyte solution obtained in the above "Preparation of Nonaqueous Electrolyte Solution" was prepared.

As a separator, a porous polyethylene film was prepared.

<Production of Coin-type Battery>

The negative electrode, the positive electrode, and the separator were punched out in disc shapes of 14 mm, 13 mm, and 17 mm in diameter, respectively, whereby a coin-shaped negative electrode, a coin-shaped positive electrode, and a coin-shaped separator were each obtained.

The thus obtained coin-shaped negative electrode, coin-shaped separator, and coin-shaped positive electrode were disposed in layers in the order mentioned inside a stainlesssteel battery can (size: 2032). Subsequently, 20 μL of the nonaqueous electrolyte solution was injected into the battery can to impregnate the separator, the positive electrode, and the negative electrode with the nonaqueous electrolyte solution.

Next, an aluminum plate (thickness: 1.2 mm, diameter: 16 mm) and a spring were placed on the positive electrode, and a battery can lid was caulked via a polypropylene gasket to tightly seal the resulting battery.

In this manner, a coin-type lithium secondary battery precursor having the configuration illustrated in FIG. 2 was obtained. This lithium secondary battery precursor had a size of 20 mm in diameter and 3.2 mm in height.

[Comparative Examples 1 and 2, and Examples 2 to 49]

Lithium secondary battery precursors were obtained in the same manner as in Example 1, except that, in the above-described "Preparation of Nonaqueous Electrolyte Solution", each additive shown in Table 4 was added such that the content (% by mass) thereof with respect to a total amount of a nonaqueous electrolyte solution to be eventually obtained would be as shown in Table 4.

In Comparative Example 2, lithium trifluoromethylcarbonyl trifluoromethyl sulfonamide (C-1) represented by the following Formula (C-1) was used.

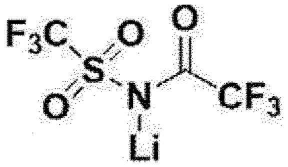

( C-1 )

[Evaluation Tests]

Each of the thus obtained lithium secondary battery precursors was subjected to the below-described aging treatment to obtain a first battery. The below-described initial charge-discharge treatment was performed on the thus obtained first battery to obtain a second battery. The thus obtained second battery was subjected to the below-described treatment for evaluation of direct-current resistance to obtain a third battery, which was subsequently subjected to a high-temperature storage treatment to obtain a fourth battery. The thus obtained fourth battery was subjected to the below-described late charge-discharge treatment to obtain a fifth battery.

Using the thus obtained first to fifth batteries, the capacity after high-temperature storage, the resistance after high-temperature storage, and the resistance increase rate were measured in accordance with the respective measurement methods described below. The results thereof are shown in Table 4.

<Aging Treatment>

Each lithium secondary battery precursor was subjected to the following aging treatment to obtain a first battery.

The battery precursor was charged to a final voltage range of from 1.5 V to 3.5 V in a temperature range of from 25 to 70°C and then rested for a period ranging from 5 to 50 hours. Subsequently, the battery precursor was charged to a final voltage range of from 3.5 V to 4.2 V in a temperature range of from 25 to 70°C and then maintained for a period ranging from 5 to 50 hours. Thereafter, the battery precursor was charged to 4.2 V in a temperature range of from 25 to 70°C and then discharged to 2.5 V.

<Initial Charge-Discharge Treatment>

The first battery was subjected to the following initial charge-discharge treatment to obtain a second battery.

The first battery was maintained in a temperature environment of 25°C for 12 hours. Subsequently, the first battery was constant-current constant-voltage charged at a charge rate of 0.2 C (0.2C-CCCV) up to 4.2 V (SOC (State of Charge) 100%), rested for 30 minutes, and then constant-current discharged at a discharge rate of 0.2 C (0.2C-CC) to 2.5 V. These operations were performed for a total of three cycles to stabilize the battery. Thereafter, this battery was constant-current constant-voltage charged at a charge rate of 0.2 C (0.5C-CCCV) up to 4.2 V, rested for 30 minutes, and then constant-current discharged at a discharge rate of 1 C (1C-CC) to 2.5 V, whereby a second battery was obtained.

<Treatment for Evaluation of Direct-Current Resistance>

The second battery was subjected to the following treatment for evaluation of direct-current resistance to obtain a third battery.

The treatment for evaluation of direct-current resistance was performed in a temperature environment of 25°C. The second battery was subjected to CC discharging to 2.5 V at a discharge rate of 0.2 C and then CCCV charging up to 3.7 V at a charge rate of 0.2 C. The term "CCCV charging" used herein means to perform charging at a constant current and a constant voltage.

Subsequently, the second battery was subjected to CC10s discharging at a discharge rate of 0.2 C and then CClOs charging at a charge rate of 0.2 C. The term "CClOs discharging" used herein means to perform discharging at a constant current for 10 seconds. The term "CC10s charging" used herein means to perform charging at a constant current for 10 seconds.

Next, the second battery was subjected to CC10s discharging at a discharge rate of 0.5 C and subsequent CC25s charging at a charge rate of 0.2 C. Then, the second battery was subjected to CC10s discharging at a discharge rate of 1 C and subsequent CC50s charging at a charge rate of 0.2 C. Thereafter, the second battery was subjected to CC10s discharging at a discharge rate of 2 C and subsequent CC100s charging at a charge rate of 0.2 C, whereby a third battery was obtained.

<High-Temperature Storage Treatment>

The third battery was subjected to the following high-temperature storage treatment to obtain a fourth battery.

The third battery was constant-current charged up to 4.2 V at a charge rate of 0.2 C in a temperature environment of 25°C. Subsequently, this battery in a charged state was left to stand for 14 days in a 60°C atmosphere. By this, a fourth battery was obtained.

<Late Charge-Discharge Treatment>

The fourth battery was subjected to the following late charge-discharge treatment to obtain a fifth battery.

The fourth battery was allowed to dissipate its heat in a temperature environment of 25°C and subjected to first discharging and then first charging, followed by second discharging. The "first discharging" refers to that the fourth battery was constant-current discharged at a discharge rate of 1 C (1C-CC) to 2.5 V. The "first charging" refers to that the fourth battery was constant-current constant-voltage charged at a charge rate of 0.2 C (0.2 C-CCCV) up to 4.2 V. The "second discharging" refers to that the fourth battery was constant-current discharged at a discharge rate of 1 C (1C-CC) to 2.5 V. A fifth battery was obtained as a result.

<Method of Measuring Capacity after High-Temperature Storage>

As indicated by the below-described equation (X1), a relative value of the discharge capacity of each fourth battery of Examples with respect to the discharge capacity of the fourth battery of Comparative Example 1 was defined as "capacity after high-temperature storage [%]". The capacity after high-temperature storage represents the capacity that was obtained when the second discharging was performed in the above-described late charge-discharge treatment.

Capacity after high-temperature storage [relative value; %] = (Discharge capacity of fourth battery [mAh/g]/Discharge capacity of fourth battery of Comparative Example 1 [mAh/g]) × 100 (X1)

<Method of Measuring Resistance after High-Temperature Storage>

As indicated by the below-described equation (X2), a relative value of the direct-current internal resistance (DCIR) of each fifth battery with respect to the direct-current internal resistance (DCIR) of the fifth battery of Comparative Example 1 was defined as "capacity after high-temperature storage [%]".

Resistance after high-temperature storage [relative value; %] = (Direct-current resistance of fifth battery [Ω]/Direct-current resistance of fifth battery of Comparative Example 1 [Ω]) × 100 (X2)

The direct-current resistance was measured by the following method. The fifth battery was subjected to the same treatment for evaluation of direct-current resistance as the one described above. The direct-current resistance (Ω) of the fifth battery was determined based on the amount of decrease in voltage due to "CClOs discharging" at each discharge rate of 0.2 C to 1 C (= Voltage before initiation of discharging - Voltage at 10th second after initiation of discharging) and each current value (i.e. the current value corresponding to each discharge rate of 0.2 C to 1 C).

<Method of Measuring Resistance Increase Rate>

As indicated by the below-described equation (X3), a relative value of resistance increase rate with respect to the resistance increase rate of Comparative Example 1 was defined as "resistance increase rate [%]".

Resistance increase rate [relative value; %] = (Resistance increase rate/Resistance increase rate of Comparative Example 1) × 100 (X3)

In equation (X3), the resistance increase rate is a value obtained by dividing the direct-current resistance (Ω) of the fourth battery by the direct-current resistance (Ω) of the second battery. The direct-current resistance (Ω) of the fourth battery and the direct-current resistance (Ω) of the second battery are each measured in the same manner as the direct-current resistance (Ω) of the fifth battery in the above-described method of measuring the resistance after high-temperature storage.

The relative value of the direct-current resistance of the fifth battery subjected to the high-temperature storage test corresponds to the rate (%) of increase in the direct-current resistance caused by storage (hereinafter, also simply referred to as "resistance increase rate"). The term "increase rate" used herein is expressed as: "100%" when the resistance neither increases nor decreases; "higher than 100%" when the resistance increases; or "lower than 100%" when the resistance decreases.

The reason why attention was given to the resistance increase rate is because, although a low resistance value itself is an important in the battery performance, a reduction in the resistance increase rate due to deterioration and the like during a storage period is also an extremely important performance.

[Table 4]

| | Electrolyte solution for lithium secondary battery | | | | | | | Lithium secondary battery | | |
|---|---|---|---|---|---|---|---|---|---|---|
| | Content of additives | | | | | | | | | |
| | (1) | | | (C-1) | (II) | (III) | (IV) | Capacity after high-temperature storage | Resistance after high-temperature storage | Resistance increase rate |
| | | | | | (II-1) | (III-1) | (IV-1) | | | |
| | Compound No. | Synthesis Example | (% by mass) | (% by mass) | (% by mass) | (% by mass) | (% by mass) | [%] | [%] | [%] |
| Comparative Example 1 | - | - | - | - | - | - | - | 100 | 100 | 100 |
| Comparative Example 2 | - | - | - | 0.5 | - | - | - | 100 | 106 | 102 |
| Example 1 | (1-5) | 1 | 0.5 | - | - | - | - | 101 | 95 | 96 |
| Example 2 | | | 0.5 | - | 0.5 | - | - | 103 | 91 | 93 |
| Example 3 | | | 0.5 | - | - | 0.5 | - | 103 | 93 | 87 |
| Example 4 | | | 0.5 | - | - | - | 0.5 | 105 | 84 | 91 |
| Example 5 | (I-10) | 2 | 0.5 | - | - | - | - | 103 | 96 | 92 |
| Example 6 | | | 0.5 | - | 0.5 | - | - | 106 | 84 | 93 |
| Example 7 | | | 0.5 | - | - | 0.5 | - | 106 | 87 | 84 |
| Example 8 | | | 0.5 | - | - | - | 0.5 | 108 | 91 | 91 |
| Example 9 | (1-1) | 3 | 0.5 | - | - | - | - | 100 | 98 | 98 |
| Example 10 | (1-2) | 4 | 0.5 | - | - | - | - | 100 | 99 | 91 |
| Example 11 | | | 0.5 | - | 0.5 | - | - | 101 | 92 | 94 |
| Example 12 | | | 0.5 | - | - | 0.5 | - | 102 | 91 | 82 |
| Example 13 | | | 0.5 | - | - | - | 0.5 | 102 | 88 | 87 |
| Example 14 | (1-4) | 6 | 0.5 | - | - | - | - | 101 | 98 | 89 |
| Example 15 | | | 0.5 | - | 0.5 | - | - | 102 | 90 | 93 |
| Example 16 | | | 0.5 | - | - | 0.5 | - | 103 | 90 | 81 |
| Example 17 | | | 0.5 | - | - | - | 0.5 | 103 | 87 | 88 |

(continued)

| | Electrolyte solution for lithium secondary battery | | | | | | | Lithium secondary battery | | |
|---|---|---|---|---|---|---|---|---|---|---|
| | Content of additives | | | | | | | | | |
| | (1) | | | (C-1) | (II) | (III) | (IV) | Capacity after high-temperature storage | Resistance after high-temperature storage | Resistance increase rate |
| | | | | | (II-1) | (III-1) | (IV-1) | | | |
| | Compound No. | Synthesis Example | (% by mass) | (% by mass) | (% by mass) | (% by mass) | (% by mass) | [%] | [%] | [%] |
| Example 18 | (I-11) | 11 | 0.5 | - | - | - | - | 101 | 92 | 90 |
| Example 19 | | | 0.5 | - | 0.5 | - | - | 106 | 89 | 99 |
| Example 20 | | | 0.5 | - | - | 0.5 | - | 104 | 90 | 83 |
| Example 21 | | | 0.5 | - | - | - | 0.5 | 106 | 88 | 89 |
| Example 22 | (I-12) | 12 | 0.5 | - | - | - | - | 102 | 98 | 90 |
| Example 23 | (I-13) | 13 | 0.5 | - | - | - | - | 102 | 100 | 95 |
| Example 24 | (I-14) | 14 | 0.5 | - | - | - | - | 104 | 95 | 94 |
| Example 25 | | | 0.5 | - | 0.5 | - | - | 105 | 80 | 92 |
| Example 26 | | | 0.5 | - | - | 0.5 | - | 104 | 79 | 79 |
| Example 27 | | | 0.5 | - | - | - | 0.5 | 107 | 78 | 89 |
| Example 28 | (I-16) | 16 | 0.05 | - | - | - | - | 102 | 93 | 92 |
| Example 29 | (I-23) | 23 | 0.5 | - | - | - | - | 100 | 98 | 86 |
| Example 30 | | | 0.5 | - | 0.5 | - | - | 100 | 95 | 97 |
| Example 31 | | | 0.5 | - | - | 0.5 | - | 102 | 94 | 79 |
| Example 32 | | | 0.5 | - | - | - | 0.5 | 103 | 99 | 88 |
| Example 33 | (I-27) | 27 | 0.5 | - | - | - | - | 101 | 93 | 87 |
| Example 34 | (I-28) | 28 | 0.5 | - | - | - | - | 101 | 91 | 91 |
| Example 35 | (I-30) | 30 | 0.5 | - | - | - | - | 101 | 96 | 93 |
| Example 36 | (I-34) | 34 | 0.5 | - | - | - | - | 100 | 86 | 85 |
| Example 37 | (I-37) | 37 | 0.5 | - | - | - | - | 103 | 88 | 92 |

(continued)

| | Electrolyte solution for lithium secondary battery | | | | | | | | Lithium secondary battery | | |
|---|---|---|---|---|---|---|---|---|---|---|---|
| | Content of additives | | | | | | | | | | |
| | (1) | | | (C-1) | (II) (II-1) | (III) (III-1) | (IV) (IV-1) | | Capacity after high-temperature storage | Resistance after high-temperature storage | Resistance increase rate |
| | Compound No. | Synthesis Example | (% by mass) | (% by mass) | (% by mass) | (% by mass) | (% by mass) | | [%] | [%] | [%] |
| Example 38 | (I-39) | 39 | 0.2 | - | - | - | - | | 101 | 99 | 96 |
| Example 39 | (I-42) | 42 | 0.5 | - | - | - | - | | 105 | 94 | 92 |
| Example 40 | (I-43) | 43 | 0.5 | - | - | - | - | | 100 | 89 | 95 |
| Example 41 | (I-44) | 44 | 0.5 | - | - | - | - | | 103 | 93 | 100 |
| Example 42 | (I-45) | 45 | 0.5 | - | - | - | - | | 102 | 95 | 91 |
| Example 43 | | | 0.5 | - | 0.5 | - | - | | 103 | 86 | 99 |
| Example 44 | | | 0.5 | - | - | 0.5 | - | | 102 | 92 | 90 |
| Example 45 | | | 0.5 | - | - | - | 0.5 | | 102 | 90 | 98 |
| Example 46 | (I-46) | 46 | 0.5 | - | - | - | - | | 101 | 100 | 93 |
| Example 47 | | | 0.5 | - | 0.5 | - | - | | 102 | 88 | 94 |
| Example 48 | | | 0.5 | - | - | 0.5 | - | | 101 | 92 | 75 |
| Example 49 | | | 0.5 | - | - | - | 0.5 | | 103 | 85 | 96 |

In Table 4, "-" means that the corresponding component was not incorporated, and "Content of additives" indicates the content (% by mass) of each additive with respect to a total amount of the respective nonaqueous electrolyte solution for a lithium secondary battery. "(I)" represents a lithium (N-carbonyl)sulfonamide compound (1); "(II)" represents a lithium fluorophosphate compound (II); "(C-1)" represents lithium trifluoromethylcarbonyl trifluoromethyl sulfonamide (C-1); "(III)" represents a cyclic dicarbonyl compound (III); "(IV)" represents a cyclic sulfur-containing ester compound (IV); "(I-1)" represents lithium methoxycarbonyl tosylamide (I-1); "(I-2)" represents lithium ethoxycarbonyl tosylamide (1-2); "(I-4)" represents lithium isopropoxycarbonyl tosylamide (I-4); "(I-5)" represents lithium butoxycarbonyl tosylamide (I-5); "(I-10)" represents lithium methoxycarbonyl trifluoromethyl sulfonamide (1-10); "(I-11)" represents lithium ethoxycarbonyl trifluoromethyl sulfonamide (I-11); "(1-12)" represents lithium propoxycarbonyl trifluoromethyl sulfonamide (1-12); "(I-13)" represents lithium isopropoxycarbonyl trifluoromethyl sulfonamide (I-13); "(I-14)" represents lithium butoxycarbonyl trifluoromethyl sulfonamide (I-14); "(I-16)" represents lithium methoxycarbonylmethyl sulfonamide (I-16); "(I-23)" represents lithium benzoylethoxy sulfonamide (I-23); "(I-27)" represents lithium ethoxycarbonyl-2,2,2-trifluoroethoxy sulfonamide (I-27); "(I-28)" represents lithium-(2,2,2-trifluoroethoxy)carbonyl-(2,2,2-trifluoroethoxy)sulfonamide (I-28); "(I-30)" represents lithium ethoxycarbonyl-p-tolyloxy sulfonamide (I-30); "(I-34)" represents lithium-4-methoxyphenoxycarbonyl trifluoromethyl sulfonamide (I-34); "(I-37)" represents lithium allyloxycarbonyl trifluoromethyl sulfonamide (I-37); "(I-39)" represents lithium methoxycarbonyl-4-trifluoromethylphenyl sulfonamide (I-39); "(I-42)" represents lithium fluorosulfonylmethoxycarbonylamide (I-42); "(I-43)" represents lithium fluorosulfonylethoxycarbonylamide (I-43); "(I-44)" represents lithium fluorosulfonylpropoxycarbonylamide (I-44); "(I-45)" represents lithium fluorosulfonylbutoxycarbonylamide (I-45); "(I-46)" represents lithium fluorosulfonylbenzyloxycarbonylamide (I-46); "(II-1)" represents lithium difluorophosphate (II-1); "(III-1)" represents lithium bis(oxalato)borate (III-1); and "(IV-1)" represents a cyclic sulfur-containing ester compound (IV-1).

The nonaqueous electrolyte solutions of Examples 1 to 49 contained a lithium (*N*-carbonyl)sulfonamide compound (I); therefore, the lithium secondary batteries of Examples 1 to 49 had a capacity after high-temperature storage of 100% or more, a resistance after high-temperature storage of 100% or less, and a resistance increase rate of 100% or lower. In other words, it was found that, in the lithium secondary batteries of Examples 1 to 49, an increase in the direct-current resistance and a decrease in the discharge capacity can be inhibited even when these lithium secondary batteries are stored in a high-temperature environment.

On the other hand, the nonaqueous electrolyte solution of Comparative Example 2 contained trifluoromethylcarbonyl trifluoromethyl sulfonamide (C-1) but not a lithium (N-carbonyl)sulfonamide compound (1). Therefore, the lithium secondary battery of Comparative Example 2 had a capacity after high-temperature storage of 100%, a resistance after high-temperature storage of 106%, and a resistance increase rate of 102%. In other words, it was found that, in the lithium secondary battery of Comparative Example 2, an increase in the direct-current resistance and a decrease in the discharge capacity cannot be inhibited when the lithium secondary battery is stored in a high-temperature environment.

The disclosure of Japanese Patent Application No. 2021-044154 filed on March 17, 2021, and the disclosure of Japanese Patent Application No. 2021-143890 filed on September 3, 2021, are hereby incorporated by reference in their entirety.

All the documents, patent applications, and technical standards that are described in the present specification are hereby incorporated by reference to the same extent as if each individual document, patent application, or technical standard is concretely and individually described to be incorporated by reference.

## Claims

1. A lithium (N-carbonyl)sulfonamide compound represented by the following Formula (1):

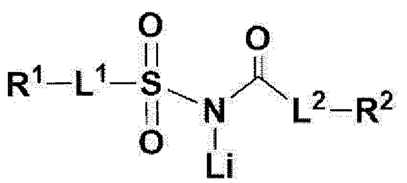

(I)

wherein, in Formula (I):

each of $R^1$ and $R^2$ represents an alkyl group having from 1 to 10 carbon atoms, wherein at least one hydrogen atom of the alkyl group is optionally substituted with a halogen atom, an alkenyl group having from 2 to 10 carbon atoms, wherein at least one hydrogen atom of the alkenyl group is optionally substituted with a halogen atom, an alkynyl group having from 2 to 10 carbon atoms, wherein at least one hydrogen atom of the alkynyl

group is optionally substituted with a halogen atom, or an aryl group, wherein at least one hydrogen atom of the aryl group is optionally substituted with a halogen atom, an alkoxy group having from 1 to 6 carbon atoms or an alkyl group having from 1 to 6 carbon atoms, and
$L^1$ and $L^2$ each represent a single bond or -O-, and $L^1$ and $L^2$ are not both single bonds.

2. An additive for a lithium secondary battery, comprising a lithium (N-carbonyl)sulfonamide compound (I) represented by the following Formula (I):

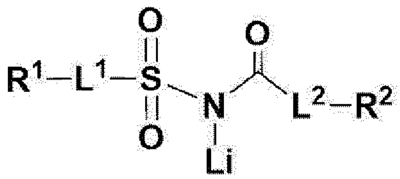

(I)

wherein, in Formula (I):

each of $R^1$ and $R^2$ represents an alkyl group having from 1 to 10 carbon atoms, wherein at least one hydrogen atom of the alkyl group is optionally substituted with a halogen atom that is not a trifluoromethyl group, an alkenyl group having from 2 to 10 carbon atoms, wherein at least one hydrogen atom of the alkenyl group is optionally substituted with a halogen atom, an alkynyl group having from 2 to 10 carbon atoms, wherein at least one hydrogen atom of the alkynyl group is optionally substituted with a halogen atom, or an aryl group, wherein at least one hydrogen atom of the aryl group is optionally substituted with a halogen atom, an alkoxy group having from 1 to 6 carbon atoms, or an alkyl group having from 1 to 6 carbon atoms, and
$L^1$ and $L^2$ each represent a single bond or -O-.

3. The additive for a lithium secondary battery according to claim 2, wherein:

each of $R^1$ and $R^2$ represents, in place of the alkyl group, the alkenyl group, the alkynyl group, or the aryl group: an alkyl group having from 1 to 10 carbon atoms, wherein at least one hydrogen atom of this alkyl group is optionally substituted with a halogen atom, the alkenyl group, the alkynyl group, the aryl group, an aralkyl group having from 7 to 16 carbon atoms, wherein at least one hydrogen atom of an aromatic ring in the aralkyl group is optionally substituted with a halogen atom, an alkoxy group having from 1 to 6 carbon atoms, or an alkyl group having from 1 to 6 carbon atoms, or $R^1$ and $R^2$ represent a halogen atoms, and
a case in which $R^1$ is a halogen atom and $L^1$ is -O-, a case in which $R^2$ is a halogen atom and $L^2$ is -O-, and a case in which $R^1$ and $R^2$ are both the alkyl groups or the aryl groups, and $L^1$ and $L^2$ are both single bonds, are excluded.

4. A nonaqueous electrolyte solution for a lithium secondary battery, the solution comprising a lithium (N-carbonyl)sulfonamide compound (I) represented by the following Formula (I):

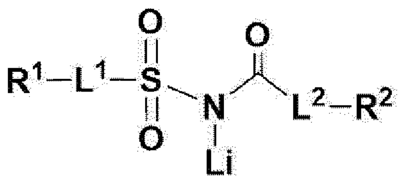

(I)

wherein, in Formula (I):

each of $R^1$ and $R^2$ represents an alkyl group having from 1 to 10 carbon atoms, wherein at least one hydrogen atom of the alkyl group is optionally substituted with a halogen atom that is not a trifluoromethyl group, an alkenyl group having from 2 to 10 carbon atoms, wherein at least one hydrogen atom of the alkenyl group is optionally substituted with a halogen atom, an alkynyl group having from 2 to 10 carbon atoms, wherein at least one hydrogen atom of the alkynyl group is optionally substituted with a halogen atom, or an aryl group, wherein at least one hydrogen atom of the aryl group is optionally substituted with a halogen atom, an alkoxy group having from 1 to 6 carbon atoms or an alkyl group having from 1 to 6 carbon atoms, and
$L^1$ and $L^2$ each represent a single bond or -O-.

**5.** The nonaqueous electrolyte solution for a lithium secondary battery according to claim 4, wherein:

$R^1$ and $R^2$ each represent, in place of the alkyl group, the alkenyl group, the alkynyl group, or the aryl group: an alkyl group having from 1 to 10 carbon atoms, wherein at least one hydrogen atom of this alkyl group is optionally substituted with a halogen atom, the alkenyl group, the alkynyl group, the aryl group, an aralkyl group having from 7 to 16 carbon atoms, wherein at least one hydrogen atom of an aromatic ring in the aralkyl group is optionally substituted with a halogen atom, an alkoxy group having from 1 to 6 carbon atoms, or an alkyl group having from 1 to 6 carbon atoms, or $R^1$ and $R^2$ represent a halogen atoms, and
a case in which $R^1$ is a halogen atom and $L^1$ is -O-, a case in which $R^2$ is a halogen atom and $L^2$ is -O-, and a case in which $R^1$ and $R^2$ are both the alkyl groups or the aryl groups, and $L^1$ and $L^2$ are both single bonds, are excluded.

**6.** The nonaqueous electrolyte solution for a lithium secondary battery according to claim 4 or 5, further comprising an electrolyte,
wherein the electrolyte is at least one selected from the group consisting of lithium hexafluorophosphate ($LiPF_6$), lithium tetrafluoroborate ($LiBF_4$), lithium hexafluoroarsenate ($LiAsF_6$), lithium hexafluorotantalate ($LiTaF_6$), lithium trifluoromethane sulfonate ($LiCF_3SO_3$), lithium bis(trifluoromethanesulfonyl)imide ($Li(CF_3SO_2)_2N$), and lithium bis(pentafluoroethanesulfonyl)imide ($Li(C_2F_5SO_2)_2N$).

**7.** The nonaqueous electrolyte solution for a lithium secondary battery according to any one of claims 4 to 6, wherein, in the lithium (N-carbonyl)sulfonamide compound (I):

$R^1$ represents the aryl group,
$L^1$ represents a single bond,
$R^2$ represents the alkyl group, the alkenyl group, the alkynyl group, the aryl group, or the aralkyl group, and
$L^2$ represents -O-.

**8.** The nonaqueous electrolyte solution for a lithium secondary battery according to any one of claims 4 to 6, wherein, in the lithium (N-carbonyl)sulfonamide compound (I):

$R^1$ represents the alkyl group,
$L^1$ represents a single bond,
$R^2$ represents the alkyl group, the alkenyl group, the alkynyl group, the aryl group, or the aralkyl group, and
$L^2$ represents -O-.

**9.** The nonaqueous electrolyte solution for a lithium secondary battery according to any one of claims 4 to 6, wherein, in the lithium (N-carbonyl)sulfonamide compound (I):

$R^1$ represents a fluorine atom,
$L^1$ represents a single bond,
$R^2$ represents the alkyl group, the alkenyl group, the alkynyl group, the aryl group, or the aralkyl group, and
$L^2$ represents -O-.

**10.** The nonaqueous electrolyte solution for a lithium secondary battery according to any one of claims 4 to 9, comprising a compound (II) that is at least one selected from the group consisting of lithium monofluorophosphate and lithium difluorophosphate.

**11.** The nonaqueous electrolyte solution for a lithium secondary battery according to any one of claims 4 to 10, comprising a compound (III) represented by the following Formula (III):

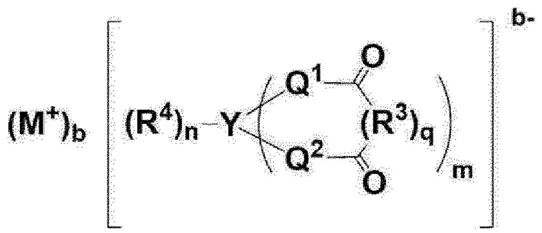

( III )

wherein, in Formula (III):

M represents an alkali metal,
Y represents a transition element, or an element of Group 13, 14, or 15 of the periodic table,
b represents an integer from 1 to 3,
m represents an integer from 1 to 4,
n represents an integer from 0 to 8,
q represents 0 or 1,
$R^3$ represents an alkylene group having from 1 to 10 carbon atoms, a halogenated alkylene group having from 1 to 10 carbon atoms, an arylene group having from 6 to 20 carbon atoms, or a halogenated arylene group having from 6 to 20 carbon atoms, the halogenated alkylene group, the arylene group and the halogenated arylene group each optionally containing a substituent or a heteroatom in a structure thereof and, when q is 1 and m is from 2 to 4, m instances of $R^3$ are optionally bound to each other,
$R^4$ represents a halogen atom, an alkyl group having from 1 to 10 carbon atoms, a halogenated alkyl group having from 1 to 10 carbon atoms, an aryl group having from 6 to 20 carbon atoms, or a halogenated aryl group having from 6 to 20 carbon atoms, the halogenated alkyl group, the aryl group and the halogenated aryl group each optionally containing a substituent or a heteroatom in a structure thereof and, when n is from 2 to 8, n instances of $R^4$ are optionally bound to each other to form a ring, and
each of $Q^1$ and $Q^2$ independently represents an oxygen atom or a carbon atom.

**12.** The nonaqueous electrolyte solution for a lithium secondary battery according to any one of claims 4 to 11, comprising a compound (IV) represented by the following Formula (IV):

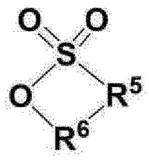 ( IV )

wherein, in Formula (IV):

$R^5$ represents an oxygen atom, an alkylene group having from 1 to 6 carbon atoms, or an alkenylene group having from 2 to 6 carbon atoms,
$R^6$ represents an alkylene group having from 1 to 6 carbon atoms, an alkenylene group having from 2 to 6 carbon atoms, a group represented by the following Formula (iv-1), or a group represented by the following Formula (iv-2):

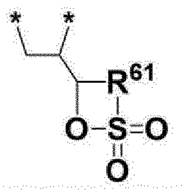 ( iv-1 ) 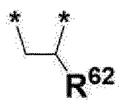 ( iv-2 )

wherein, in Formula (iv-1) and Formula (iv-2), * represents a bonding position,
in Formula (iv-1), $R^{61}$ represents an oxygen atom, an alkylene group having from 1 to 6 carbon atoms, an alkenylene group having from 2 to 6 carbon atoms, or an oxymethylene group, and
in Formula (iv-2), $R^{62}$ represents an alkyl group having from 1 to 6 carbon atoms, or an alkenyl group having from 2 to 6 carbon atoms.

**13.** The nonaqueous electrolyte solution for a lithium secondary battery according to any one of claims 4 to 12, wherein a content of the lithium (N-carbonyl)sulfonamide compound (I) is from 0.01% by mass to 5% by mass with respect to a total amount of the nonaqueous electrolyte solution for a lithium secondary battery.

**14.** A lithium secondary battery precursor, comprising:

a casing; and
a positive electrode, a negative electrode, a separator, and an electrolyte solution, which are housed in the casing, wherein:

the positive electrode is configured to occlude and release lithium ions,
the negative electrode is configured to occlude and release lithium ions, and
the electrolyte solution is the nonaqueous electrolyte solution for a lithium secondary battery according to any one of claims 4 to 13.

**15.** The lithium secondary battery precursor according to claim 14, wherein the positive electrode comprises a lithium-containing composite oxide represented by the following Formula (C1) as a positive electrode active material:

$LiNi_aCo_bMn_cO_2$ (C1)
wherein, in Formula (C1), each of a, b, and c independently represents a number larger than 0 but smaller than 1, and a sum of a, b, and c is from 0.99 to 1.00.

**16.** A method of producing a lithium secondary battery, the method comprising:

a step of preparing the lithium secondary battery precursor according to claim 14 or 15; and
a step of charging and discharging the lithium secondary battery precursor.

**17.** A lithium secondary battery, obtained by charging and discharging the lithium secondary battery precursor according to claim 14 or 15.

FIG.1

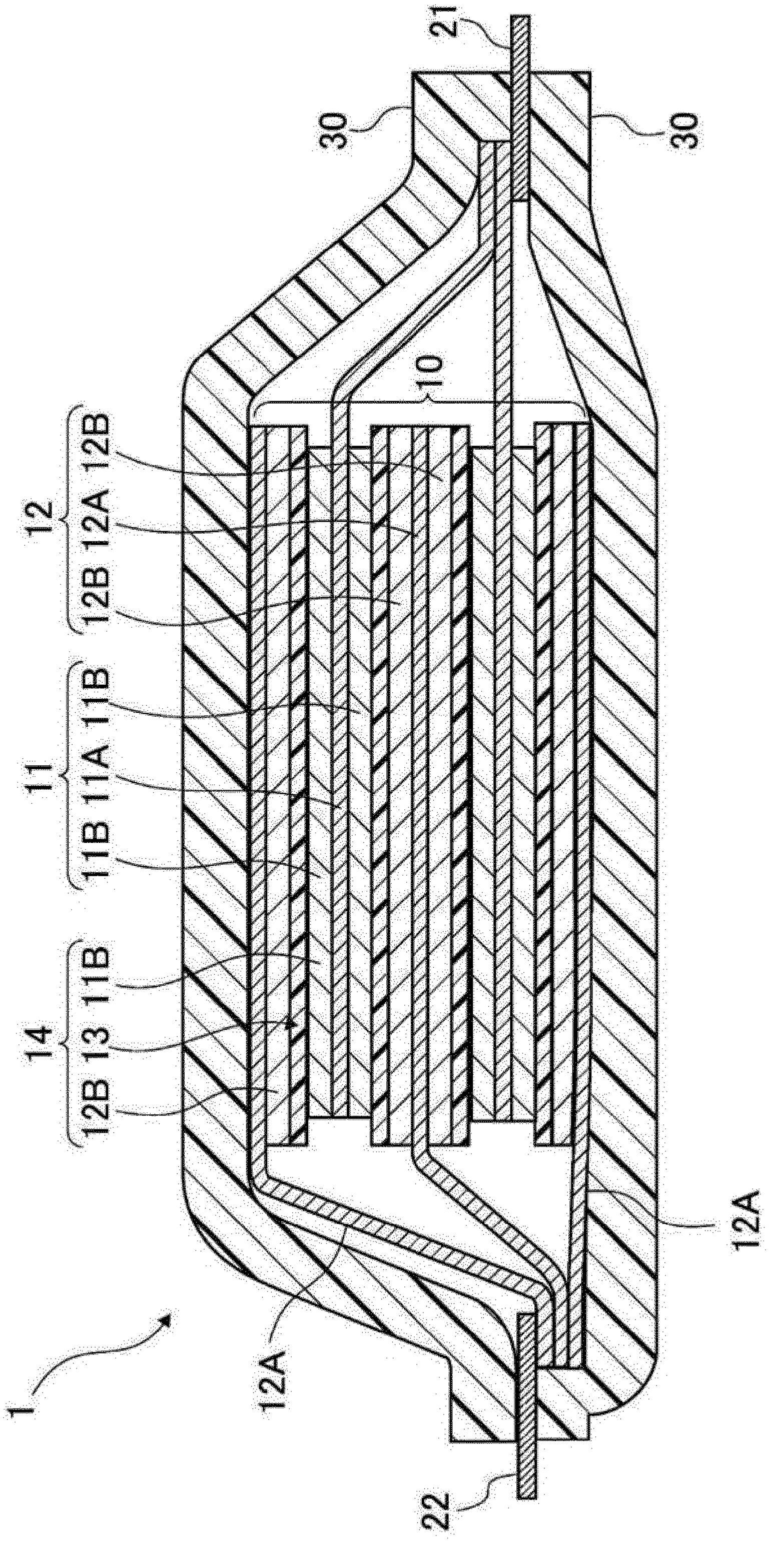

FIG.2

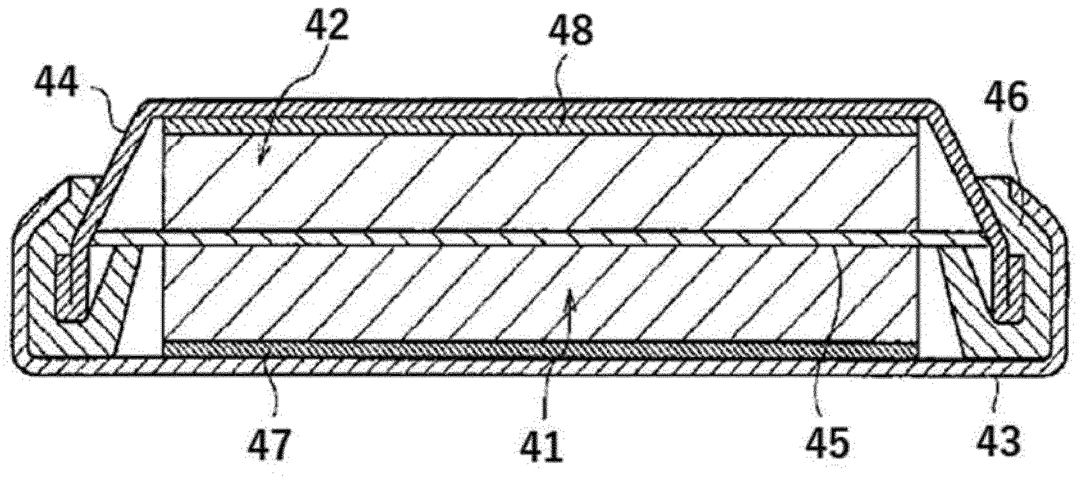

## INTERNATIONAL SEARCH REPORT

International application No.
**PCT/JP2022/006194**

### A. CLASSIFICATION OF SUBJECT MATTER

***C07C 307/02***(2006.01)i; ***C07C 311/53***(2006.01)i; ***H01M 4/505***(2010.01)i; ***H01M 4/525***(2010.01)i; ***H01M 10/052***(2010.01)i; ***H01M 10/0567***(2010.01)i; ***H01M 10/058***(2010.01)i
FI: C07C307/02 CSP; C07C311/53; H01M4/505; H01M4/525; H01M10/052; H01M10/0567; H01M10/058

According to International Patent Classification (IPC) or to both national classification and IPC

### B. FIELDS SEARCHED

Minimum documentation searched (classification system followed by classification symbols)

C07C307/02; C07C311/53; H01M4/505; H01M4/525; H01M10/052; H01M10/0567; H01M10/058

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | WO 2018/176134 A1 (HYDRO-QUEBEC) 04 October 2018 (2018-10-04) claims, pp. 4, 28, example 5 | 2, 4, 13-17 |
| Y | | 6, 10-12 |
| A | | 1, 3, 5, 7-9 |
| X | JP 2011-134459 A (KONICA MINOLTA HOLDINGS INC.) 07 July 2011 (2011-07-07) claims, paragraph [0026], examples | 3, 5, 13-17 |
| Y | | 6, 10-12 |
| A | | 1-2, 4, 7-9 |
| X | JP 2006-210022 A (TOYOTA MOTOR CORP.) 10 August 2006 (2006-08-10) claims, paragraphs [0012]-[0015], [0039], [0040], examples | 2, 4, 6, 13-17 |
| Y | | 6, 10-12 |
| A | | 1, 3, 5, 7-9 |
| Y | WO 2020/121850 A1 (MITSUI CHEMICALS, INC.) 18 June 2020 (2020-06-18) claims | 10-12 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 April 2022** | **19 April 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/006194**

**C. DOCUMENTS CONSIDERED TO BE RELEVANT**

| Category* | Citation of document, with indication, where appropriate, of the relevant passages | Relevant to claim No. |
|---|---|---|
| A | WO 2017/126701 A1 (PIOTREK CO., LTD.) 27 July 2017 (2017-07-27) examples, claims | 1-17 |

Form PCT/ISA/210 (second sheet) (January 2015)

**INTERNATIONAL SEARCH REPORT**
**Information on patent family members**

International application No.
**PCT/JP2022/006194**

| Patent document cited in search report | Publication date (day/month/year) | Patent family member(s) | Publication date (day/month/year) |
|---|---|---|---|
| WO 2018/176134 A1 | 04 October 2018 | JP 2020-515558 A | |
| | | US 2020/0087262 A1<br>example 5, claims | |
| | | EP 3601233 A1 | |
| | | CA 3057377 A | |
| | | KR 10-2019-0134691 A | |
| JP 2011-134459 A | 07 July 2011 | (Family: none) | |
| JP 2006-210022 A | 10 August 2006 | (Family: none) | |
| WO 2020/121850 A1 | 18 June 2020 | (Family: none) | |
| WO 2017/126701 A1 | 27 July 2017 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020515558 A
- JP 2019153443 A
- WO 2017156179 A
- JP 2021044154 A
- JP 2021143890 A


### Non-patent literature cited in the description


- *Journal of the Chemical Society. Perkin transactions I,* 1982, 677-680
- **PICARD, J.A. et al.** *J. Med. Chem.,* 1996, vol. 39, 1243